# EUROPEAN PATENT APPLICATION

(11) **EP 4 570 791 A1**
(43) Date of publication of application: **18.06.2025**
(21) Application number: 23307215.6
(22) Date of filing: 14.12.2023
(51) Int. Cl.: C07D 335/16, C07C 327/16

(54) **THIOESTER PHOTOINITIATORS**

(71) Applicant: ARKEMA FRANCE, 92800 Puteaux (FR); Centre National de la Recherche Scientifique, 75016 Paris (FR); Université de Haute-Alsace, 68200 Mulhouse (FR)
(72) Inventor: SQUIRES, Kelly, Wetherby, LS22 7NS (GB); SEHNAL, Petr, Wetherby, LS22 7NS (GB); PLENDERLEITH, Richard, Wetherby, LS22 7NS (GB); GARRA, Patxi, 08193 Bellaterra (ES); FAGGI, Enrico, 08193 Bellaterra (ES); SPRICK, Élodie, 69110 Sainte-Foy-lès-Lyon (FR); LALEVEE, Jacques, 68057 Mulhouse (FR); BECHT, Jean-Michel, 68057 Mulhouse (FR)
(74) Representative: Lavoix

(57) **Abstract**

The invention relates to thioester compounds, their preparation process and their use as photoinitiators, preferably as photoinitiators activable under 250 to 550 nm, in particular 250 to 460 nm, light irradiation, notably to cure formulations comprising one or more ethylenically unsaturated compounds.

## Description

### TECHNICAL FIELD

The invention relates to thioester compounds, their preparation process and their use as photoinitiators, preferably as photoinitiators activable under 250 to 550 nm, in particular 250 to 460 nm, light irradiation, notably to cure formulations comprising one or more ethylenically unsaturated compounds.

### TECHNICAL BACKGROUND

The increasing use of UV-visible curing technologies in areas such as high-speed printing, surface coating and additive manufacturing places high demands on the parameters of polymer networks that form in these processes. In particular, reactive photoinitiators are needed, that are thermally and chemically stable and that, after being activated by light or UV-radiation, can act as catalysts for a variety of anionic and/or free radical initiated polymerization reactions.

The most common photoinitiators are active under UV-B and UV-C light sources but there is a push to find photoinitiators active at longer wavelengths as the industry moves to using LED curing.

Photoinitiators which can be effectively activated by low-energy light sources such as LEDs without the need for additional sensitizers, but show high thermal stability in formulations before light exposure, are required.

Some common photoinitiators, such as (2,4,6-trimethylbenzoyldiphenylphosphine oxide (SpeedCure TPO), 2-Benzyl-2-dimethylamino-1-(4-morpholinophenyl)-butan-1-one (SpeedCure BDMB), 2-(dimethylamino)-2-[(4-methylphenyl)methyl]-1-[4-(morpholin-4-yl)phenyl]butan-1-one (Omnirad 379) and 2-hydroxy-2-methyl-1-phenylpropanone (SpeedCure 97), used for curing at longer wavelengths under LED (365 nm upwards) are subject to reclassification.
Commercially available Esacure 3644 (IGM) is a ketocoumarin that is able to cure under LED wavelengths, but because it is a Type II photoinitiator it requires an amine for hydrogen abstraction to do so.

A further serious disadvantage of common radical photoinitiators (for example phosphine oxides such as 2,4,6-trimethylbenzoyl diphenylphosphine oxide (TPO)) is their susceptibility to oxygen inhibition and subsequent inability to surface cure. This results in tacky coatings and excessive yellowing within the coating due to additional passes under the lamp.

Thus, there is a need for photoinitiators that can cure ethylenically unsaturated monomers without additional components, that can achieve a tack-free surface cure and/or that can display some levels of photobleaching.

There is also a particular need for an initiating system that is active under LED wavelengths and preferably does not suffer from oxygen inhibition.

Further, the photoinitiators and their photoproducts need to impart minimal coloration to the finished product and show low volatility and low toxicity.

CN 116693487 discloses coumarin-based thioester photoinitiators having an aryl or heteroaryl side chain. Said photoinitiators are disclosed as suitable for LED photopolymerization.

There is therefore a need for adequately red-shifted photoinitiators which display satisfactory curing under a wide wavelength range of LED light in ethylenically unsaturated monomer formulations, some of which also show reduced or no oxygen inhibition effects.

### SUMMARY OF THE INVENTION

It is a first object of the invention to provide a photoinitiator according to formula (I), (II) or (III): 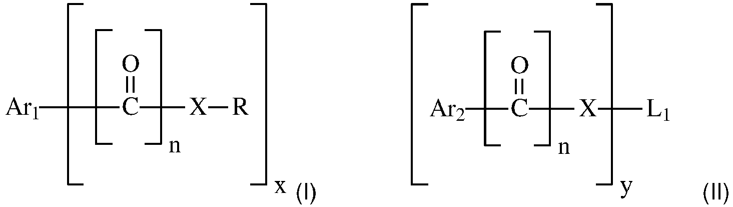 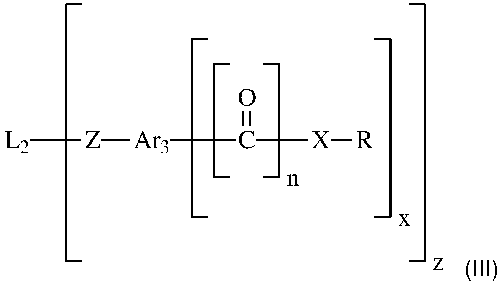 wherein n, x, y, z, X, Ar₁, Ar₂, Ar₃, R, Z, L₁ and L₂ are as defined herein.

It is a second object of the invention to provide a process for the preparation of a compound according to the invention, comprising
- reacting a compound of formula (A) with a compound of formula (B) to obtain a compound of formula (I); or
   reacting a compound of formula (C) with a compound of formula (D) to obtain a compound of formula (II); or
   reacting a compound of formula (E) with a compound of formula (B) to obtain a compound of formula (III): 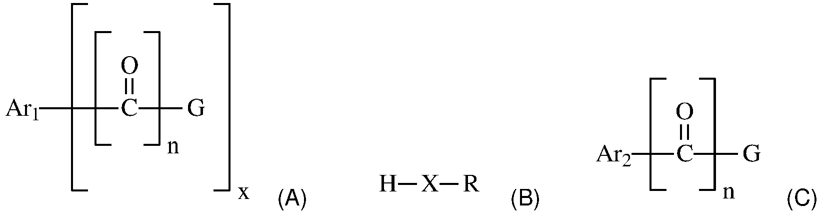 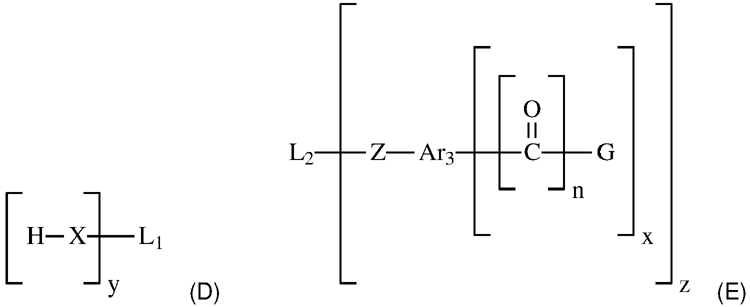
wherein
Ar₁, Ar₂, Ar₃, L₁, L₂, R, X, Z, G, n, x, y and z are as defined herein.

It is a third object to provide a curable composition comprising:
- one or more compounds of formula (I), (II) or (III) as defined herein; and
- one or more ethylenically unsaturated compounds.

In a fourth object, the invention also provides the use of a compound of formula (I), (II) or (III) as defined herein as a photoinitiator, in particular as a photoinitiator having photobleaching properties.

In a fifth object, the invention further provides a process for curing one or more ethylenically unsaturated compounds, said process comprising:
- mixing one or more ethylenically unsaturated compounds with a compound of formula (I), (II) or (III) as defined herein; and
- irradiating said mixture with at least one light source, preferably with at least one LED light source, more preferably with at least one LED light source having a maximum output wavelength in the range of 250 to 550 nm, in particular 250 to 460 nm, more particularly 340 to 430 nm.

In a sixth object, the invention also provides a cured product obtainable by curing the curable composition of the invention.

### DETAILED DESCRIPTION

The invention will now be described in more detail without limitation in the following description.

### Definitions

In the present application, the term "comprise(s) a/an" means "comprise(s) one or more".

Unless mentioned otherwise, the % by weight in a compound or a composition are expressed based on the weight of the compound, respectively of the composition.

The term "halogen" means an atom selected from C!, Br, F and I.

The term "alkyl" means a monovalent saturated acyclic hydrocarbon group of formula -CₙH₂ₙ₊₁ wherein n is 1 to 200. An alkyl may be linear or branched. A « (C₁-C₂₀) alkyl » means an alkyl having 1 to 20 carbon atoms. Examples of alkyl groups include methyl, ethyl, propyl, isopropyl, butyl, isobutyl, tert-butyl, pentyl, hexyl, 2-methylbutyl, 2,2-dimethylpropyl, n-hexyl, 2-methylpentyl, 2,2-dimethylbutyl, n-heptyl, 2-ethylhexyl, and the like.

The term "haloalkyl" means an alkyl as defined above wherein at least one hydrogen atom is replaced by a halogen atom (i.e. Cl, Br, I or F).

The term "perfluoroalkyl" means an alkyl as defined above wherein all of the hydrogen atoms are replaced by a fluorine atom.

The term "alkylene" means a linker derived from an alkyl by removing one hydrogen for each point of attachment of the linker.

The term "alkenyl" means a monovalent acyclic hydrocarbon group comprising one or more carbon-carbon double bonds. An alkenyl may be linear or branched. Examples of alkenyl groups include ethenyl, propenyl, butenyl, pentenyl, hexenyl, octenyl, and the like.

The term "aryl" means an optionally substituted polyunsaturated aromatic group. The aryl may contain a single ring (i.e. phenyl) or more than one ring wherein at least one ring is aromatic. When the aryl comprises more than one ring, the rings may be fused, linked via a covalent bond (for example biphenyl). The aromatic ring may optionally comprise one to two additional fused rings. The term "aryl" also encompasses aromatic groups comprising one or more heteroatoms independently selected from N, O and S as ring atoms. The term "aryl" also encompasses partially hydrogenated derivatives of the carbocyclic system is described above. Examples of aryl groups include phenyl, naphthyl, biphenyl, phenanthrenyl, pyrenyl, naphthacenyl, pyrimidinyl, pyrazinyl, pyridazinyl, pyridinyl (including 2-aminopyridine), triazinyl, furanyl, quinolyl, isoquinolyl, quinoxalinyl, quinazolinyl, thienyl, imidazolyl, thiazolyl, indolyl (such as indol-3-yl), pyrryl, oxazolyl, benzofuranyl, benzothienyl, benzopyranyl, benzopyranonyl, benzodioxolyl, pyrazolyl, benzothiazolyl, isoxazolyl, triazolyl (including 1,2,4-triazole, 1,2,3-triazole, and 5-amino-1,2,4-triazole), tetrazolyl, indazolyl, isothiazolyl, 1,2,4-thiadiazolyl, purinyl, carbazolyl, isoxazolyl, benzimidazolyl, indolinyl, pyranyl, pyrazolyl, triazolyl, oxadiazolyl (including 1,2,3-oxadiazole, 1,2,4-oxadiazole, 1,2,5-oxadiazole, 3-amino-1,2,4-oxadiazole, 1,3,4-oxadiazole), thianthrenyl, indolizinyl, isoindolyl, isobenzofuranyl, pyrrolyl, benzoxazolyl, xanthenyl, 2H-pyrrolyl, 3H-indolyl, 4H-quinolizinyl, phthalazinyl, acridinyl, naphthyridinyl, quinazolinyl, phenanthridinyl, pymiridinyl, phenanthrolinyl, phenazinyl, isothiazolyl, phenothiazinyl, isoxazolyl, furazanyl, phenoxazinyl, xanthonyl, thioxanthenyl, thioxanthonyl, dibenzofuranyl, dibenzothienyl, acridinyl, acridonyl, phenoxathiinyl, chromanyl, isochromanyl, anthraquinonyl, naphthoquinonyl, dibenzodioxinyl, groups, and the like.

The term "arylene" means a linker derived from an aryl by removing one hydrogen for each point of attachment of the linker.

The term "cycloalkyl" means a monovalent non-aromatic alicyclic group comprising one or more cycles. The term cycloalkyl also encompasses non-aromatic cyclic groups comprising one or more heteroatoms independently selected from N, O and S as ring atoms. Examples of cycloalkyl groups include cyclopentyl, cyclohexyl, cycloheptyl, isobornyl, 1-decalin, norbornyl, 5damantly, oxiranyl, oxetanyl, tetrahydrofuranyl, tetrahydropyranyl, dioxanyl, pyrrolidinyl, piperidinyl, piperazinyl, morpholinyl, imidazolidinyl, oxazolidinyl, dioxolanyl, and the like.

The term "cycloalkylene" means a linker derived from a cycloalkyl by removing one hydrogen for each point of attachment of the linker.

The term "alkoxy" means a group of formula -O-alkyl, wherein the alkyl is as defined above.

The term "aryloxy" means a group of formula -O-aryl, wherein the aryl is as defined above.

The term "linker" means a plurivalent group. A linker may connect at least two moieties of a compound together, in particular 2 to 16 moieties of a compound together. For example, a linker that connects two moieties of a compound together is referred to as a divalent linker, a linker that connects three moieties of a compound together is referred to as a trivalent linker, etc....

The term "hydrocarbon linker" means a linker having a carbon backbone chain which may optionally be interrupted by one or more heteroatoms selected from N, O, S, Si and mixtures thereof. A hydrocarbon linker may be aliphatic, cycloaliphatic or aromatic. A hydrocarbon linker may be saturated or unsaturated. A hydrocarbon linker may be optionally substituted.

The term "aliphatic" means a non-aromatic acyclic compound. It may be linear or branched, saturated or unsaturated. It may be substituted by one or more groups, for example selected from alkyl, hydroxyl, halogen (Br, Cl, I, F), isocyanate, carbonyl, amine, carboxylic acid, -C(=O)-OR', -C(=O)-O-C(=O)-R', each R' being independently a C₁-C₂₀ alkyl. It may comprise one or more bonds selected from ether, ester, amide, urethane, urea and mixtures thereof.

The term "acyclic" means a compound that does not comprise any rings.

The term "cycloaliphatic" means a non-aromatic cyclic compound. It may be substituted by one or more groups as defined for the term "aliphatic". It may comprise one or more bonds as defined for the term "aliphatic".

The term "aromatic" means a compound comprising an aromatic ring, which means that respects Hückel's aromaticity rule, in particular a compound comprising a phenyl group. It may be substituted by one or more groups as defined for the term "aliphatic". It may comprise one or more bonds as defined for the term "aliphatic".

The term "saturated" means a compound that does not comprise any double or triple carbon-carbon bonds.

The term "unsaturated" means a compound that comprises a double or triple carbon-carbon bond, in particular a double carbon-carbon bond.

The term "polyether linker" means a linker comprising at least two ether bonds.

The term "polythioether linker" means linker comprising at least two thioether bonds.

The term "polyester linker" means a linker comprising at least two ester bonds.

The term "polyorganosiloxane linker" means a linker comprising at least two organosiloxane bonds. The organosiloxane may, for example be a dimethylsiloxane bond.

The term "polybutadiene linker" means a linker comprising at least two units derived from the polymerization of butadiene, in particular at least two units selected from -CH₂-CH=CH-CH₂- and CH₂-CH(CH=CH₂)-.

The term "isocyanate group" means a -N=C=O group.

The term "isocyanurate linker" means a linker comprising an isocyanurate moiety, in particular a moiety of formula:

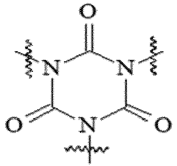

As used herein "optionally substituted" refers to unsubstituted groups or groups substituted with one or more substituents identical or different.

Unless mentioned otherwise, optional substituents of alkyl and alkenyl groups can be independently selected from the group consisting of: -OH, -SH, halogen, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are independently selected from H, linear or branched -(C₁-C₂₀)alkyl and an optionally substituted -(C₅-C₁₂)aryl.

Unless mentioned otherwise, optional substituents of aryl and cycloalkyl can be independently selected from the group consisting of: -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are independently selected from H, linear or branched -(C₁-C₂₀)alkyl and an optionally substituted -(C₅-C₁₂)aryl.

### Compound of formula (I), (II) or (III)

The invention relates to a compound of formula (I), (II) or (III): 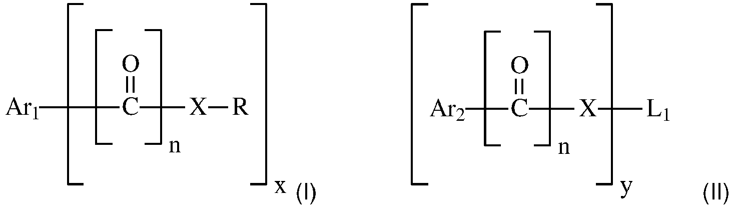 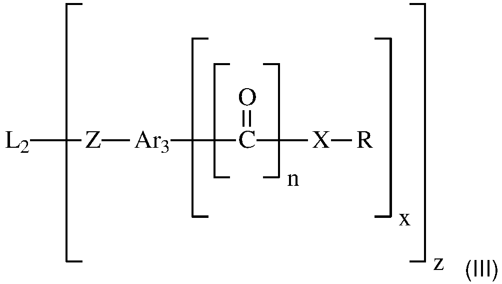 wherein:
each n is independently 1 or 2;
each x is independently an integer from 1 to 6;
each y and z is independently an integer from 2 to 6;
each X is independently S, Se or Te;
each Ar₁, Ar₂ and Ar₃ is independently an optionally substituted aryl provided that:
   - each -[C(=O)]ₙ-X-R moiety is directly connected to an aromatic ring having 6 carbon ring atoms;
   - when Ar₁ is an optionally substituted phenyl then n is equal to 2;
each R is independently chosen from the group consisting of:
   - an optionally substituted linear or branched -(C₁-C₂₀)alkyl, said alkyl being optionally interrupted by one or more groups chosen from -O-, -C(=O)- and -S-;
   - an optionally substituted linear or branched -(C₂-C₂₀)alkenyl, said alkenyl being optionally interrupted by one or more groups chosen from -O- and -C(=O)-;
   - an optionally substituted -(C₅-C₁₂)aryl; and
   - an optionally substituted -(C₃-C₁₂)cycloalkyl said cycloalkyl being optionally interrupted by one or more heteroatoms independently chosen from O, N and S;
each Z is independently a bond, -CH₂-, -O-, -S-, -NR¹-, -C(=O)-O-, -O-C(=O)-, -C(=O)-S-, -S-C(=O)-, -C(=O)-NR'-, -NR¹-C(=O)-, -O-C(=O)-NR¹-, -NR'-C(=O)-O-, -O-C(=O)-O-, -NR¹-C(=O)-NR¹-, wherein each R¹ is independently H, a linear or branched -(C₁-C₆)alkyl or a -(C₅-C₁₂)aryl;
L₁ is a y-valent linker; and
L₂ is a z-valent linker.

The preferred embodiments hereafter can be considered singly or combined with each other when applicable, and can be applied to formula (I), (II) or (III) and any one of the formulae described hereafter:
In formula (I), (II) and (III), each Ar₁, Ar₂ and Ar₃ is independently an optionally substituted aryl. As used herein, the term "optionally substituted aryl" means an aryl optionally substituted by one or more groups selected from -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ, and -C(=O)NR_{d}Rₑ, wherein Rₐ, R_{b}, R_{d} and Rₑ identical or different are independently chosen from H and linear or branched -(C₁-C₂₀)alkyl, and R_{c} is chosen from linear or branched -(C₁-C₂₀)alkyl and -(C₅-C₁₂)aryl optionally substituted by one or more of -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -NR_{d}Rₑ, -NO₂ and -CN.

According to an embodiment, each Ar₁, Ar₂ and Ar₃ is independently an optionally substituted aryl selected from phenyl, naphthyl, anthracenyl, pyrenyl, benzodioxolyl, thioxanthonyl, anthraquinonyl and naphthoquinonyl.

According to an embodiment, the compound is according to formula (I) and Ar₁ is an aryl according to any one of the following formulae (Ar₁-a) to (Ar₁-g): 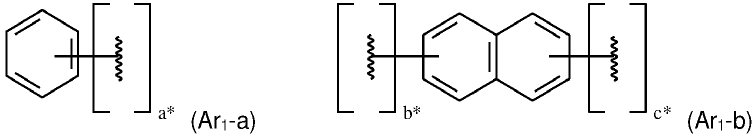 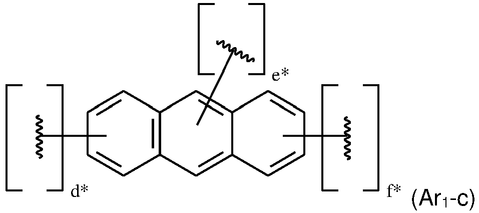 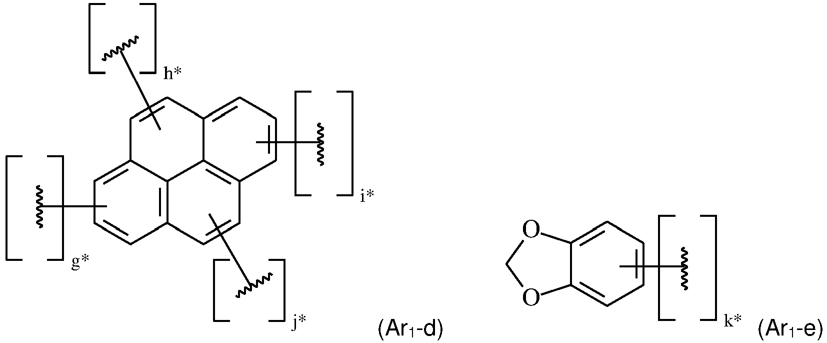 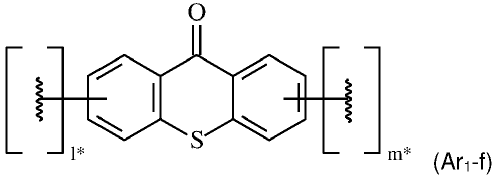 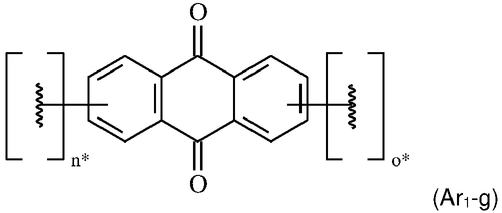 wherein
a* is an integer from 1 to 4 and a* = x;
b* and c* are independently an integer from 0 to 2 and b* + c* = x;
d*, e* and f* are independently an integer from 0 to 2 and d* + e* + f* = x;
g*, h*, i* and j* are independently an integer from 0 to 2 and g* + h* + i* + j* = x;
k* is an integer from 1 to 2 and k* = x;
l* and m* are independently an integer from 0 to 2 and l* + m* = x;
n* and o* are independently an integer from 0 to 2 and n* + o* = x;
each symbol 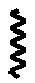 represents a point of attachment to a -[C(=O)]ₙ-X-R moiety; and
each aryl is independently optionally substituted by one or more groups selected from -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ, and -C(=O)NR_{d}Rₑ, wherein Rₐ, R_{b}, R_{c}, R_{d} and Rₑ are as defined above.

In a preferred embodiment, Ar₁ is an aryl according to formula (Ar₁-a) or (Ar₁-f). In a particularly preferred embodiment, Ar₁ is an aryl according to formula (Ar₁-a) wherein a* is 1 or Ar₁ is an aryl according to formula (Ar₁-f) wherein l* is 0 and m* is 1.

According to another embodiment, the compound is according to formula (II) and Ar₂ is an aryl according to one of the following formulae (Ar₂-a) to (Ar₂-h): 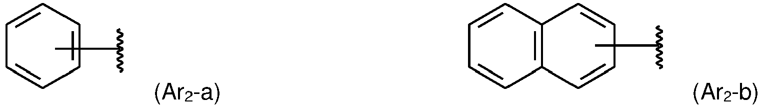 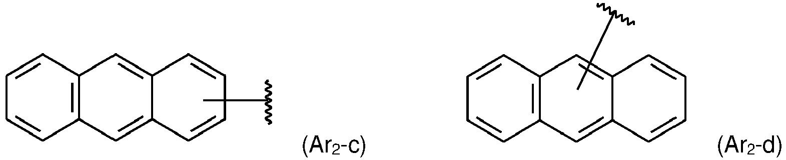 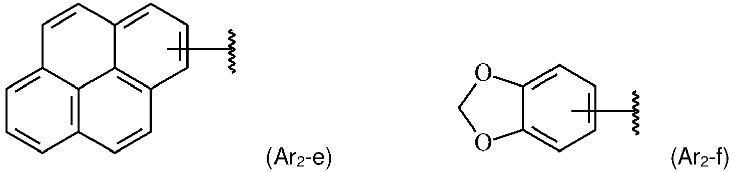 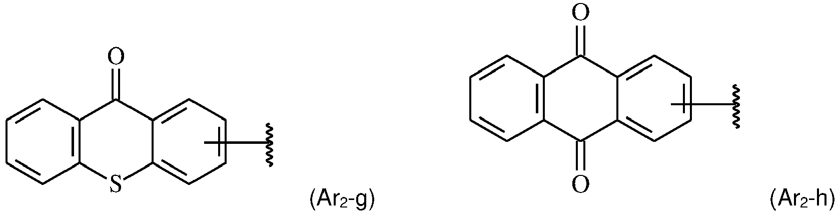 wherein
each symbol 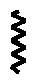 represents a point of attachment to a -[C(=O)]ₙ-X-R moiety; and
each aryl is independently optionally substituted by one or more groups selected from -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ, and -C(=O)NR_{d}Rₑ, wherein Rₐ, R_{b}, R_{c}, R_{d} and Rₑ are as defined above.

In a preferred embodiment, Ar₂ is an aryl according to formula (Ar₂-a) or (Ar₂-g).

According to another embodiment, the compound is according to formula (III) and Ar₃ is an aryl according to one of the following formulae (Ar₃-a) to (Ar₃-h): 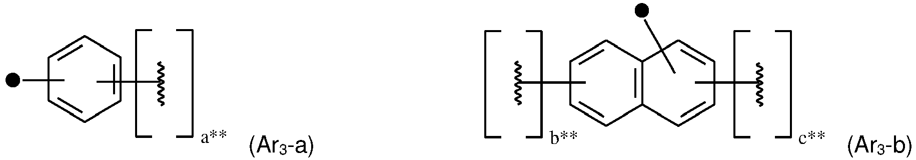 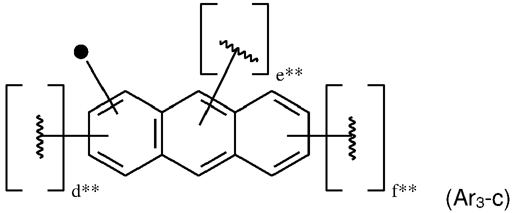 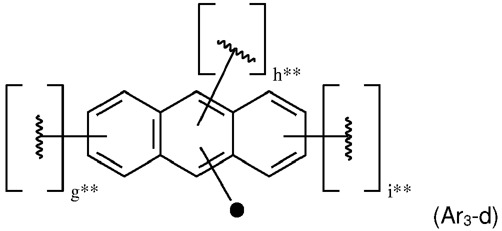 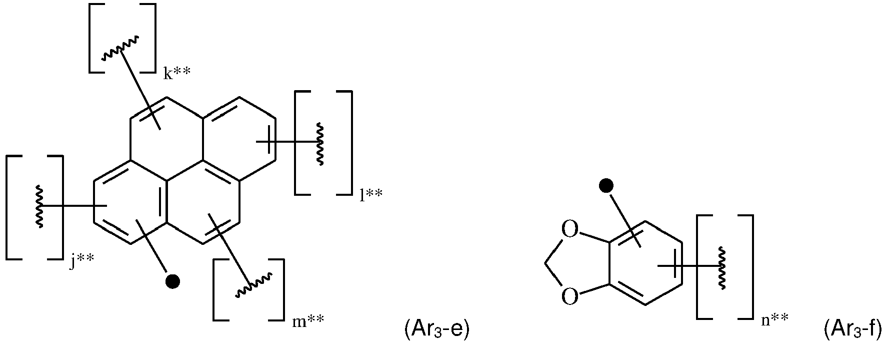 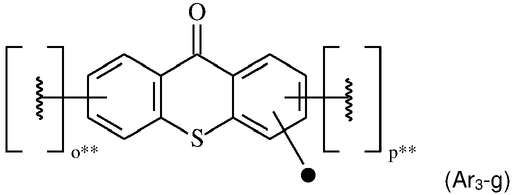 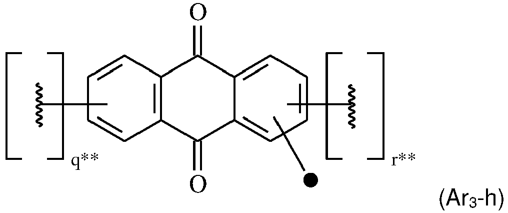 wherein
a** is an integer from 1 to 4 and a** = x;
b** and c** are independently an integer from 0 to 2 and b** + c** = x;
d**, e** and f** are independently an integer from 0 to 2 and d** + e** + f** = x;
g**, h** and i** are independently an integer from 0 to 2 and g** + h** + i** = x;
j**, k**, l** and m** are independently an integer from 0 to 2 and j** + k** + l** + m** = x;
n** is an integer from 1 to 2 and n** = x;
o** and p** are independently an integer from 0 to 2 and o** + p** = x;
q** and r** are independently an integer from 0 to 2 and q** + r** = x;
each symbol 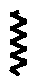 represents a point of attachment to a -[C(=O)]ₙ-X-R moiety;
each symbol ● represents a point of attachment to a L-Z- moiety;
each aryl is independently optionally substituted by one or more groups selected from -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ, and -C(=O)NR_{d}Rₑ wherein Rₐ, R_{b}, R_{c}, R_{d} and Rₑ are as defined above.

In a preferred embodiment, Ar₃ is an aryl according to formula (Ar₃-a) or (Ar₃-g). In a particularly preferred embodiment, Ar₃ is an aryl according to formula (Ar₃-a) wherein a** is 1 or Ar₃ is an aryl according to formula (Ar₃-g) wherein o** is 0 and p** is 1 or wherein o** is 1 and p** is 0.

In formula (II), L₁ is a y-valent linker. In formula (III), L₂ is a z-valent linker.

In particular, L₁ and L₂ may independently be a divalent, trivalent, tetravalent, pentavalent or hexavalent linker. Even more particularly, L₁ and L₂ may independently be a divalent, trivalent or tetravalent linker.

More particularly, L₁ and L₂ may independently be selected from an aromatic linker, an aliphatic linker, a cycloaliphatic linker, a polyether linker, a polythioether linker, a polyester linker, a polyorganosiloxane linker, a polybutadiene linker, and combinations thereof.

According to an embodiment, L₁ and L₂ are independently selected from:
- a divalent moiety according to any one of formulae (L1) to (L7):

   -CH₂-CH₂-O-CH₂-O-CH₂-CH₂-[S-S-CH₂-CH₂-O-CH₂-O-CH₂-CH₂]_{a*}- (L0)

   -(CR⁶R⁷)ₐ- (L1)

   -[(CR⁸R⁹)_{b}-W]_{c}-(CR⁸R⁹)_{b}- (L2)

   -[(CR¹⁰R¹¹)_{d}-W]ₑ-(CR¹²R¹³)_{f}-[W-(CR¹⁰R¹¹)_{d}]ₑ- (L3)

   -[(CR¹⁴R¹⁵)_{g}-O-C(=O)-(CR¹⁶R¹⁷)ₕ-C(=O)-O]ᵢ-(CR¹⁴R¹⁵)_{g}- (L4)

   -(CR¹⁸R¹⁹)ⱼ-C(=O)-O-(CR²⁰R²1)ₖ-O-C(=O)-(CR¹⁸R¹⁹)ⱼ- (L5)

   -(CR²²R²³)ₗ-Cy-[_{A}-Cy]ₒ-(CR²²R²³)ₗ- (L6)

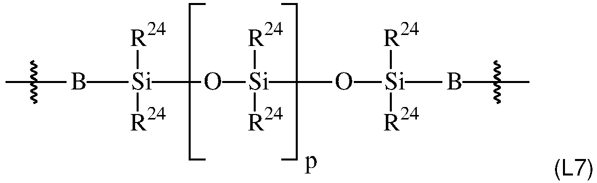

   wherein:
   - R⁶, R⁷, R¹², R¹³, R¹⁴, R¹⁵, R¹⁶, R¹⁷, R¹⁸, R¹⁹, R²⁰, R²¹, R²² and R²³ are independently H or alkyl;
   - R⁸, R⁹, R¹⁰ and R¹¹ are independently H or methyl;
   - each R²⁴ is independently alkyl, haloalkyl, alkenyl, cycloalkyl, aryl, aralkyl, alkaryl, alkoxy or aryloxy;
   - each W is independently O or S;
   - each Cy is an optionally substituted ring, in particular an optionally substituted arylene or an optionally substituted cycloalkylene;
   - A is a bond or a linker such as -O-, -S-, Alk, -C(=O)-, -C(=O)-O-Alk-O-C(=O)-, -SO-, -SO₂-, -C(=CCl₂)- and -Alk-Ph-Alk-;
   - each Alk is independently an optionally substituted alkylene;
   - Ph is an optionally substituted phenylene;
   - each B is independently a bond or a hydrocarbon linker which is optionally interrupted by one or more functional groups selected from -O-, -(C=O)-, -(C=O)-O- and -O-(C=O)- ;
   - a, f, g, h and k are independently an integer from 2 to 20;
   - each I is independently an integer from 0 to 20;
   - b and d are independently an integer from 2 to 4;
   - o is an integer equal to 0 or 1;
   - c, i and j are independently an integer from 1 to 20;
   - each e is independently an integer from 0 to 20 with the proviso that at least one e is not 0;
   - p is 0 to 100;
- a trivalent moiety according to any one of formulae (L8) to (L11): 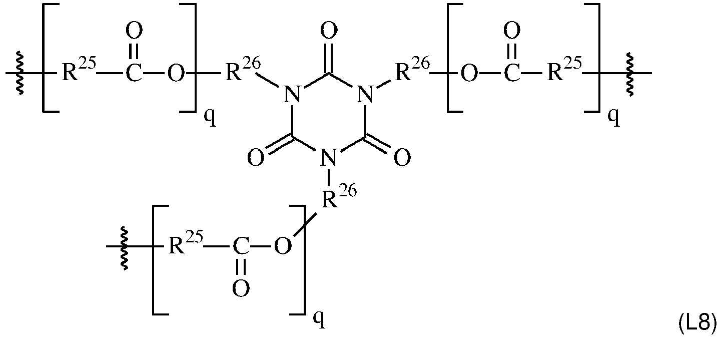 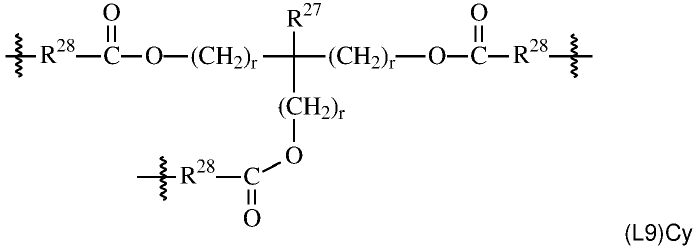 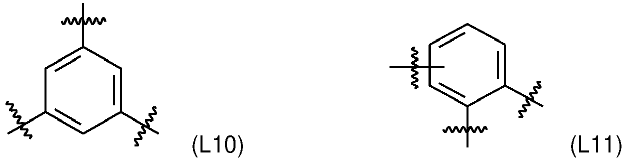 wherein:
   - each R²⁵ and R²⁶ is independently an alkylene;
   - R²⁷ is H, alkyl or alkoxy, preferably R²⁷ is H or alkyl;
   - each R²⁸ is independently an alkylene;
   - each q is independently an integer equal to 0 or 1;
   - each r is independently an integer from 0 to 2 with the proviso that not more than one r is equal to 0, preferably each r is equal to 1 or one r is equal to 0 and the two other r are equal to 1;
- a tetravalent moiety according to formula (L12) or (L13): 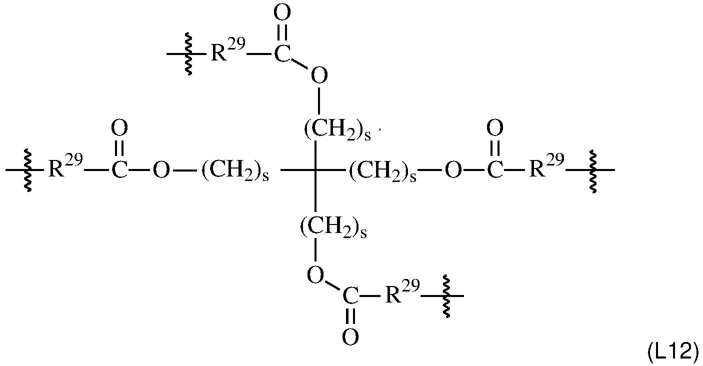 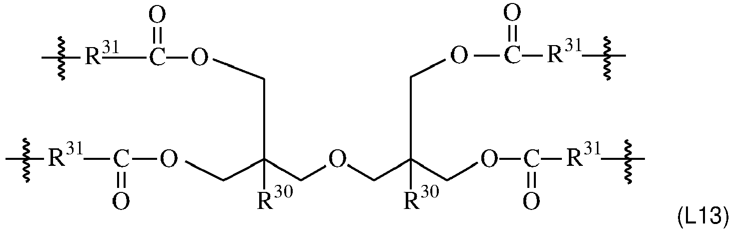 wherein:
   - each R²⁹ and R³¹ is independently alkylene;
   - each R³⁰ is independently H, alkyl or alkoxy, preferably R³⁰ is alkyl;
   - each s is independently an integer from 0 to 2 with the proviso that not more than one s is equal to 0, preferably each s is equal to 1;
- a hexavalent moiety according to formula (L14): 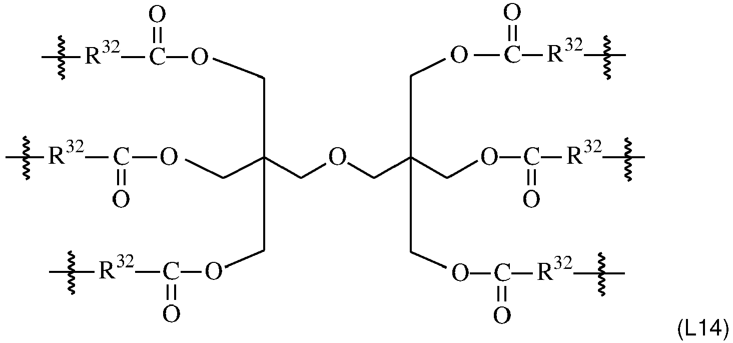 wherein each R³² is independently a linear or branched alkylene;
where
alkyl refers to a linear or branched -(C₁-C₂₀)alkyl,
alkylene refers to a linear or branched -(C₁-C₂₀)alkylene-,
alkoxy refers to linear or branched -O(C₁-C₂₀)alkyl;
aryl refers to a -(C₅-C₁₂)aryl;
alkenyl refers to a linear or branched -(C₂-C₂₀)alkenyl;
cycloalkyl refers to a -(C₃-C₁₂)cycloalkyl;
alkaryl refers to a -(C₅-C₁₂)-arylene-(C₁-C₂₀)alkyl;
aralkyl refers to a -(C₁-C₂₀)-alkylene-(C₅-C₁₂)aryl;
"optionally substituted" refers to none, or one or more substituents chosen from -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, where Rₐ, R_{b}, R_{c}, R_{d} and Rₑ are defined above.

More particularly still, L₁ and L₂ may independently be the residues of a polythiol (i.e. the residue that is obtained by removing the SH groups of a polythiol). Examples of suitable polythiols, also referred to herein as P_{SH}, include ethane-1,2-dithiol, propane-1,3-dithiol, butane-1,4-dithiol, hexane-1,6-dithiol, octane-1,8-dithiol, decane-1,10-dithiol, 1,8-dimercapto-3,6-dioxaoctane (DMDO), di-, tri- or polyethylene glycol di(ethanethiol), the reaction product of a polyol and a mercapto-functionalized carboxylic acid such as thioglycolic acid, 3-mercaptopropionic acid or 3-mercaptobutyric acid (available as Thiocure^{®} from Bruno Bock or as Karenz^{®} MT from Showa Denko) such as ethylene glycol bis(mercaptoacetate), ethylene glycol bis(3-mercaptopropionate), ethylene glycol bis(3-mercaptobutyrate), 1,2-propylene glycol bis(mercaptoacetate), 1,2-propylene glycol bis(3-mercaptopropionate), 1,2-propylene glycol bis(3-mercaptobutyrate), 1,3-propylene glycol bis(mercaptoacetate), 1,3-propylene glycol bis(3-mercaptopropionate), 1,3-propylene glycol bis(3-mercaptobutyrate), 1,4-butanediol bis(mercaptoacetate), 1,4-butanediol bis(3-mercaptopropionate), 1,4-butanediol bis(3-mercaptobutyrate), 1,6-hexanediol bis(mercaptoacetate), 1,6-hexanediol bis(3-mercaptopropionate), 1,6-hexanediol bis(3-mercaptobutyrate), di-, tri- or polyethylene glycol bis(mercaptoacetate), di-, tri- or polyethylene glycol bis(3-mercaptopropionate), di-, tri- or polyethylene glycol bis(3-mercaptobutyrate), di-, tri- or polypropylene glycol bis(mercaptoacetate), di-, tri- or polypropylene glycol bis(3-mercaptopropionate), di-, tri- or polypropylene glycol bis(3-mercaptobutyrate), trimethylolpropane tris(mercaptoacetate), trimethylolpropane tris(3-mercaptopropionate), trimethylolpropane tris(3-mercaptobutyrate), glycerol tris(mercaptoacetate), glycerol tris(3-mercaptopropionate), glycerol tris(3-mercaptobutyrate), pentaerythritol tetrakis(3-mercaptopropionate), pentaerythritol tetrakis(3-mercaptobutyrate), dipentaerythritol hexakis(mercaptoacetate), dipentaerythritol hexakis(3-mercaptopropionate), dipentaerythritol hexakis(3-mercaptobutyrate) and pentaerythritol tetrakis(mercaptoacetate), cyclohexane dithiol, cyclohexyl dimethanethiol, dipentene dimercaptan, tris(3-mercaptopropyl) isocyanurate, tris[2-(3-mercaptopropionyloxy)ethyl] isocyanurate, tris[2-(3-mercaptobutyryloxy)ethyl] isocyanurate, mercaptan-terminated polymers (such as Capcure^{®} 3-800 (BASF), GPM-800 (Gabriel Performance Products), Capcure^{®} LOF (BASF), GPM-800LO (Gabriel Performance Products),), a mercapto-functionalized polyorganosiloxane (such as Silmer SH JO, Silmer SH Q20 and Silmer SH 208-30Q available from Siltech), polysulfide polymers (such as Thiokol^{®} LP available from Toray), as well as the alkoxylated (e.g., ethoxylated and/or propoxylated) derivatives thereof, and combinations thereof.

In formulae (I) and (III), each R is independently chosen from the group consisting of:
- an optionally substituted linear or branched -(C₁-C₂₀)alkyl, said alkyl being optionally interrupted by one or more groups chosen from -O-, -C(=O)- and -S-;
- an optionally substituted linear or branched -(C₂-C₂₀)alkenyl;
- an optionally substituted -(C₅-C₁₂)aryl; and
- an optionally substituted -(C₃-C₁₂)cycloalkyl said cycloalkyl being optionally interrupted by one or more heteroatoms independently chosen from O, N and S.

In a preferred embodiment, R is an optionally substituted -(C₅-C₁₂)aryl. As used herein, the term "optionally substituted -(C₅-C₁₂)aryl" means a (C₅-C₁₂)aryl which is optionally substituted by one or more groups chosen from OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

In particular, R may be a -(C₅-C₁₂)aryl selected from phenyl, naphthyl, furanyl, thiophenyl, pyrrolyl and pyridinyl, all of which may be optionally substituted by one or more groups as defined above for the -(C₅-C₁₂)aryl.

More particularly, R may be a phenyl or naphthyl, said phenyl or naphthyl being optionally substituted by one or more groups as defined above for the optionally substituted -(C₅-C₁₂)aryl.

Even more particularly, R may be a phenyl or a naphthyl, said phenyl or naphthyl being optionally substituted by one or more groups selected from halogen, -CF₃, -NO₂, -CN, -(C₅-C₁₂)aryl, linear or branched -(C₁-C₂₀)alkyl group, linear or branched -O(C₁-C₂₀)alkyl group, linear or branched -S(C₁-C₂₀)alkyl group, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above, preferably Rₐ, R_{b}, R_{c}, R_{d} and Rₑ are independently a linear or branched -(C₁-C₂₀)alkyl.

More particularly still, R may be the residue of an aromatic monothiol (i.e the residue that is obtained by removing the SH group of an aromatic monothiol). Examples of suitable aromatic thiols, also referred to herein as M_{SH-Ar}, include thiophenol, 1- or 2-naphthalenethiol, 2-, 3- or 4-methylbenzenethiol, 2-, 3- or 4-isopropylbenzenethiol, 2-, 3- or 4-tert-butylbenzenethiol, 2-, 3- or 4-methoxybenzenethiol, 2-, 3- or 4-chlorobenzenethiol, 2-, 3- or 4-bromobenzenethiol, 2-, 3- or 4-fluorobenzenethiol, 2-, 3- or 4-nitrobenzenethiol, 2-, 3- or 4-cyanobenzenethiol, 2-, 3- or 4-trifluoromethylbenzenethiol, 2-, 3- or 4-methylthiobenzenethiol, methyl 2-, 3- or 4-mercaptobenzoate, 2,4,6-trimethylbenzenethiol, 2-, 3- or 4-(dimethylamino)benzenethiol and 2-, 3- or 4-(diethylamino)benzenethiol.

In an alternative embodiment, R is selected from:
- an optionally substituted linear or branched -(C₁-C₂₀)alkyl, said alkyl being optionally interrupted by one or more groups chosen from -O-, -C(=O)- and -S-;
- an optionally substituted linear or branched -(C₂-C₂₀)alkenyl, said alkenyl being optionally interrupted by one or more groups chosen from -O- and -C(=O)-; and
- an optionally substituted -(C₃-C₁₂)cycloalkyl, said cycloalkyl being optionally interrupted by one or more heteroatoms independently chosen from O, N and S;

As used herein, the term "optionally substituted -(C₁-C₂₀)alkyl" refers to a -(C₁-C₂₀)alkyl which is optionally substituted by one or more groups chosen from -OH, -SH, halogen, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -NO₂, -CN, -CF₃, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

As used herein, the term "optionally substituted -(C₂-C₂₀)alkenyl" refers to a -(C₂-C₂₀)alkenyl which is optionally substituted by one or more groups chosen from -OH, -SH, halogen, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -NO₂, -CN, -CF₃, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

As used herein, the term "optionally substituted -(C₃-C₁₂)cycloalkyl" refers to a -(C₃-C₁₂)cycloalkyl which is optionally substituted by one or more groups chosen from -OH, -SH, halogen, linear or branched -(C₁-C₂₀)alkyl group, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₅-C₁₂)aryl, -O(C₅-C₁₂)aryl, -S(C₅-C₁₂)aryl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

In particular, R may be chosen from a linear or branched -(C₁-C₂₀)alkyl, cyclopentyl, cyclohexyl, tetrahydrofuranyl, methyltetrahydrofuranyl and tetrahydropyranyl, said alkyl being optionally interrupted by one or more groups chosen from -O- and -S-, said alkyl being optionally substituted by one or more groups chosen from -(C₅-C₁₂)aryl, -C(=O)ORₐ and -NR_{d}Rₑ wherein Rₐ, R_{d} and Rₑ are as defined above, said cyclohexyl being optionally substituted by one or more groups chosen from the group consisting of -OH, -SH, halogen, linear or branched -(C₁-C₂₀)alkyl group, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₅-C₁₂)aryl, -O(C₅-C₁₂)aryl, -S(C₅-C₁₂)aryl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

More particularly, R may be the residue of a monothiol (i.e the residue that is obtained by removing the SH group of a monothiol). Examples of suitable monothiols, also referred to herein as M_{SH-2}, include methyl mercaptan, ethyl mercaptan, n-propyl mercaptan, isopropyl mercaptan, n-butyl mercaptan, sec-butyl mercaptan, tert-butyl mercaptan, n-pentyl mercaptan, n-hexyl mercaptan, 2-ethylhexyl mercaptan, n-octyl mercaptan, n-nonyl mercaptan, tert-nonyl mercaptan, n-decyl mercaptan, n-dodecyl mercaptan, tert-dodecyl mercaptan, n-hexadecyl mercaptan, polyethylene glycol) methyl ether thiol, 2-(dimethylamino)ethanethiol, allyl mercaptan, cyclohexyl mercaptan, cyclohexyl methanethiol, tetrahydro-2H-pyran-4-thiol, furan-2-ylmethanethiol, tetrahydrofuran-3-thiol, 2-methyltetrahydrofuran-3-thiol, benzyl mercaptan, 1-phenylethyl mercaptan, 2-phenylethanethiol, methyl thioglycolate, ethyl thioglycolate, butyl thioglycolate, 2-ethylhexyl thioglycolate, isooctyl thioglycolate, isotridecyl thioglycolate, methyl 3-mercaptopropionate, ethyl 2-mercaptopropionate, ethyl 3-mercaptopropionate, ethyl 3-mercaptobutyrate, isopropyl 3-mercaptopropionate, butyl 3-mercaptopropionate, 3-methoxybutyl 3-mercaptopropionate 2-ethylhexyl 3-mercaptopropionate, isotridecyl 3- mercaptopropionate, octadecyl 3-mercaptopropionate, and mixtures thereof.

In formulae (I), (II) and (III), each X is independently S, Se or Te. Preferably, each X is S.

According to a preferred embodiment, the compound is according to formula (Ia): 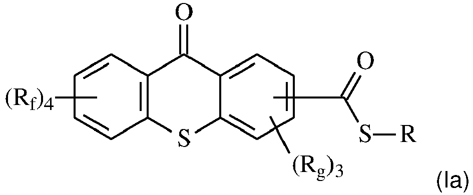 wherein
R is as defined above; and
each R_{f} and R_{g} is independently selected from H, -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

According to another preferred embodiment, the compound is according to formula (Ib): 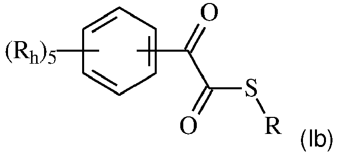 wherein
R is as defined above; and
each Rₕ is independently selected from H, -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

According to another preferred embodiment, the compound is according to formula (Ic): 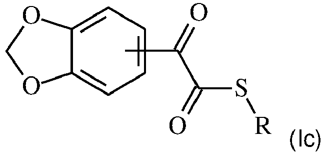 wherein R is as defined above.

According to another preferred embodiment, the compound is according to formula (IIa): 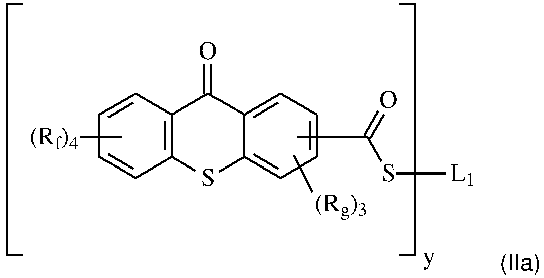 wherein
y is 2 to 4;
L₁ is as defined above; and
each R_{f} and R_{g} is independently selected from H, -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

According to another preferred embodiment, the compound is according to formula (IIb): 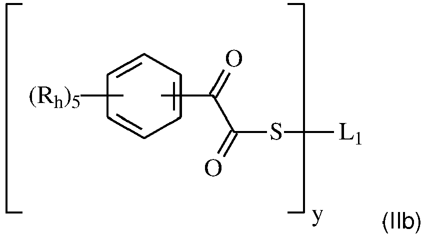 wherein
y is 2 to 4;
L₁ is as defined above; and
each Rₕ is independently selected from H, -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

In a particularly preferred embodiment, the compound of formula (I), (II) or (III) is according to one of the following structures:

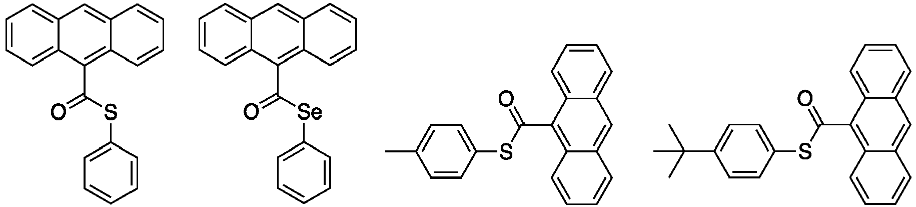

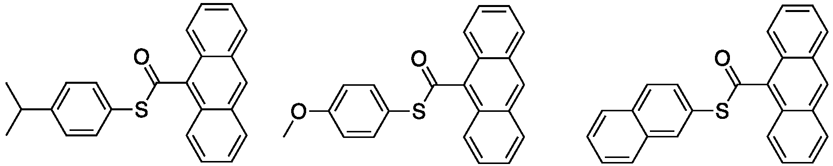

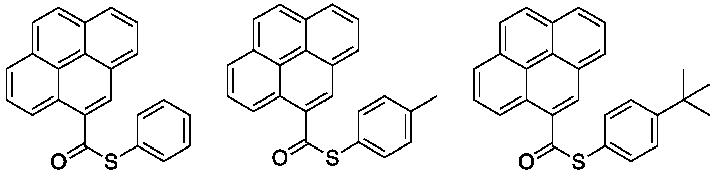

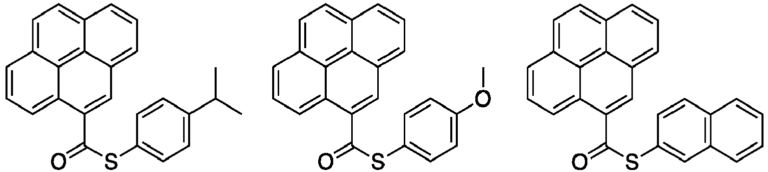

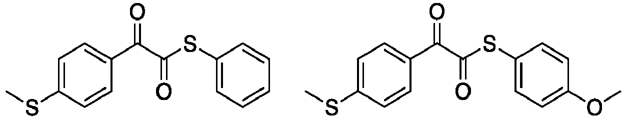

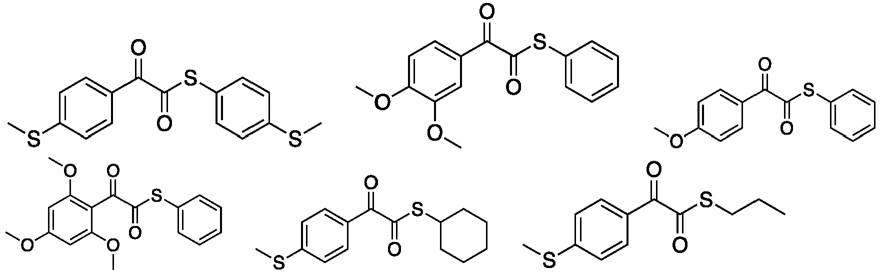

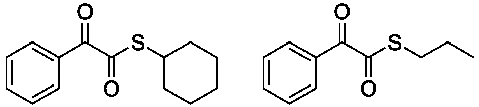

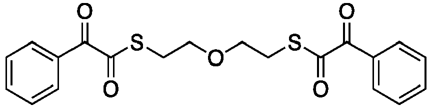

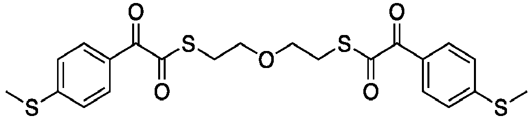

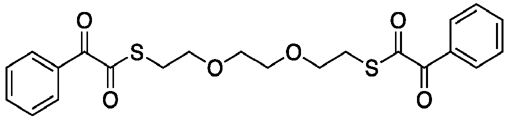

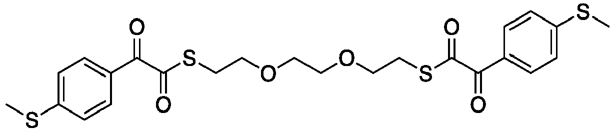

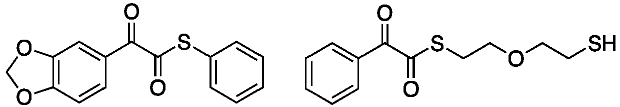

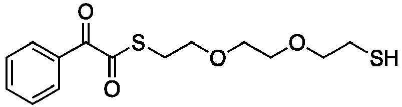

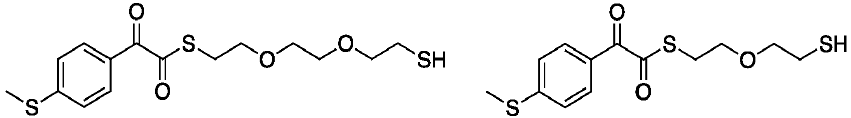

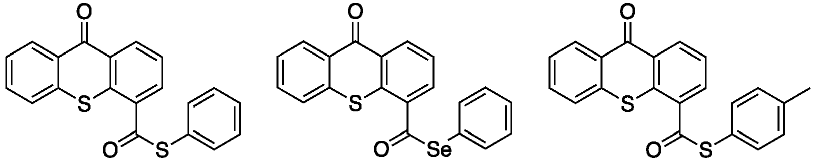

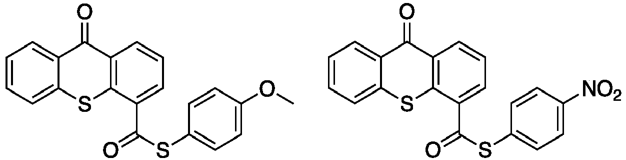

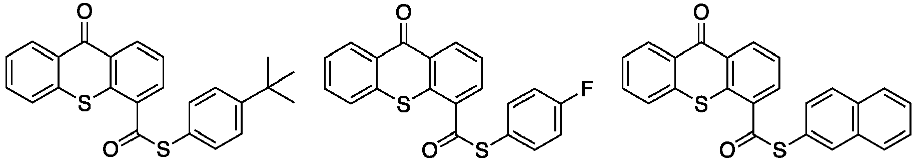

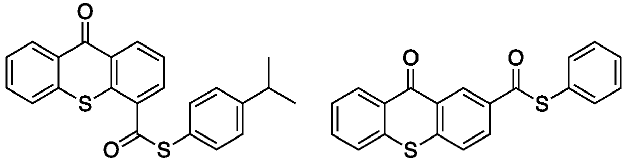

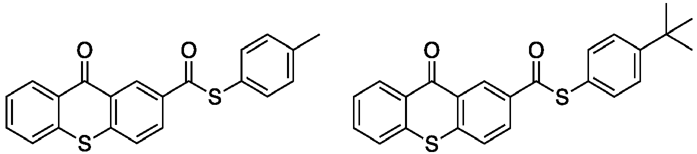

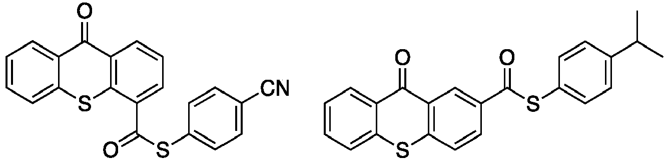

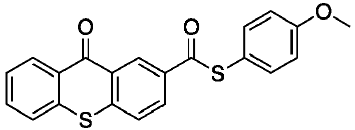

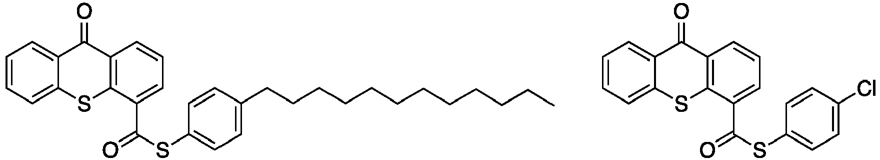

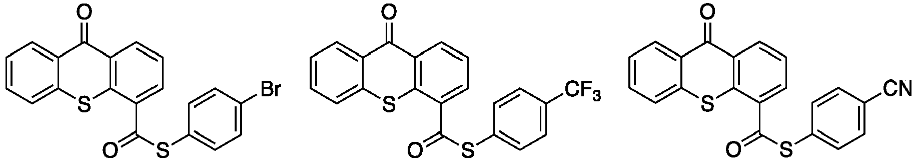

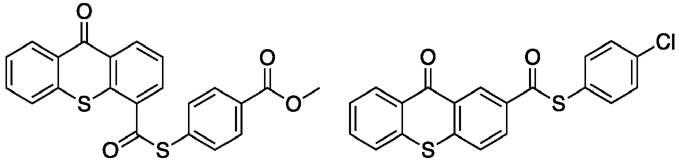

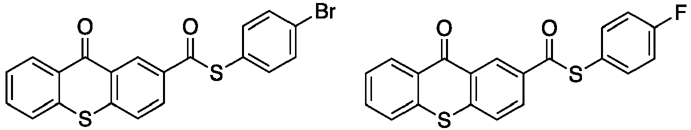

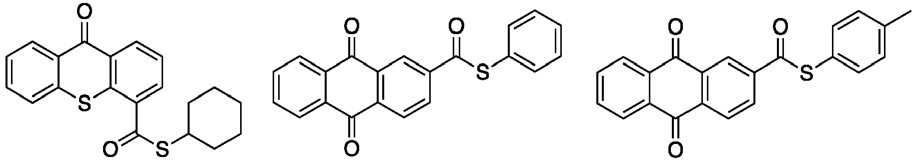

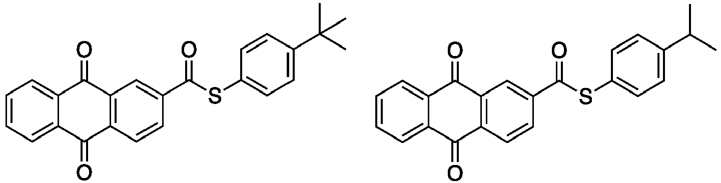

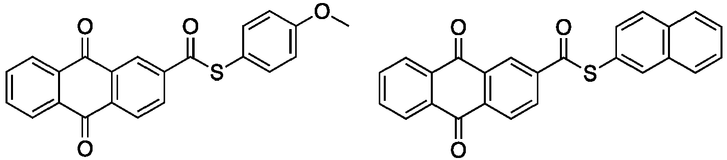

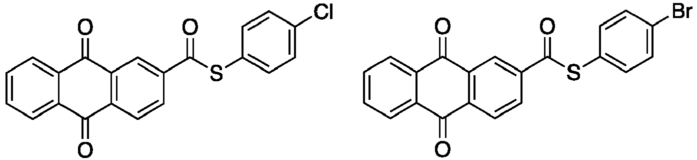

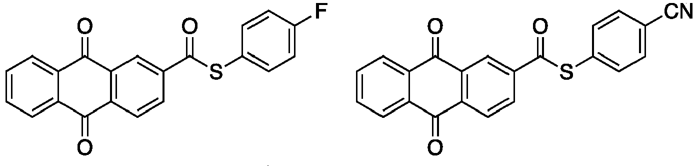

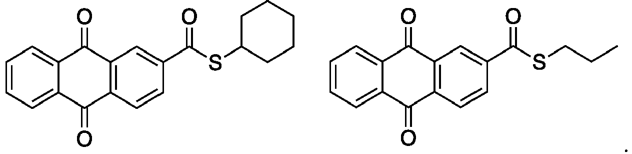

Advantageously, the compounds of formula (I), (II) or (III) according to the present invention may be synthesized from readily available commercial reagents. They have been demonstrated to be stable when formulated and have excellent solubility in organic solvents and various monomers.

While not wishing to be bound by any particular theory, the compounds of formula (I), (II) or (III) according to the present invention may be capable of initiating cure of compositions containing (meth)acrylate-functionalized compounds (via free radical polymerization), cyanoacrylate-functionalized compounds (via anionic polymerization), and mixtures of both types of compounds.

This ability enables a wide formulation latitude and is particularly advantageous when formulating for applications where specific physical properties of the cured material are desired.

### Process for the preparation of a compound of formula (I), (II) or (III)

The invention also relates to a process for the preparation of a compound of formula (I), (II) or (III) as defined herein, comprising
- reacting a compound of formula (A) with a compound of formula (B) to obtain a compound of formula (I); or
- reacting a compound of formula (C) with a compound of formula (D) to obtain a compound of formula (II); or
- reacting a compound of formula (E) with a compound of formula (B) to obtain a compound of formula (III): 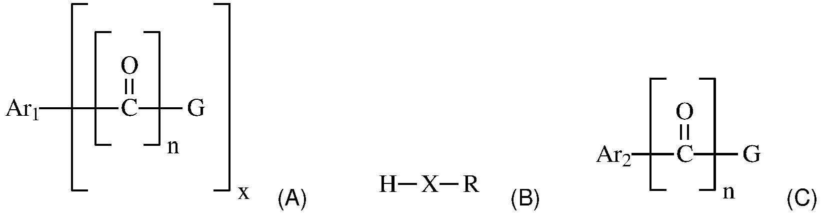 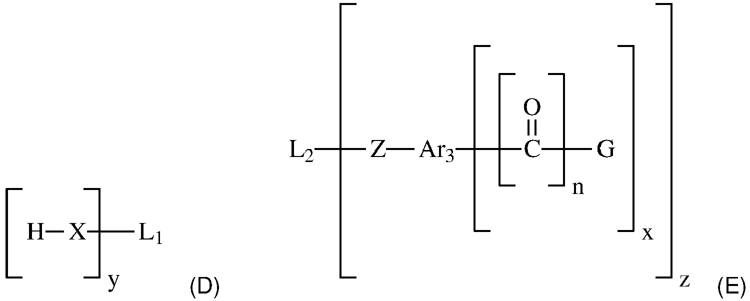
wherein
Ar₁, Ar₂, Ar₃, L₁, L₂, R, X, Z, n, x, y and z are as defined herein;
each G is independently OH, a halogen atom or -O-C(=O)-J;
J is alkyl or aryl, in particular tert-butyl.

According to an embodiment, compound (A) or (E) is reacted with compound (B) when R represents a linear or branched -(C₁-C₂₀)alkyl or a -(C₃-C₁₂)cycloalkyl in Formula (I).

Typically, the reaction of a compound of formula (A) and (E) typically where G is halogen, with a compound of formula (B) may be carried out in an organic solvent, preferably a non-polar aprotic solvent such as THF, in the presence of a base, preferably a weak organic base such as triethylamine.

The reaction may be conducted in a wide range of temperature, from -20°C to the reflux temperature of the reactional mixture.

Typically, the reaction of a compound of formula (C) typically where G is OH with a compound of formula (D) may be carried out in an organic solvent, preferably a non-polar aprotic solvent such as dichloromethane, in the presence of an activating agent of carboxylic acids, such as N,N'-Dicyclohexylcarbodiimide (DCC) in the presence of a nucleophilic catalyst, such as 4-Dimethylaminopyridine (DMAP).

The reaction may be conducted in a wide range of temperature, from -20°C to the reflux temperature of the reactional mixture.

The process for the preparation of a compound of formula (I) or (III) where n=2 may also comprise reacting a compound of formula (A) or (E) where n=1 and G is alkyl, such as methyl with a compound of formula (B) in the presence of K₂S₂O₈.

The process for the preparation of a compound of formula (I) or (III) where n=2 may also comprise reacting a compound of formula (A) or (C) where n=2 and G is OH with a compound of formula (B) or with a compound of formula (F): 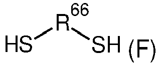 wherein R⁶⁶ is a linear or branched -(C₁-C₆) alkyl, said alkyl being optionally interrupted by one or more groups chosen from -O- and -C=O-.

According to an embodiment, the process of the invention may also comprise the step of preparing intermediates of formula (A), (C) or (E).

According to an embodiment, the compound of formula (A) or (E) where G is a halogen atom may typically be prepared by halogenation of the corresponding compound of formula (A) or (E) where G is OH.

As an illustrative example, the compound (A) where G is halogen may be obtained by reacting the compound (A) where G is OH with a halogenated electrophilic reagent such as thionyl chloride or acyl halide.

According to another embodiment, the compound of formula (A) where n=2 may typically be prepared by reacting a compound of formula (A) where n is 1 and G is CH₃ with an oxidizing agent such as selenium oxide (SeO₂) in the presence of a base, such as pyridine.

Generally, starting products (A), (C), (E) as well as reagents (B), (D) and (F) are commercially available or can be synthesized by application or adaptation of known procedures. For example, reagents (B), (D) and (F) may be a polythiol as defined above for P_{SH}.

The process can comprise a further step of purifying the compound of formula (I), (II) or (III) obtained, for example by column chromatography.

Alternatively, the process for the preparation of a compound of formula (I), (II) or (III) may comprise:
- reacting a compound of formula (J) with a compound of formula (B) to obtain a compound of formula (I); or
- reacting a compound of formula (K) with a compound of formula (D) to obtain a compound of formula (II); or
- reacting a compound of formula (L) with a compound of formula (B) to obtain a compound of formula (III): 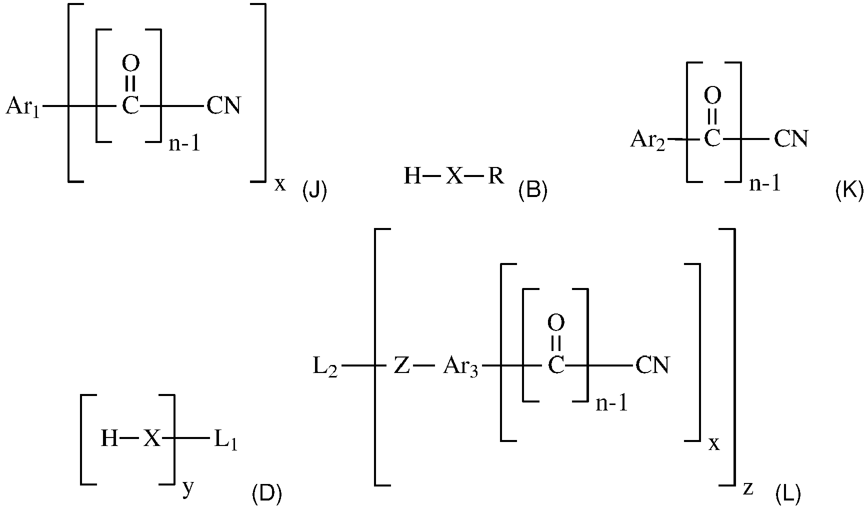
wherein Ar₁, Ar₂, Ar₃, L₁, L₂, R, X, Z, n, x, y and z are as defined herein.

Typically, the reaction of a compound of formula (J), (K) or (L) with a compound of formula (B) or (D) may be carried out in an organic solvent, preferably a non-polar organic solvent such as benzene, toluene or a dialkylether, in the presence of an acid, preferably a strong acid such as HCl.

Alternatively, the process for the preparation of a compound of formula (I), (II) or (III) may comprise:
- reacting a compound of formula (J) with hydrogen sulfide (H₂S) and a compound of formula (M) to obtain a compound of formula (I); or
- reacting a compound of formula (K) with hydrogen sulfide (H₂S) and a compound of formula (N) to obtain a compound of formula (II); or
- reacting a compound of formula (L) with hydrogen sulfide (H₂S) and a compound of formula (M) to obtain a compound of formula (III): 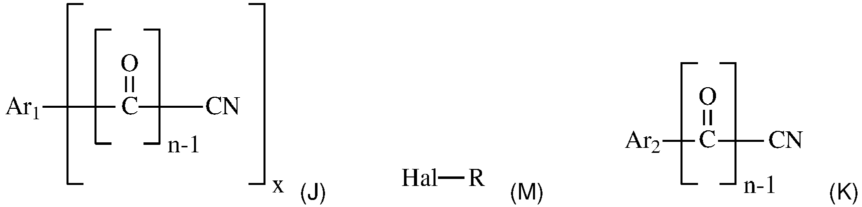 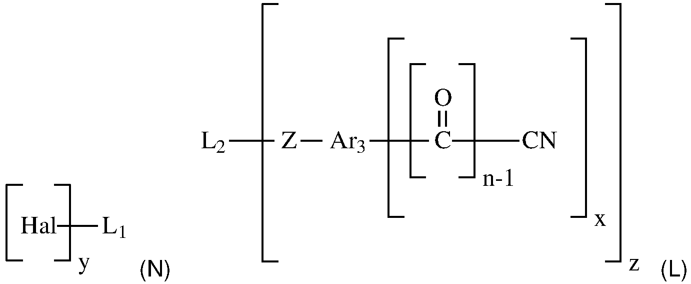
wherein
Ar₁, Ar₂, Ar₃, L₁, L₂, R, Z, n, x, y and z are as defined herein;
Hal is a halogen atom chosen from C!, Br, I.

Typically, the reaction of a compound of formula (J), (K) or (L) with hydrogen sulfide and a compound of formula (M) or (N) may be carried out in water or an organic solvent, preferably a polar organic solvent such as dimethylformamide or acetone, in the presence of a base, preferably a weak organic base such as pyridine.

The reaction may be conducted in a wide range of temperature, from -20°C to the reflux temperature of the reactional mixture, preferably at 50-80 °C.

### Curable composition

The invention also relates to a curable (or polymerizable) composition comprising one or more of compounds of formula (I), (II) or (III) as defined herein and one or more ethylenically unsaturated compounds.

The curable composition may comprise 0.05% to 10%, in particular 0.1% to 5%, more particularly 0.15 to 2%, by weight of compound of formula (I), (II) or (III) as defined herein based on the total weight of the curable composition. If the curable composition comprises a mixture of compounds of formula (I), (II) or (III) as defined herein, the above weight percentage may be calculated using the weight of the mixture of compounds of formula (I), (II) or (III) as defined herein.

The curable composition may include from 20% to 99.95%, preferably from 30% to 98%, more preferably from 40% to 97%, by weight of ethylenically unsaturated compounds based on the total weight of the curable composition. If the composition comprises a mixture of ethylenically unsaturated compounds, the above weight percentages may be calculated using the weight of the mixture of ethylenically unsaturated compounds.

The curable composition of the invention may further comprise one or more compounds selected from:
- an anionic synergist;
- a radical photoinitiator;
- an acid stabilizer;
- a radical stabilizer,
- an additive; and
- a solvent.

### Ethvlenicallv unsaturated compounds

The curable composition comprises one or more ethylenically unsaturated compounds.

As used herein, the term "ethylenically unsaturated compound" means a compound that comprises a polymerizable carbon-carbon double bond. A polymerizable carbon-carbon double bond is a carbon-carbon double bond that can react with another carbon-carbon double bond in a polymerization reaction. A polymerizable carbon-carbon double bond is generally comprised in a group selected from acrylate, methacrylate, cyanoacrylate, acrylamide, methacrylamide, styrene, maleate, fumarate, itaconate, methylene malonate, methylene acetyl acetonate, methylene beta-diketone, allyl, propenyl, vinyl and combinations thereof, preferably selected from acrylate, methacrylate, cyanoacrylate, allyl and vinyl, more preferably selected from acrylate, methacrylate and cyanoacrylate. The carbon-carbon double bonds of a phenyl ring are not considered as polymerizable carbon-carbon double bonds.

According to some preferred embodiments, the one or more ethylenically unsaturated compounds comprise at least one of a cyanoacrylate-functionalized compound, a (meth)acrylate-functionalized compound and mixtures thereof.

The curable composition may comprise a cyanoacrylate. The curable composition may comprise a mixture of cyanoacrylates.

As used herein, the term "cyanoacrylate" refers to an organic compound which contains a carbon-carbon double bond, wherein one carbon atom involved in the carbon-carbon double bond is substituted with a cyano (-CN) group and an ester group.

Examples of suitable cyanoacrylates include methyl cyanoacrylate, ethyl cyanoacrylate, n-propyl cyanoacrylate, iso-propyl cyanoacrylate, n-butyl cyanoacrylate, sec-butyl cyanoacrylate, iso-butyl cyanoacrylate, tert-butyl cyanoacrylate, n-pentyl cyanoacrylate, 1-methylbutyl cyanoacrylate, 1-ethylpropyl cyanoacrylate, neopentyl cyanoacrylate, n-hexyl cyanoacrylate, 1-methylpentyl cyanoacrylate, n-heptyl cyanoacrylate, n-octyl cyanoacrylate, 2-octyl cyanoacrylate, 2-ethylhexyl cyanoacrylate, n-nonyl cyanoacrylate, n-decyl cyanoacrylate, n-undecyl cyanoacrylate, n-dodecyl cyanoacrylate, n-octadecyl cyanoacrylate, allyl cyanoacrylate, cyclohexyl cyanoacrylate, 2-methoxyethyl cyanoacrylate, 2-ethoxyethyl cyanoacrylate, phenoxyethyl cyanoacrylate, 2-(1-alkoxy)propyl cyanoacrylate (for example 2-(1-methoxy)propyl cyanoacrylate), 2-(2'-alkoxy)-methoxyethyl-2"-cyanoacrylate (for example 2-(2'-methoxy)-methoxyethyl-2"-cyanoacrylate), 2-(2'-alkoxy)-ethoxyethyl-2"-cyanoacrylate (for example 2-(2'-methoxy)-ethoxyethyl-2"-cyanoacrylate, 2-(2'-ethoxy)-ethoxyethyl-2"-cyanoacrylate, 2-(2'-propyloxy)-ethoxyethyl-2"-cyanoacrylate, 2-(2'-butoxy)-ethoxyethyl-2"-cyanoacrylate, 2-(2'-pentyloxy)-ethoxyethyl-2"-cyanoacrylate or 2-(2'-hexyloxy)-ethoxyethyl-2"-cyanoacrylate), 2-(2'-alkoxy)-propyloxypropyl-2"-cyanoacrylate (for example 2-(2'-methoxy)-propyloxypropyl-2"-cyanoacrylate, 2-(2'-ethoxy)-propyloxypropyl-2"-cyanoacrylate, 2-(2'-propyloxy)-propyloxypropyl-2"-cyanoacrylate, 2-(2'-butyloxy)-propyloxypropyl-2"-cyanoacrylate, 2-(2'-pentyloxy)-propyloxypropyl-2"-cyanoacrylate, 2-(2'-hexyloxy)-propyloxypropyl-2"-cyanoacrylate), 2-(2'-alkoxy)-butyloxybutyl-2"-cyanoacrylate (for example 2-(2'-methoxy)-butyloxybutyl-2"-cyanoacrylate, 2-(2'-ethoxy)-butyloxybutyl-2"-cyanoacrylate, 2-(2'-butyloxy)-butyloxybutyl-2"-cyanoacrylate), 2-(3'-alkoxy)-propyloxyethyl-2"-cyanoacrylate for example 2-(3'-methoxy)-propyloxyethyl-2"-cyanoacrylate), 2-(3'-alkoxy)-butyloxyethyl-2"-cyanoacrylate (for example 2-(3'-methoxy)-butyloxyethyl-2"-cyanoacrylate), 2-(3'-alkoxy)-propyloxypropyl-2"-cyanoacrylate (for example 2-(3'-methoxy)-propyloxypropyl-2"-cyanoacrylate), 2-(3'-alkoxy)-butyloxypropyl-2"-cyanoacrylate (for example 2-(3'-methoxy)-butyloxypropyl-2"-cyanoacrylate 2-(2'-alkoxy)-ethoxypropyl-2"-cyanoacrylate (for example 2-(2'-methoxy)-ethoxypropyl-2"-cyanoacrylate), 2-(2'-alkoxy)-ethoxybutyl-2"-cyanoacrylate (for example 2-(2'-methoxy)-ethoxybutyl-2"-cyanoacrylate), trimethylsilyethyl cyanoacrylate, trimethylsilypropyl cyanoacrylate, tetrahydrofurfuryl cyanoacrylate, 1,6-hexanediol bis-cyanoacrylate, trimethylsilyloxyethyl cyanoacrylate triethylsilyloxyethyl cyanoacrylate, phenylethyl cyanoacrylate, and mixtures thereof.

More preferably, the cyanoacrylate is a mono-functional cyanoacrylate, more preferably selected from 2-methoxyethyl cyanoacrylate, 2-ethoxyethyl cyanoacrylate, methyl cyanoacrylate, ethyl cyanoacrylate, n-propyl cyanoacrylate, n-heptyl cyanoacrylate, n-octyl cyanoacrylate, iso-propyl cyanoacrylate or mixtures thereof.

When the curable composition comprises a cyanoacrylate, the amount of cyanoacrylate may be from 70% to 99% by weight, preferably from 75% to 95% by weight, and more preferably from 70 to 90% by weight based on the total weight of the curable composition.

The curable composition may comprise a (meth)acrylate-functionalized compound. The curable composition may comprise a mixture of (meth)acrylate-functionalized compounds.

As used herein, the term "(meth)acrylate-functionalized compound" means a compound comprising a (meth)acrylate group, in particular an acrylate group. The term "(meth)acrylate-functionalized compound" here encompasses containing more than one (meth)acrylate group, such as 2, 3, 4, 5 or 6 (meth)acrylate groups, commonly referred to as "oligomers" comprising a (meth)acrylate group. The term "(meth)acrylate group" encompasses acrylate groups (-O-CO-CH=CH₂) and methacrylate groups (-O-CO-C(CH₃)=CH₂).

In one embodiment, the curable composition may contain from 40% to 99%, from 45% to 90%, from 50% to 85%, or from 50% to 80% by weight of (meth)acrylatefunctionalized compounds based on the total weight of the curable composition.

Alternatively, the curable composition may contain from 5% to 50%, from 10% to 45%, from 15% to 40% or from 15% to 30%, by weight of (meth)acrylate-functionalized compounds based on the total weight of the curable composition.

The (meth)acrylate-functionalized compound may be selected from a (meth)acrylate-functionalized monomer, a (meth)acrylate-functionalized oligomer and mixtures thereof.

As used herein, the term "(meth)acrylate-functionalized monomer" means a monomer comprising a (meth)acrylate group, in particular an acrylate group. The term "(meth)acrylate-functionalized oligomer" means an oligomer comprising a (meth)acrylate group, in particular an acrylate group.

In one embodiment, the ethylenically unsaturated compound comprises a (meth)acrylate-functionalized monomer. The ethylenically unsaturated compound may comprise a mixture of (meth)acrylate-functionalized monomers.

The (meth)acrylate-functionalized monomer may have a molecular weight of less than 600 g/mol, in particular from 100 to 550 g/mol, more particularly 200 to 500 g/mol.

The (meth)acrylate-functionalized monomer may have 1 to 6 (meth)acrylate groups, in particular 1 to 3 (meth)acrylate groups.

The (meth)acrylate-functionalized monomer may comprise a mixture of (meth)acrylate-functionalized monomers having different functionalities. For example, the (meth)acrylate-functionalized monomer may comprise a mixture of a (meth)acrylatefunctionalized monomer containing a single acrylate or methacrylate group per molecule (referred to herein as "mono(meth)acrylate-functionalized compounds") and a (meth)acrylate-functionalized monomer containing 2 or more, preferably 2 or 3, acrylate and/or methacrylate groups per molecule.

In one embodiment, the (meth)acrylate functionalized monomer comprises a mono(meth)acrylate-functionalized monomer. The mono(meth)acrylate-functionalized monomer may advantageously function as a reactive diluent and reduce the viscosity of the composition of the invention.

Examples of suitable mono(meth)acrylate-functionalized monomers include, but are not limited to, mono-(meth)acrylate esters of aliphatic alcohols (wherein the aliphatic alcohol may be straight chain, branched or alicyclic and may be a mono-alcohol, a di-alcohol or a polyalcohol, provided only one hydroxyl group is esterified with (meth)acrylic acid); mono-(meth)acrylate esters of aromatic alcohols (such as phenols, including alkylated phenols); mono-(meth)acrylate esters of alkylaryl alcohols (such as benzyl alcohol); mono-(meth)acrylate esters of oligomeric and polymeric glycols such as diethylene glycol, triethylene glycol, dipropylene glycol, tripropylene glycol, polyethylene glycol, and polypropylene glycol); mono-(meth)acrylate esters of monoalkyl ethers of glycols and oligoglycols; mono-(meth)acrylate esters of alkoxylated (e.g., ethoxylated and/or propoxylated) aliphatic alcohols (wherein the aliphatic alcohol may be straight chain, branched or alicyclic and may be a mono-alcohol, a di-alcohol or a polyalcohol, provided only one hydroxyl group of the alkoxylated aliphatic alcohol is esterified with (meth)acrylic acid); mono-(meth)acrylate esters of alkoxylated (e.g., ethoxylated and/or propoxylated) aromatic alcohols (such as alkoxylated phenols); caprolactone mono(meth)acrylates; and the like.

The following compounds are specific examples of mono(meth)acrylatefunctionalized monomers suitable for use in the curable compositions of the present invention: methyl (meth)acrylate; ethyl (meth)acrylate; n-propyl (meth)acrylate; n-butyl (meth)acrylate; isobutyl (meth)acrylate; n-hexyl (meth)acrylate; 2-ethylhexyl (meth)acrylate; n-octyl (meth)acrylate; isooctyl (meth)acrylate; n-decyl (meth)acrylate; n-dodecyl (meth)acrylate; tridecyl (meth)acrylate; tetradecyl (meth)acrylate; hexadecyl (meth)acrylate; 2-hydroxyethyl (meth)acrylate; 2- and 3-hydroxypropyl (meth)acrylate; 2-methoxyethyl (meth)acrylate; 2-ethoxyethyl (meth)acrylate; 2- and 3-ethoxypropyl (meth)acrylate; tetrahydrofurfuryl (meth)acrylate; alkoxylated tetrahydrofurfuryl (meth)acrylate; 2-(2-ethoxyethoxy)ethyl (meth)acrylate; cyclohexyl (meth)acrylate; glycidyl (meth)acrylate; isodecyl (meth)acrylate; lauryl (meth)acrylate; 2-phenoxyethyl (meth)acrylate; alkoxylated phenol (meth)acrylates; alkoxylated nonylphenol (meth)acrylates; cyclic trimethylolpropane formal (meth)acrylate; isobornyl (meth)acrylate; tricyclodecanemethanol (meth)acrylate; tert-butylcyclohexanol (meth)acrylate; trimethylcyclohexanol (meth)acrylate; diethylene glycol monomethyl ether (meth)acrylate; diethylene glycol monoethyl ether (meth)acrylate; diethylene glycol monobutyl ether (meth)acrylate; triethylene glycol monoethyl ether (meth)acrylate; ethoxylated lauryl (meth)acrylate; methoxy polyethylene glycol (meth)acrylates; hydroxyl ethyl-butyl urethane (meth)acrylates; 3-(2-hydroxyalkyl)oxazolidinone (meth)acrylates; and combinations thereof.

In one embodiment, the (meth)acrylate functionalized monomer may comprise a (meth)acrylate-functionalized monomer containing two or more (meth)acrylate groups per molecule.

Examples of suitable (meth)acrylate-functionalized monomers containing two or more (meth)acrylate groups per molecule include acrylate and methacrylate esters of polyhydric alcohols (organic compounds containing two or more, e.g., 2 to 6, hydroxyl groups per molecule). Specific examples of suitable polyhydric alcohols include C2-20 alkylene glycols (glycols having a C2-10 alkylene group may be preferred, in which the carbon chain may be branched; e.g., ethylene glycol, trimethylene glycol, 1,2-propylene glycol, 1,2-butanediol, 1,3-butanediol, 2,3-butanediol, tetramethylene glycol (1,4-butanediol), 1,5-pentanediol, 1,6-hexanediol, 1,8-octanediol, 1,9-nonanediol, 1,12-dodecanediol, cyclohexane-1,4-dimethanol, bisphenols, and hydrogenated bisphenols, as well as alkoxylated (e.g., ethoxylated and/or propoxylated) derivatives thereof), diethylene glycol, glycerin, alkoxylated glycerin, triethylene glycol, dipropylene glycol, tripropylene glycol, trimethylolpropane, alkoxylated trimethylolpropane, ditrimethylolpropane, alkoxylated ditrimethylolpropane, pentaerythritol, alkoxylated pentaerythritol, dipentaerythritol, alkoxylated dipentaerythritol, cyclohexanediol, alkoxylated cyclohexanediol, cyclohexanedimethanol, alkoxylated cyclohexanedimethanol, norbornene dimethanol, alkoxylated norbornene dimethanol, norbornane dimethanol, alkoxylated norbornane dimethanol, polyols containing an aromatic ring, cyclohexane-1,4-dimethanol ethylene oxide adducts, bis-phenol ethylene oxide adducts, hydrogenated bisphenol ethylene oxide adducts, bisphenol propylene oxide adducts, hydrogenated bisphenol propylene oxide adducts, cyclohexane-1,4-dimethanol propylene oxide adducts, sugar alcohols and alkoxylated sugar alcohols. Such polyhydric alcohols may be fully or partially esterified (with (meth)acrylic acid, (meth)acrylic anhydride, (meth)acryloyl chloride or the like), provided they contain at least two (meth)acrylate functional groups per molecule.

Exemplary (meth)acrylate-functionalized monomers containing two or more (meth)acrylate groups per molecule may include bisphenol A di(meth)acrylate; hydrogenated bisphenol A di(meth)acrylate; ethylene glycol di(meth)acrylate; diethylene glycol di(meth)acrylate; triethylene glycol di(meth)acrylate; tetraethylene glycol di(meth)acrylate; polyethylene glycol di(meth)acrylate; propylene glycol di(meth)acrylate; dipropylene glycol di(meth)acrylate; tripropylene glycol di(meth)acrylate; tetrapropylene glycol di(meth)acrylate; polypropylene glycol di(meth)acrylate; polytetramethylene glycol di(meth)acrylate; 1,2-butanediol di(meth)acrylate; 2,3-butanediol di(meth)acrylate; 1,3-butanediol di(meth)acrylate; 1,4-butanediol di(meth)acrylate; 1,5-pentanediol di(meth)acrylate; 1,6-hexanediol di(meth)acrylate; 1,8-octanediol di(meth)acrylate; 1,9-nonanediol di(meth)acrylate; 1,10-nonanediol di(meth)acrylate; 1,12-dodecanediol di(meth)acrylate; neopentyl glycol di(meth)acrylate; 2-methyl-2,4-pentanediol di(meth)acrylate; polybutadiene di(meth)acrylate; cyclohexane-1,4-dimethanol di(meth)acrylate; tricyclodecane dimethanol di(meth)acrylate; metallic di(meth)acrylates; modified metallic di(meth)acrylates; glyceryl di(meth)acrylate; glyceryl tri(meth)acrylate; trimethylolethane tri(meth)acrylate; trimethylolethane di(meth)acrylate; trimethylolpropane tri(meth)acrylate; trimethylolpropane di(meth)acrylate; pentaerythritol di(meth)acrylate; pentaerythritol tri(meth)acrylate; pentaerythritol tetra(meth)acrylate, di(trimethylolpropane) diacrylate; di(trimethylolpropane) triacrylate; di(trimethylolpropane) tetraacrylate, sorbitol penta(meth)acrylate; di(pentaerythritol) tetraacrylate; di(pentaerythritol) pentaacrylate; di(pentaerythritol) hexa(meth)acrylate; tris (2-hydroxyethyl) isocyanurate tri(meth)acrylate; as well as the alkoxylated (e.g., ethoxylated and/or propoxylated) derivatives thereof; and combinations thereof.

The curable composition of the invention may comprise 0 to 99.5%, in particular 5 to 90%, more particularly 10 to 80%, even more particularly 15 to 75%, more particularly still 20 to 70% by weight of (meth)acrylate-functionalized monomer based on the total weight of the curable composition. In particular, the curable composition of the invention may comprise 5 to 50% or 10 to 50% or 15 to 50% or 20 to 50% or 25 to 50% or 30 to 50%, by weight of (meth)acrylate-functionalized monomer based on the total weight of the curable composition. Alternatively, the curable composition of the invention may comprise 50 to 99.5% or 55 to 99.5% or 60 to 99.5% or 65 to 99.5% or 70 to 99.5%, by weight of (meth)acrylate-functionalized monomer based on the total weight of the curable composition.

In one embodiment, the ethylenically unsaturated compound comprises a (meth)acrylate-functionalized oligomer. The ethylenically unsaturated compound may comprise a mixture of (meth)acrylate-functionalized oligomers.

The (meth)acrylate-functionalized oligomer may be selected in order to enhance the flexibility, strength and/or modulus, among other attributes, of a cured polymer prepared using the curable composition of the present invention.

The (meth)acrylate functionalized oligomer may have 1 to 18 (meth)acrylate groups, in particular 2 to 6 (meth)acrylate groups, more particularly 2 to 6 acrylate groups.

The (meth)acrylate functionalized oligomer may have a number average molecular weight equal or more than 600 g/mol, in particular 800 to 15,000 g/mol, more particularly 1,000 to 5,000 g/mol.

In particular, the (meth)acrylate-functionalized oligomers may be selected from the group consisting of epoxy (meth)acrylates, polyester (meth)acrylates, polyether (meth)acrylates, urethane (meth)acrylates, (meth)acrylated poly(meth)acrylates and mixtures thereof.

Non-limiting examples of epoxy (meth)acrylates are the reaction products of an epoxide (such as glycidyl ethers, glycidyl esters, cycloaliphatic epoxides or epoxides obtained by epoxidation of mono- and/or polyunsaturated compounds) with a (meth)acrylating agent (such as (meth)acrylic acid, (meth)acrylic anhydride, (meth)acryloyl chloride or combinations thereof). The epoxide may be selected from 1,2,3,4-diepoxybutane; 1,2,4,5-diepoxypentane; 1,2,5,6-diepoxyhexane; 1,2,7,8-diepoxyoctane; 1,2,9,10-diepoxydecane; bisphenol A diglycidyl ether, bisphenol F diglycidyl ether, bisphenol S diglycidyl ether, brominated bisphenol A diglycidyl ether, brominated bisphenol F diglycidyl ether, brominated bisphenol S diglycidyl ether, epoxy novolak resin, hydrogenated bisphenol A diglycidyl ether, hydrogenated bisphenol F diglycidyl ether, hydrogenated bisphenol S diglycidyl ether, 3,4-epoxycyclohexylmethyl-3',4'-epoxycyclohexanecarboxylate, 2-(3,4-epoxycyclohexyl-5,5-spiro-3,4-epoxy)cyclohexane-1,4-dioxane, bis(3,4-epoxycyclohexylmethyl)adipate, vinylcyclohexene oxide, 4-vinylepoxycyclohexane, bis(3,4-epoxy-6-methylcyclohexylmethyl)adipate,3,4-epoxy-6-methylcyclohexy I-3',4'-epoxy-6'-methylcyclohexanecarboxylate, methylenebis(3,4-epoxycyclohexane), dicyclopentadiene diepoxide, di(3,4-epoxycyclohexylmethyl) ether of ethylene glycol, ethylenebis(3, 4-epoxycyclohexanecarboxylate), ethylene glycol diglycidyl ether, 1,2- or 1,3-propylene glycol diglycidyl ether, 1,2-, 1,3- or 1,4-butanediol diglycidyl ether, 1,5-pentanediol diglycidyl ether, 1,6-hexanediol diglycidyl ether, 1,7-hexanediol diglycidyl ether, 1,8-octanediol diglycidyl ether, 1,9-nonanediol diglycidyl ether, 1,10-decanediol diglycidyl ether, 1,12-dodecanediol diglycidyl ether, 2-methyl-1,3-propanediol diglycidyl ether, neopentyl glycol diglycidyl ether, 2,2-diethyl-1,3-propane diol diglycidyl ether, 3-methyl-1,5-pentanediol diglycidyl ether, 3,3-dimethyl-1,5-pentanediol diglycidyl ether, 2,4-diethyl-1,5-pentanediol diglycidyl ether, 3,3-butylethyl-1,5-pentane diol diglycidyl ether, di-, tri- or tetra(ethylene glycol) diglycidyl ether, di-, tri- or tetra(1,2-propylene glycol) diglycidyl ether, di-, tri- or tetra(1,3-propylene glycol) diglycidyl ether, di-, tri- or tetra(1,4-butylene glycol) diglycidyl ether, a polyethylene glycol) diglycidyl ether, a polypropylene glycol) diglycidyl ether, a poly(trimethylene glycol) diglycidyl ether, a poly(tetramethylene glycol) diglycidyl ether, a polyethylene glycol-co-propylene glycol) diglycidyl ether, glycerol triglycidyl ether, a polyglycerol polyglycidyl ether, trimethylolmethane triglycidyl ether, trimethylolethane triglycidyl ether, trimethylolpropane triglycidyl ether, di(trimethylolpropane) tetraglycidyl ether, pentaerythritol tetraglycidyl ether, diglycidyl cyclohexanedicarboxylate, cyclohexane diglycidyl ether, cyclohexane-1,4-dimethanol diglycidyl ether, tricyclodecane dimethanol diglycidyl ether, isosorbide diglycidyl ether, pyrocatechol diglycidyl ether, resorcinol diglycidyl ether, cardol diglycidyl ether, phloroglucinol triglycidyl ether, pyrogallol triglycidyl ether, tris(hydroxyphenyl)methane triglycidyl ether, tris(hydroxyphenyl)ethane triglycidyl ether, diglycidyl phthalate, diglycidyl terephthalate, diglycidyl isophthalate, polyglycidyl ethers of a polyether polyol obtained by the addition of one or more alkylene oxides to an aliphatic polyhydric alcohol such as ethylene glycol, propylene glycol, and glycerol, diglycidyl esters of aliphatic long-chain (C6-C22) dibasic acids, monoglycidyl ethers of aliphatic higher alcohols, monoglycidyl ethers of phenol, cresol, butyl phenol, or polyether alcohols obtained by the addition of alkylene oxide to these compounds, glycidyl esters of higher fatty acids, an epoxidized vegetable oil (such as epoxidized soybean oil and epoxidized linseed oil), epoxybutylstearic acid, epoxyoctylstearic acid, epoxidized polybutadiene, triglycidyl isocyanurate and the like.

Non-limiting examples of polyester (meth)acrylates are the reaction products of a hydroxyl group-terminated polyester polyol with a (meth)acrylating agent (such as (meth)acrylic acid, (meth)acrylic anhydride, (meth)acryloyl chloride or combinations thereof). The reaction process may be conducted such that a significant concentration of residual hydroxyl groups remain in the polyester (meth)acrylate or may be conducted such that all or essentially all of the hydroxyl groups of the polyester polyol have been (meth)acrylated. The polyester polyols can be made by polycondensation reactions of a polyhydroxyl functional component (in particular a diol) and a polycarboxylic acid functional compound (in particular, a dicarboxylic acid or anhydride). To prepare the polyester (meth)acrylates, the hydroxyl groups of the polyester polyol are then partially or fully esterified by reacting with the (meth)acrylating agent. Polyester (meth)acrylates may also be synthesized by reacting a hydroxyl-containing (meth)acrylate such as a hydroxyalkyl (meth)acrylate (e.g., hydroxyethyl acrylate) with a polycarboxylic acid. The polyhydroxyl functional and polycarboxylic acid functional components can each have linear, branched, cycloaliphatic or aromatic structures and can be used individually or as mixtures.

Non-limiting examples of polyether (meth)acrylates are the condensation reaction products of a polyether polyol with a (meth)acrylating agent (such as (meth)acrylic acid, (meth)acrylic anhydride, (meth)acryloyl chloride or combinations thereof). Suitable polyether polyols can be linear or branched substances containing ether bonds and terminal hydroxyl groups. Polyether polyols can be prepared by ring opening polymerization of epoxides and other oxygen-containing heterocyclic compounds (e.g., ethylene oxide, 1,2-propylene oxide, butene oxide, tetrahydrofuran and combinations thereof) with a starter molecule. Suitable starter molecules include water, hydroxyl functional materials, polyester polyols and amines. Polyetherols may also be obtained by the condensation of diols such as glycols.

Non-limiting examples of urethane (meth)acrylates are the condensation reaction products of at least one polyisocyanate (e.g., diisocyanate, triisocyanate), at least one polyol (such as a polyether polyol or a polyester polyol) and a hydroxyl-functionalized (meth)acrylate (such as 2-hydroxyethyl (meth)acrylate or 3-hydroxypropyl (meth)acrylate) to provide terminal (meth)acrylate groups. For example, the urethane (meth)acrylate may contain two, three, four or more (meth)acrylate groups per molecule. The order of addition of the components to prepare the urethane (meth)acrylate is well known in the art. For example, the hydroxyl-functionalized (meth)acrylate may be first reacted with the polyisocyanate to obtain an isocyanate-functionalized (meth)acrylate, which is then reacted with the polyol. In yet another embodiment, the polyisocyanate may be first reacted with the polyol to obtain an isocyanate-functionalized polyol, which is thereafter reacted with a hydroxyl-functionalized (meth)acrylate. Alternatively, all the components may be combined and reacted at the same time.

Non-limiting examples of (meth)acrylated poly(meth)acrylates are substances having an oligomeric (meth)acrylic backbone which is functionalized with one or (meth)acrylate groups (which may be at a terminus of the oligomer or pendant to the acrylic backbone). The (meth)acrylic backbone may be a homopolymer, random copolymer or block copolymer comprised of repeating units of (meth)acrylic monomers. The (meth)acrylic monomers may be any monomeric (meth)acrylate such as C₁-C₆ alkyl (meth)acrylates as well as functionalized (meth)acrylates such as (meth)acrylates bearing hydroxyl, carboxylic acid and/or epoxy groups. (Meth)acrylated poly(meth)acrylates may be prepared using any procedures known in the art, such as by oligomerizing (meth)acrylic monomers, at least a portion of which are functionalized with hydroxyl, carboxylic acid and/or epoxy groups (e.g., hydroxyalkyl(meth)acrylates, (meth)acrylic acid, glycidyl (meth)acrylate) to obtain a functionalized poly(meth)acrylate, which is then reacted with one or more (meth)acrylate-containing reactants to introduce the desired (meth)acrylate functional groups.

### Anionic synergist

The curable composition may further comprise an anionic synergist. Said anionic synergist may be present when the curable composition comprises a cyanoacrylate-functionalized compound, in particular to enhance the cure rate of said cyanoacrylate-functionalized compound.

In particular, the anionic synergist may be a metallocene.

Preferred metallocenes for use in the present invention include metallocenes in which each of the aromatic electron system ligands in the metallocene is a π-arene, indenyl, or η⁵-cyclopentadienyl ligand. The metallocene may be based on a transition metal selected from the group consisting of iron, osmium, and ruthenium. Iron-containing metallocenes, i.e., ferrocenes, are employed in certain aspects of the invention. However, in other embodiments, the metallocene compound may be an osmocene or a ruthenocene. Combinations of two or more different metallocene compounds may be used.

More particularly, the anionic synergist may be a ferrocene.

A ferrocene may be according to the following formula (VI): 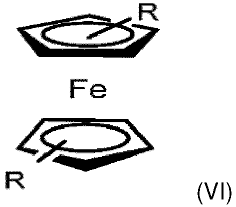 wherein each R is independently hydrogen or a linear or branched C₁-C₄ alkyl.

Specific examples of metallocenes useful in the present invention are disclosed in US 6503959, which is incorporated herein by reference.

The amount of anionic synergist in the curable composition may be from 50 to 1000 ppm based on the weight of the curable composition.

### Acid Stabilizer

The curable composition may further comprise an acid stabilizer. Said acid stabilizer may be present when the curable composition comprises a cyanoacrylate-functionalized compound, in particular to enhance the storage stability of the curable composition and prevent premature curing.

In particular, the acid stabilizer may be a Lewis acid. Examples of suitable Lewis acid include of boron trifluoride, boron trifluoride etherate complexes (e.g., boron trifluoride diethyl etherate complex), boron trifluoride dihydrate, trimethylsilyl triflate, sulphur dioxide, sulphur trioxide, nitrogen oxide and mixtures thereof, most preferably from boron trifluoride etherate complexes.

Other types of suitable acid stabilizers include protic acids such as hydrogen halides (e.g., hydrogen fluoride) and sulfonic acids (e.g., p-toluenesulfonic acid).

The amount of acid stabilizer in the curable composition may be from 1 to 1000 ppm , e.g. from 10 to 100 ppm based on the total weight of the curable composition.

### Radical photoinitiator

The curable composition may further comprise a radical photoinitiator, in particular a radical photoinitiator having Norrish type I activity and/or Norrish type II activity, more particularly a radical photoinitiator having Norrish type I activity. The radical photoinitiator is distinct from the compound of formula (I), (II) or (III).

Non-limiting types of radical photoinitiators suitable for use in the curable compositions include, for example, benzoins, benzoin ethers, acetophenones, α-hydroxy acetophenones, benzil, benzil ketals, phosphine oxides, acylphosphine oxides, α-hydroxyketones, phenylglyoxylates, α-aminoketones, benzoyl formates, acylgermanyl compounds, polymeric derivatives thereof, and mixtures thereof., but are not limited to, benzoin, benzoin methyl ether, benzoin ethyl ether, benzoin isopropyl ether, alphamethylbenzoin, alpha-phenylbenzoin, Michler's ketone, 1-hydroxyphenyl ketones, acetophenone, 2,2-diethyloxyacetophenone, benzil, α-hydroxyketone, 2,4,6-trimethylbenzoyldiphenyl phosphine oxide, 2,2-dimethoxy-1,2-phenylacetophenone, 1-hydroxycyclohexyl phenyl ketone, 2-methyl-1-[4-(methylthio) phenyl]-2-morpholinopropanone, 2-hydroxy-2-methyl-1-phenyl-propanone, oligomeric α-hydroxy ketone, benzoyl phosphine oxides, phenylbis(2,4,6-trimethylbenzoyl)phosphine oxide, ethyl(2,4,6-trimethylbenzoyl)phenyl phosphinate, anisoin, benzoin isobutyl ether, 4-benzoylbiphenyl, 2-benzyl-2-(dimethylamino)-4'-morpholinobutyrophenone, 4,4'-dimethylbenzil, diphenyl(2,4,6-trimethylbenzoyl)phosphine oxide /2-hydroxy-2-methylpropiophenone 50/50 blend, 4'-ethoxyacetophenone, 2,4,6-trimethylbenzoyldiphenylphosphine oxide, 3'-hydroxyacetophenone, 4'-hydroxyacetophenone, methybenzoylformate, 4'-phenoxyacetophenone, polymeric derivatives thereof and combinations thereof.

Preferred radical photoinitiators are acetophenones, α-hydroxy acetophenones, phosphine oxides and acylphosphine oxides, more preferably acetophenones and acylphosphine oxides.

In particular, the radical photoinitiator may be selected from an acetophenone such as SpeedCure^{®} BKL (2,2-dimethoxy-1,2-phenylacetophenone); an acylphosphine oxide such as SpeedCure^{®} XKM (ethyl phenyl(2,4,6-trimethylbenzoyl)phosphinate), SpeedCure^{®} BPO (phenyl bis(2,4,6-trimethylbenzoyl)-phosphine oxide), SpeedCure^{®} TPO (2,4,6-trimethylbenzoyldiphenylphosphine oxide) or SpeedCure^{®} TPO-L (ethyl (2,4,6-trimethylbenzoyl)phenyl phosphinate); and mixtures thereof.

The amount of radical photoinitiator may be varied as may be appropriate depending on the radical photoinitiator(s) selected, the amounts and types of polymerizable species present in the curable composition, the radiation source and the radiation conditions used, among other factors. Typically, however, the amount of radical photoinitiator may be from 0% to 10%, for example 0.05% to 10%, in particular 0.1% to 5%, more particularly 0.5 to 2%, by weight of radical photoinitiator based on the total weight of the curable composition. For example, the amount of radical photoinitiator may be from 0.01% to 5%, from 0.02% to 3%, from 0.05 to 2%, from 0.1 to 1.5% or from 0.2 to 1%, by weight based on the total weight of the curable composition. In another example, the amount of radical photoinitiator may be from 1% to 5%, from 1.5% to 5%, from 2 to 5%, from 2.5 to 5% or from 3 to 5%, by weight based on the total weight of the curable composition.

### Radical stabilizer

The curable composition may further comprise a radical stabilizer.

The radical stabilizer is a free radical polymerization inhibitor. It is preferably selected from the group consisting of hindered phenolic or polyphenolic compounds; preferably from hydroquinone, hydroquinone monomethyl ether, mono-tertiary-butyl hydroquinone, 2,5-ditertiary-butyl-hydroquinone, p-methoxyphenol, hydroxyanisole, butylated hydroxyanisole, hydroxyanisole butyl ether, 2,6-di-tert-butyl-p-cresol, 2,2'-methylene-bis-(6-tert-butyl-4-methylphenol), p-tert-butyl catechol, 1,3,5-trimethyl-2,4,6-tris(3,5-di-tert-butyl-4-hydroxybenzyl) benzene, hydroxytoluene butyl ether, and mixtures thereof; more preferably from hydroquinone monomethyl ether, 2,2'-methylene-bis-(6-tert-butyl-4-methylphenol), and mixtures thereof.

The curable composition may comprise from 0.01 to 0.7 % by weight, preferably from 0.01 to 0.5% by weight, more preferably from 0.01 to 0.4 % by weight, of radical stabilizer based on total weight of the curable composition.

### Solvent

Advantageously, the curable compositions may be formulated to be solvent-free, i.e., free of any non-reactive volatile substances. However, in certain other embodiments of the invention, the curable composition may contain one or more solvents, in particular one or more organic solvents, which may be non-reactive organic solvents. In various embodiments, the solvent(s) may be relatively volatile, e.g., solvents having a boiling point at atmospheric pressure of not more than 150° C. In other embodiments, the solvent(s) may have a boiling point at atmospheric pressure of at least 40°C.

The solvent(s) may be selected so as to be capable of solubilizing one or more components of the curable composition and/or adjusting the viscosity or other rheological properties of the curable composition.

However, the curable compositions may alternatively be formulated so as to contain little or no non-reactive solvent, e.g., less than 10% or less than 5% or even 0% non-reactive solvent, based on the total weight of the curable composition. Such solvent-less or lowsolvent compositions may be formulated using various components, including for example low viscosity reactive diluents, which are selected so as to render the curable composition sufficiently low in viscosity, even without solvent being present, that the curable composition can be easily applied at a suitable application temperature to a substrate surface so as to form a relatively thin, uniform layer.

Suitable solvents may include, for example, organic solvents such as: ketones; esters; carbonates; alcohols; aromatic solvents such as xylene, benzene, toluene, and ethylbenzene; alkanes; glycol ethers; ethers; amides; as well as combinations thereof.

In various embodiments of the invention, the curable compositions described herein are formulated to have a viscosity of less than 10,000 mPa.s (cP), or less than 5,000 mPa.s (cP), or less than 4,000 mPa.s (cP), or less than 3,000 mPa.s (cP), or less than 2,500 mPa.s (cP), or less than 2,000 mPa.s (cP), or less than 1,500 mPa.s (cP), or less than 1,000 mPa.s (cP) or even less than 500 mPa.s (cP) as measured at 25°C using a Brookfield viscometer, model DV-II, using a 27 spindle (with the spindle speed varying typically between 20 and 200 rpm, depending on viscosity). In advantageous embodiments of the invention, the viscosity of the curable composition is from 200 to 1000 cPs at 25°C.

### Additives

The curable compositions may optionally contain one or more additives instead of or in addition to the above-mentioned ingredients. Such additives include, but are not limited to, free radical chain transfer agents, antioxidants, ultraviolet absorbers, light blockers, photostabilizers, foam inhibitors, flow or leveling agents, fillers, colorants, fluorophores, pigments, dispersants (wetting agents), slip additives, plasticizers, thixotropic agents, matting agents, impact modifiers, adhesion promoters, thermoplastics such as acrylic resins that do not contain any free radical-polymerizable functional groups, waxes or other various additives, including any of the additives conventionally utilized in the coating, sealant, adhesive, molding, 3D printing or ink arts.

### Uses

The invention also relates to the use of a compound of formula (I), (II) or (III) as defined herein as described above as a photoinitiator. In particular, the compound of formula (I) may be used as a photoinitiator having photobleaching properties.

The compound of formula (I), (II) or (III) may be used as a photoinitiator that is activable by irradiation with a LED light source, preferably a LED light source having a maximum output wavelength in the range of 250 to 550 nm, in particular 250 to 460 nm, more particularly 340 to 430 nm.

The photoinitiators of the present invention may have potential applications in photocurable inks, coatings, adhesives, advanced materials, electronics and in additive manufacturing (3D printing).

The invention particularly relates the use of the compounds of formula (I), (II) or (III) as photoinitiators for curing formulations comprising one or more ethylenically unsaturated compounds which may be polymerized by free radical, anionic and hybrid anionic/free radical polymerization. Said ethylenically unsaturated compounds may be as described above for the curable composition.

They advantageously show high cure speeds, low yellowing and/or photobleaching characteristics and high thermal stability in formulations. The yellowing characteristic can be measured by the Colour index 'b' value on cured films. Advantageously, the compounds of formula (I), (II) or (III) as defined herein do not suffer from oxygen inhibition.

### Curing process and cured product

The invention also relates to a process for curing one or more ethylenically unsaturated compounds, said process comprising :
mixing one or more ethylenically unsaturated compounds with a compound of formula (I), (II) or (III) according to the invention; and
irradiating said mixture with at least one light source.

The light source is generally a LED (light-emitting diode) light source, or a broadband lamp with an optical filter that limits emission to wavelengths in the range of 250 to 550 nm, in particular 250 to 460 nm.

Preferably the light source is a LED light source, more preferably a LED light source having a maximum output wavelength in the range of 250 to 550 nm, in particular 250 to 460 nm. The maximum output wavelength of the LED light source may be from 350 to 450 nm, notably from 340 to 430 nm, even more preferably of 250 nm, 365 nm, 385 nm or 405 nm.

Since the photoactivity of the compounds of formula (I), (II) or (III) is not altered by oxygen, it is not necessary to take specific precautions to prevent contact with oxygen during the curing process. According to an embodiment, the irradiation may be carried out under air or under inert atmosphere, preferably under air.

The invention also relates to a process for the preparation of a cured product, comprising curing the curable composition as defined above, preferably by irradiating the composition with at least one light source.

The light source may be as defined above for the curing process.

The curable composition may be stationary when exposed to the light source. Alternatively, the curable composition may be in motion when exposed to the light source (for example, on a conveyor belt).

Curing may be accelerated or facilitated by supplying energy to the curable composition, such as by heating the curable composition. Thus, the cured product may be deemed as the reaction product of the curable composition, formed by curing. A curable composition may be partially cured by exposure to actinic radiation, with further curing being achieved by heating the partially cured article. For example, a product formed from the curable composition may be heated at a temperature of from 40°C to 120°C for a period of time of from 5 minutes to 12 hours.

Prior to curing, the curable composition may be applied to a substrate surface in any known conventional manner, for example, by spraying, jetting, knife coating, roller coating, casting, drum coating, dipping, and the like and combinations thereof. Indirect application using a transfer process may also be used. A substrate may be any commercially relevant substrate, such as a high surface energy substrate or a low surface energy substrate, such as a metal substrate or plastic substrate, respectively. The substrates may comprise metal, paper, cardboard, glass, thermoplastics such as polyolefins, polycarbonate, acrylonitrile butadiene styrene (ABS), and blends thereof, composites, wood, leather and combinations thereof. When used as an adhesive, the curable composition may be placed between two substrates and then cured, the cured composition thereby bonding the substrates together to provide an adhered article. Curable compositions in accordance with the present invention may also be formed or cured in a bulk manner (e.g., the curable composition may be cast into a suitable mold and then cured).

The cured product obtained with the process of the invention may be an ink, a coating, a sealant, an adhesive, a molded article or a 3D-printed article.

According to a further object, the invention concerns the cured product obtainable by curing the curable composition of the invention.

The examples and figures hereafter illustrate the invention.

### Examples

### Example 1: Synthesis of thioxanthone derivatives

### S-(4-isopropylphenyl) 9-oxo-9H-thioxanthene-4-carbothioate

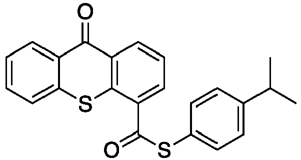

4-carboxythioxanthone (100 mg, 0.39 mmol, 1 eq.) was suspended in chloroform (2 mL), the flask and reflux condenser were flushed with nitrogen. One drop of DMF and then thionyl chloride (0.1 mL) were added to the solution and the mixture was warmed to 60°C. After 1.5 hour thionyl chloride (0.05 mL) was added to the mixture and continued reflux at 60°C for 1.5 hour, then cooled to 40°C and stirred under nitrogen overnight. The morning thionyl chloride (0.1 mL) was added and the mixture was reheated to 60°C for 1.30 hour. Then the mixture was evaporated to dryness and dried under vacuum to give the crude acyl chloride thioxanthone. This solid was suspended in anhydrous THF (1 mL). Meanwhile, anhydrous THF (2 mL) was placed in a 3-necked flask, flushed with nitrogen and cooled at 0°C. Triethylamine (0.16 mL, 1.17 mmol, 3 eq.), 4-isopropylbenzene thiol (0.12 mL, 0.78 mmol, 2 eq.) and the suspended acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(4-isopropylphenyl) 9-oxo-9H-thioxanthene-4-carbothioate as a yellow solid (77 mg, 51%).

### S-(naphthalen-2-yl) 9-oxo-9H-thioxanthene-4-carbothioate

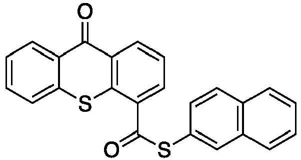

4-carboxythioxanthone (100 mg, 0.39 mmol, 1 eq.) was suspended in chloroform (2 mL), the flask and reflux condenser were flushed with nitrogen. One drop of DMF and then thionyl chloride (0.1 mL) were added to the solution and the mixture was warmed to 60°C. After 1.5 hour thionyl chloride (0.05 mL) was added to the mixture and continued reflux at 60°C for 1.5 hour, then cooled to 40°C and stirred under nitrogen overnight. The morning thionyl chloride (0.1 mL) was added and the mixture was reheated to 60°C for 1.30 hour. Then the mixture was evaporated to dryness and dried under vacuum to give the crude acyl chloride thioxanthone. This solid was suspended in anhydrous THF (1 mL). Meanwhile, anhydrous THF (2 mL) was placed in a 3-necked flask, flushed with nitrogen and cooled at 0°C. Triethylamine (0.16 mL, 1.17 mmol, 2 eq.), 2-naphthalene thiol (125 mg, 0.78 mmol, 2 eq.) and the suspended acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(naphthalen-2-yl) 9-oxo-9H-thioxanthene-4-carbothioate as a light yellow solid (81 mg, 52%).

### S-cyclohexyl 9-oxo-9H-thioxanthene-4-carbothioate

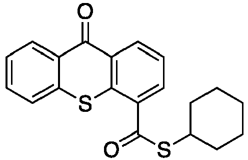

4-carboxythioxanthone (100 mg, 0.39 mmol, 1 eq.) was suspended in chloroform (2 mL), the flask and reflux condenser were flushed with nitrogen. One drop of DMF and then thionyl chloride (0.1 mL) were added to the solution and the mixture was warmed to 60°C. After 1.5 hour thionyl chloride (0.05 mL) was added to the mixture and continued reflux at 60°C for 1.5 hour, then cooled to 40°C and stirred under nitrogen overnight. The morning thionyl chloride (0.1 mL) was added and the mixture was reheated to 60°C for 1.30 hour. Then the mixture was evaporated to dryness and dried under vacuum to give the crude acyl chloride thioxanthone. This solid was suspended in anhydrous THF (1 mL). Meanwhile, anhydrous THF (2 mL) was placed in a 3-necked flask, flushed with nitrogen and cooled at 0°C. Triethylamine (0.16 mL, 1.17 mmol, 3 eq.), cyclohexane thiol (0.10 mL, 0.78 mmol, 2 eq.) and the suspended acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford the S-cyclohexyl 9-oxo-9H-thioxanthene-4-carbothioate as a light yellow solid (69 mg, 50%).

### Methyl 4-((9-oxo-9H-thioxanthene-4-carbonyl)thio)benzoate

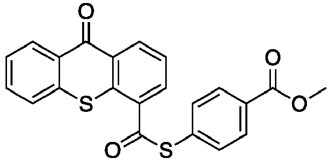

4-carboxythioxanthone (100 mg, 0.39 mmol, 1 eq.) was suspended in chloroform (2 mL), the flask and reflux condenser were flushed with nitrogen. One drop of DMF and then thionyl chloride (0.1 mL) were added to the solution and the mixture was warmed to 60°C. After 1.5 hour thionyl chloride (0.05 mL) was added to the mixture and continued reflux at 60°C for 1.5 hour, then cooled to 40°C and stirred under nitrogen overnight. The morning thionyl chloride (0.1 mL) was added and the mixture was reheated to 60°C for 1.30 hour. Then the mixture was evaporated to dryness and dried under vacuum to give the crude acyl chloride thioxanthone. This solid was suspended in anhydrous THF (1 mL). Meanwhile, anhydrous THF (2 mL) was placed in a 3-necked flask, flushed with nitrogen and cooled at 0°C. Triethylamine (0.16 mL, 1.17 mmol, 3 eq.), methyl-4-mercaptobenzoate (131 mg, 0.78 mmol, 2 eq.) and the suspended acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford methyl 4-((9-oxo-9H-thioxanthene-4-carbonyl)thio)benzoate as a yellow solid (41 mg, 26%).

### S-(4-chlorophenyl) 9-oxo-9H-thioxanthene-4-carbothioate

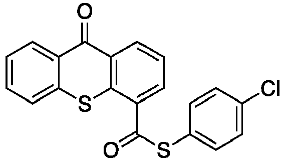

4-carboxythioxanthone (100 mg, 0.39 mmol, 1 eq.) was suspended in chloroform (2 mL), the flask and reflux condenser were flushed with nitrogen. One drop of DMF and then thionyl chloride (0.1 mL) were added to the solution and the mixture was warmed to 60°C. After 1.5 hour thionyl chloride (0.05 mL) was added to the mixture and continued reflux at 60°C for 1.5 hour, then cooled to 40°C and stirred under nitrogen overnight. The morning thionyl chloride (0.1 mL) was added and the mixture was reheated to 60°C for 1.30 hour. Then the mixture was evaporated to dryness and dried under vacuum to give the crude acyl chloride thioxanthone. This solid was suspended in anhydrous THF (1 mL). Meanwhile, anhydrous THF (2 mL) was placed in a 3-necked flask, flushed with nitrogen and cooled at 0°C. Triethylamine (0.16 mL, 1.17 mmol, 3 eq.), 4-chlorobenzenethiol (113 mg, 0.78 mmol, 2 eq.) and the suspended acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(4-chlorophenyl) 9-oxo-9H-thioxanthene-4-carbothioate as a light yellow solid (82 mg, 55%).

### Example 2: Synthesis of anthraquinone derivatives

### S-cyclohexyl 9,10-dioxo-9,10-dihydroanthracene-2-carbothioate

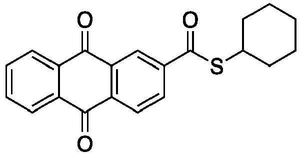

Anthraquinone-2-carboxylic acid (252 mg, 1 mmol, 1 eq.) was dissolved in thionyl chloride (2.55 mL) in a round bottom flask and stirred at 80°C for 6 hours under nitrogen. The mixture was evaporated to dryness to give the crude acyl chloride anthraquinone. This solid was dissolved in anhydrous THF (2 mL). Meanwhile, anhydrous THF (2 mL) was placed in a 3-necked flask, flushed well with nitrogen and cooled at 0°C. Triethylamine (0.42 mL, 3 mmol, 3 eq.), cyclohexane thiol (0.24 mL, 2 mmol, 2 eq.) and finally the acid chloride anthraquinone were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-cyclohexyl 9,10-dioxo-9,10-dihydroanthracene-2-carbothioate as a yellow solid (112 mg, 32%).

### S-propyl 9,10-dioxo-9,10-dihydroanthracene-2-carbothioate

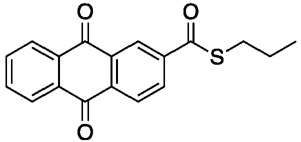

Anthraquinone-2-carboxylic acid (252 mg, 1 mmol, 1 eq.) was dissolved in thionyl chloride (2.55 mL) in a round bottom flask and stirred at 80°C for 6 hours under nitrogen. The mixture was evaporated to dryness to give the crude acyl chloride anthraquinone. This solid was dissolved in anhydrous THF (2 mL). Meanwhile, anhydrous THF (2 mL) was placed in a 3-necked flask, flushed well with nitrogen and cooled at 0°C. Triethylamine (0.42 mL, 3 mmol, 3 eq.), 1-propane thiol (0.18 mL, 2 mmol, 2 eq.) and finally the acid chloride anthraquinone were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-propyl 9,10-dioxo-9,10-dihydroanthracene-2-carbothioate as a yellow solid (116 mg, 37%).

### S-(4-cyanophenyl) 9,10-dioxo-9,10-dihydroanthracene-2-carbothioate

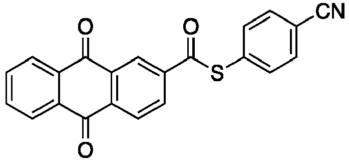

Anthraquinone-2-carboxylic acid (252 mg, 1 mmol, 1 eq.) was dissolved in thionyl chloride (2.55 mL) in a round bottom flask and stirred at 80°C for 6 hours under nitrogen. The mixture was evaporated to dryness to give the crude acyl chloride anthraquinone. This solid was dissolved in anhydrous THF (2 mL). Meanwhile, anhydrous THF (2 mL) was placed in a 3-necked flask, flushed well with nitrogen and cooled at 0°C. Triethylamine (0.42 mL, 3 mmol, 3 eq.), 4-thiobenzonitrile (270 mg, 2 mmol, 2 eq.) and finally the acid chloride anthraquinone were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(4-cyanophenyl) 9,10-dioxo-9,10-dihydroanthracene-2-carbothioate as a light yellow solid (255 mg, 69%).

### S-(4-bromophenyl) 9,10-dioxo-9,10-dihydroanthracene-2-carbothioate

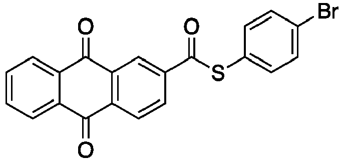

Anthraquinone-2-carboxylic acid (252 mg, 1 mmol, 1 eq.) was dissolved in thionyl chloride (2.55 mL) in a round bottom flask and stirred at 80°C for 6 hours under nitrogen. The mixture was evaporated to dryness to give the crude acyl chloride anthraquinone. This solid was dissolved in anhydrous THF (2 mL). Meanwhile, anhydrous THF (2 mL) was placed in a 3-necked flask, flushed well with nitrogen and cooled at 0°C. Triethylamine (0.42 mL, 3 mmol, 3 eq.), 4-bromobenzene thiol (378 mg, 2 mmol, 2 eq.) and finally the acid chloride anthraquinone were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(4-bromophenyl) 9,10-dioxo-9,10-dihydroanthracene-2-carbothioate as an orange solid (260 mg, 61%).

### Example 3: Synthesis of pyrene derivatives

### S-(naphthalen-2-yl) pyrene-4-carbothioate

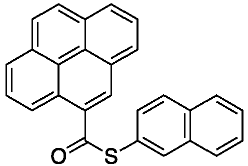

1-pyrene carboxylic acid (100 mg, 0.41 mmol, 1 eq.) was dissolved in thionyl chloride (1 mL) in a round bottom flask and stirred at 80°C for 1 hour under nitrogen. The mixture was evaporated to dryness to give the crude acyl chloride pyrene. This solid was suspended in anhydrous THF (1 mL). Meanwhile, anhydrous THF (1 mL) was placed in a 3-necked flask, flushed well with nitrogen and cooled at 0°C. Triethylamine (0.17 mL, 1.23 mmol, 3 eq.), 2-naphthalene thiol (131 mg, 0.82 mmol, 2 eq.) and finally the suspended acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(naphthalen-2-yl) pyrene-4-carbothioate as a light yellow solid (59 mg, 37%).

### S-(4-isopropylphenyl) pyrene-4-carbothioate

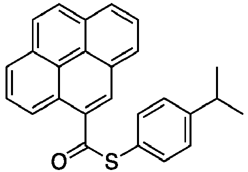

1-pyrene carboxylic acid (100 mg, 0.41 mmol, 1 eq.) was dissolved in thionyl chloride (1 mL) in a round bottom flask and stirred at 80°C for 1 hour under nitrogen. The mixture was evaporated to dryness to give the crude acyl chloride pyrene. This solid was suspended in anhydrous THF (1 mL). Meanwhile, anhydrous THF (1 mL) was placed in a 3-necked flask, flushed well with nitrogen and cooled at 0°C. Triethylamine (0.17 mL, 1.23 mmol, 3 eq.), 4-isopropylbenzene thiol (0.13 mL, 0.82 mmol, 2 eq.) and finally the suspended acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(4-isopropylphenyl) pyrene-4-carbothioate as a light yellow solid (36 mg, 23%).

### Example 4: Synthesis of anthracene derivatives :

### S-(p-tolyl) anthracene-9-carbothioate

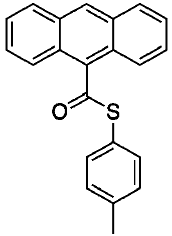

9-anthracene carboxylic acid (222 mg, 1 mmol, 1 eq.) and thionyl chloride (1.71 mL) were stirred at reflux for 3 hours under nitrogen. The mixture was evaporated to dryness to give the crude acyl chloride anthracene. This solid was suspended in anhydrous THF (2 mL). Meanwhile, anhydrous THF (2 mL) was placed in a 3-necked flask, flushed well with nitrogen and cooled at 0°C. Triethylamine (0.42 mL, 3 mmol, 3 eq.), p-toluene thiol (248 mg, 2 mmol, 2 eq.) and finally the suspended acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(p-tolyl) anthracene-9-carbothioate as a yellow solid (133 mg, 41%).

### S-(naphthalen-2-yl) anthracene-9-carbothioate

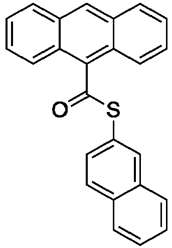

9-anthracene carboxylic acid (222 mg, 1 mmol, 1 eq.) and thionyl chloride (1.71 mL) were stirred at reflux for 3 hours under nitrogen. The mixture was evaporated to dryness to give the crude acyl chloride anthracene. This solid was suspended in anhydrous THF (2 mL). Meanwhile, anhydrous THF (2 mL) was placed in a 3-necked flask, flushed well with nitrogen and cooled at 0°C. Triethylamine (0.42 mL, 3 mmol, 3 eq.), 2-naphthalene thiol (320 mg, 2 mmol, 2 eq.) and finally the suspended acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(naphthalen-2-yl) anthracene-9-carbothioate as a yellow solid (187 mg, 51%).

### S-(4-methoxyphenyl) anthracene-9-carbothioate

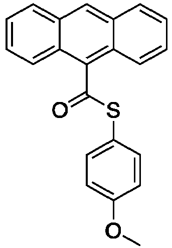

9-anthracene carboxylic acid (222 mg, 1 mmol, 1 eq.) and thionyl chloride (1.71 mL) were stirred at reflux for 3 hours under nitrogen. The mixture was evaporated to dryness to give the crude acyl chloride anthracene. This solid was suspended in anhydrous THF (2 mL). Meanwhile, anhydrous THF (2 mL) was placed in a 3-necked flask, flushed well with nitrogen and cooled at 0°C. Triethylamine (0.42 mL, 3 mmol, 3 eq.), 4-methoxybenzene thiol (0.25 mL, 2 mmol, 2 eq.) and finally the suspended acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(4-methoxyphenyl) anthracene-9-carbothioate as a light yellow solid (215 mg, 63%).

### Example 5: Synthesis of α-keto-thioesters

### S-phenyl 2-(3,4-dimethoxyphenyl)-2-oxoethanethioate

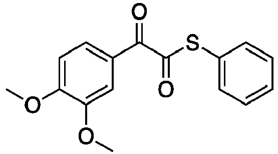

3',4'-dimethoxyacetophenone (500 mg, 2.77 mmol, 1 eq.), thiophenol (0.34 mL, 3.32 mmol, 1.2 eq.), TBAI (1.23 g, 3.32 mmol, 1.2 eq), K₂S₂O₈ (1.05 g, 3.88 mmol, 1.4 eq.) and DMSO (6 mL) were charged into a glass pressure tube and the mixture was stirred at 100°C for 1.5 hour. The mixture was cooled to room temperature, concentrated under reduced pressure and then ethyl acetate (2 x 50 mL) was added. The organic phase was washed with water (50 mL), dried with MgSO₄, filtered and the solvent was removed under reduced pressure. The obtained residue was purified by column chromatography on silica gel (cyclohexane/EtOAc 40:1) to afford S-phenyl 2-(3,4-dimethoxyphenyl)-2-oxoethanethioate as a yellow oil (352 mg, 42%).

### S-(4-(methylthio)phenyl) 2-(4-(methylthio)phenyl)-2-oxoethanethioate

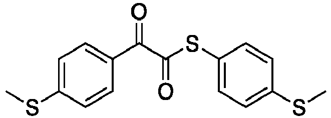

4'-(methylthio)acetophenone (500 mg, 3.01 mmol, 1 eq.), 4-(methylthio)benzene thiol (0.47 mL, 3.61 mmol, 1.2 eq.), TBAI (1.17 g, 3.61 mmol, 1.2 eq), K₂S₂O₈ (1.14 g, 4.21 mmol, 1.4 eq.) and DMSO (6 mL) were charged into a glass pressure tube and the mixture was stirred at 100°C for 1.5 hour. The mixture was cooled to room temperature, concentrated under reduced pressure and then ethyl acetate (2 x 50 mL) was added. The organic phase was washed with water (50 mL), dried with MgSO₄, filtered and the solvent was removed under reduced pressure. The obtained residue was purified by column chromatography on silica gel (cyclohexane/EtOAc 40:1) to afford S-(4-(methylthio)phenyl) 2-(4-(methylthio)phenyl)-2-oxoethanethioate as a yellow oil (423 mg, 42%).

### S-(4-methoxyphenyl) 2-(4-(methylthio)phenyl)-2-oxoethanethioate

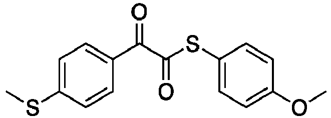

4'-(methylthio)acetophenone (200 mg, 1.20 mmol, 1 eq.), 4-methoxybenzene thiol (0.18 mL, 1.44 mmol, 1.2 eq.), TBAI (532 mg, 1.44 mmol, 1.2 eq), K₂S₂O₈ (454 mg, 1.68 mmol, 1.4 eq.) and DMSO (2 mL) were charged into a glass pressure tube and the mixture was stirred at 100°C for 1.5 hour. The mixture was cooled to room temperature, concentrated under reduced pressure and then ethyl acetate (2 x 25 mL) was added. The organic phase was washed with water (25 mL), dried with MgSO₄, filtered and the solvent was removed under reduced pressure. The obtained residue was purified by column chromatography on silica gel (cyclohexane/EtOAc 40:1) to afford S-(4-methoxyphenyl) 2-(4-(methylthio)phenyl)-2-oxoethanethioate as an orange solid (183 mg, 48%).

### S-phenyl 2-(4-(methylthio)phenyl)-2-oxoethanethioate

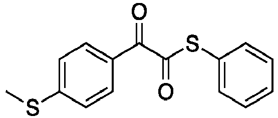

4'-(methylthio)acetophenone (200 mg, 1.20 mmol, 1 eq.), thiophenol (0.15 mL, 1.44 mmol, 1.2 eq.), TBAI (532 mg, 1.44 mmol, 1.2 eq), K₂S₂O₈ (454 mg, 1.68 mmol, 1.4 eq.) and DMSO (2 mL) were charged into a glass pressure tube and the mixture was stirred at 100°C for 1.5 hour. The mixture was cooled to room temperature, concentrated under reduced pressure and then ethyl acetate (2 x 25 mL) was added. The organic phase was washed with water (25 mL), dried with MgSO₄, filtered and the solvent was removed under reduced pressure. The obtained residue was purified by column chromatography on silica gel (cyclohexane/EtOAc 40:1) to afford S-phenyl 2-(4-(methylthio)phenyl)-2-oxoethanethioate as a yellow solid (174 mg, 50%).

### S-cyclohexyl 2-oxo-2-phenylethanethioate

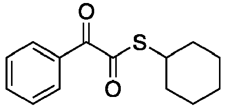

Cyclohexane thiol (0.15 mL, 1.21 mmol, 1 eq.), benzoylformic acid (200 mg, 1.33 mmol, 1.1 eq), DMAP (15 mg, 0.12 mmol, 0.1 eq.) and dichloromethane (5 mL) were taken in a round bottom flask at room temperature. The solution was then cooled at 0°C and DCC (250 mg, 1.21 mmol, 1 eq.) in dichloromethane (1 mL) was added dropwise to the stirring solution. The mixture was then allowed to warm to room temperature and stirred overnight. The precipitation was filtered off and the solvent was removed under reduced pressure. The obtained residue was purified by column chromatography on silica gel (cyclohexane/EtOAc 10:1) to afford S-cyclohexyl 2-oxo-2-phenylethanethioate as a yellow oil (84 mg, 28%).

### S-propyl 2-oxo-2-phenylethanethioate

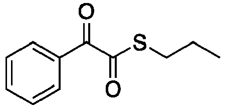

1- Propane thiol (0.11 mL, 1.21 mmol, 1 eq.), benzoylformic acid (200 mg, 1.33 mmol, 1.1 eq.), DMAP (15 mg, 0.12 mmol, 0.1 eq.) and dichloromethane (5 mL) were taken in a round bottom flask at room temperature. The solution was then cooled at 0°C and DCC (250 mg, 1.21 mmol, 1 eq.) in dichloromethane (1 mL) was added dropwise to the stirring solution. The mixture was then allowed to warm to room temperature and stirred overnight. The precipitation was filtered off and the solvent was removed under reduced pressure. The obtained residue was purified by column chromatography on silica gel (cyclohexane/EtOAc 10:1) to afford S-propyl 2-oxo-2-phenylethanethioate as a yellow oil (101 mg, 40%).

### S,S'-((ethane-1,2-diylbis(oxy))bis(ethane-2,1-diyl)) bis(2-oxo-2-phenylethanethioate)

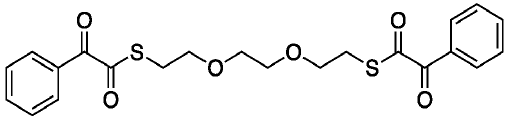

DMDO (275 mg, 1.51 mmol, 1 eq.), benzoylformic acid (500 mg, 3.33 mmol, 2.2 eq.), DMAP (37 mg, 0.30 mmol, 0.2 eq.) and dichloromethane (10 mL) were taken in a round bottom flask at room temperature. The solution was then cooled at 0°C and DCC (623 mg, 3.02 mmol, 2 eq.) in dichloromethane (5 mL) was added dropwise to the stirring solution. The mixture was then allowed to warm to room temperature and stirred overnight. The precipitation was filtered off and the solvent was removed under reduced pressure. The obtained residue was purified by column chromatography on silica gel (cyclohexane/EtOAc 10:1) to afford S,S'-((ethane-1,2-diylbis(oxy))bis(ethane-2,1-diyl)) bis(2-oxo-2-phenylethanethioate) as a yellow oil (203 mg, 30%).

### S,S'-(oxybis(ethane-2,1-diyl)) bis(2-(4-(methylthio)phenyl)-2-oxoethanethioate)

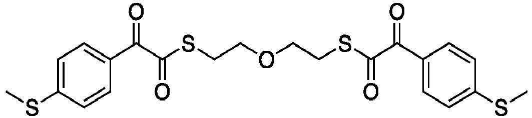

Bis (2-mercaptoethyl)ether (0.14 mL, 1.16 mmol, 1 eq.), 2-(4-(methylthio)phenyl)-2-oxoethanethioic S-acid (500 mg, 2.55 mmol, 2.2 eq.), DMAP (48 mg, 0.23 mmol, 0.2 eq.) and dichloromethane (8 mL) were taken in a round bottom flask at room temperature. The solution was then cooled at 0°C and DCC (283 mg, 2.32 mmol, 2 eq.) in dichloromethane (3 mL) was added dropwise to the stirring solution. The mixture was then allowed to warm to room temperature and stirred overnight. The precipitation was filtered off and the solvent was removed under reduced pressure. The obtained residue was purified by column chromatography on silica gel (cyclohexane/EtOAc 10:1) to afford S,S'-(oxybis(ethane-2,1-diyl)) bis(2-(4-(methylthio)phenyl)-2-oxoethanethioate) as a yellow solid (124 mg, 22%).

As an illustration, the following representative examples were synthesized by application and/or adaptation of the procedures disclosed herein:

| **Reference** | **Structure** | **Synthesis yield** |
|---|---|---|
| **3** | 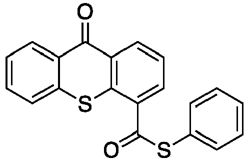 | 39% |
| **4** | 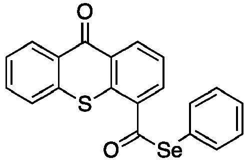 | 27% |
| **6** | 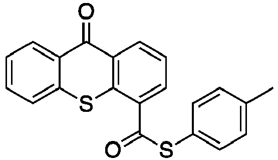 | 60% |
| **7** | 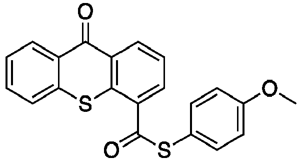 | 41% |
| **8** | 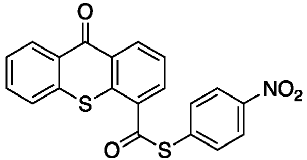 | 54% |
| **9** | 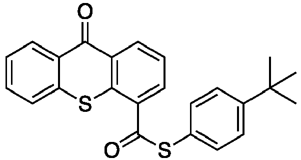 | 53% |
| **10** | 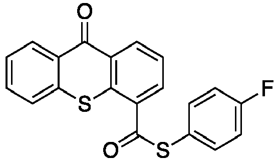 | 69% |
| **11** | 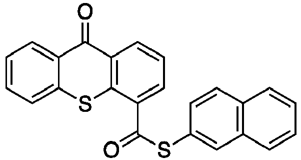 | 52% |
| **12** | 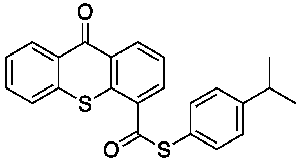 | 51% |
| **28** | 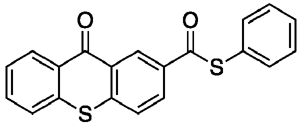 | 25% |
| **29** | 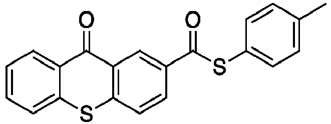 | 32% |
| **30** | 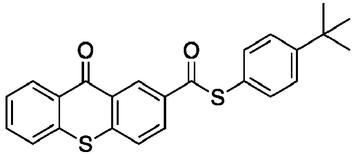 | 29% |
| **31** | 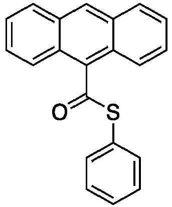 | 30% |
| **32** | 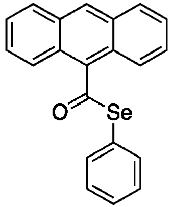 | 12% |
| **33** | 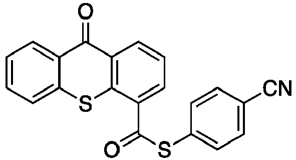 | 58% |
| **34** | 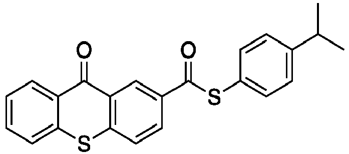 | 37% |
| **35** | 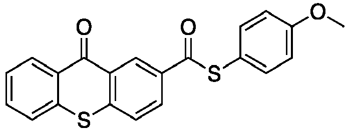 | 47% |
| **36** | 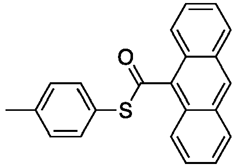 | 41% |
| 37 | 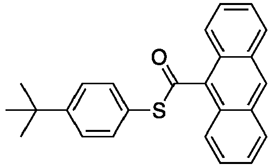 | 31% |
| 38 | 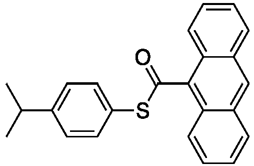 | 16% |
| 39 | 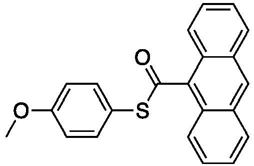 | 63% |
| 40 | 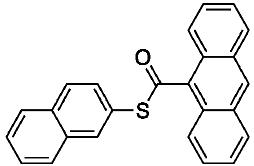 | 51% |
| 41 | 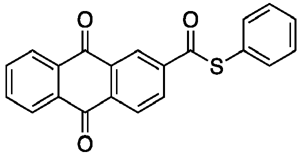 | 44% |
| 42 | 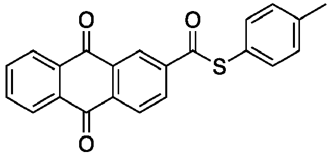 | 50% |
| 43 | 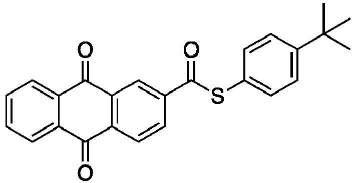 | 64% |
| **44** | 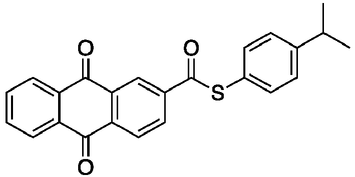 | 48% |
| **45** | 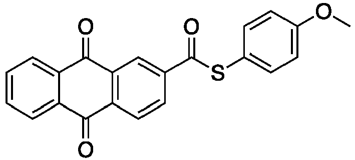 | 65% |
| **46** | 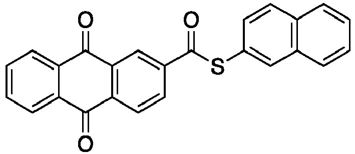 | 69% |
| **47** | 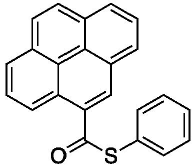 | 23% |
| **48** | 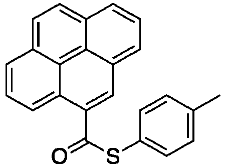 | 30% |
| **49** | 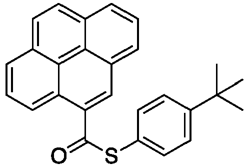 | 24% |
| **50** | 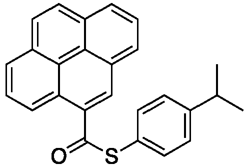 | 23% |
| **51** | 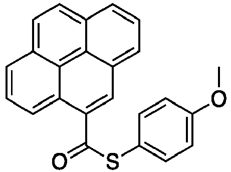 | 32% |
| **52** | 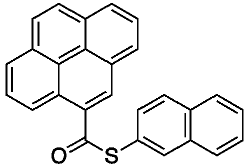 | 37% |
| **53** | 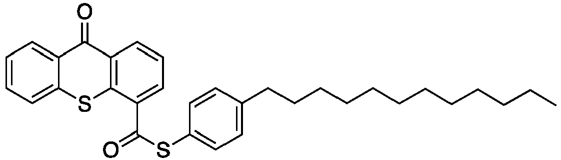 | 57% |
| **54** | 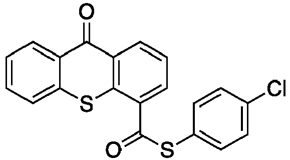 | 55% |
| **55** | 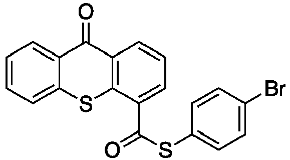 | 71% |
| **56** | 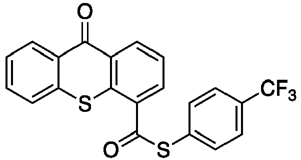 | 57% |
| **57** | 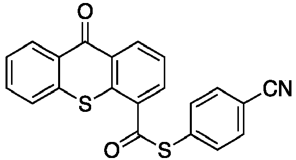 | 58% |
| **58** | 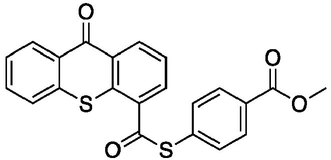 | 26% |
| **59** | 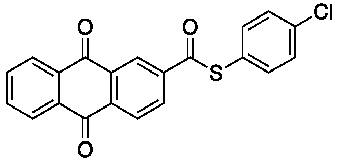 | 67% |
| **60** | 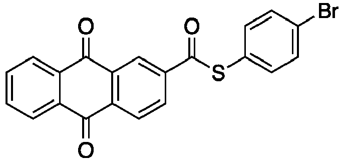 | 61% |
| **61** | 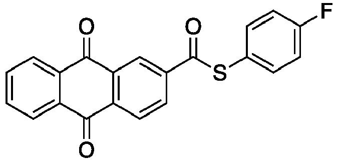 | 48% |
| **62** | 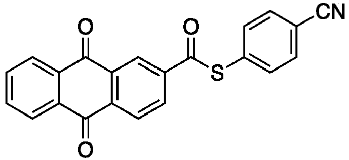 | 69% |
| **63** | 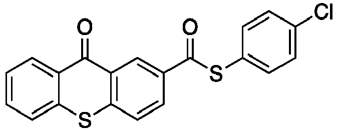 | 25% |
| **64** | 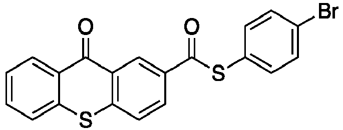 | 54% |
| **65** | 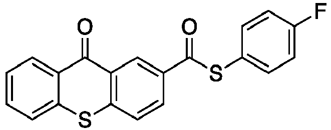 | 33% |
| **66** | 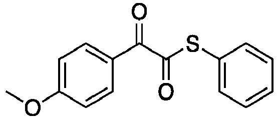 | 48% |
| **67** | 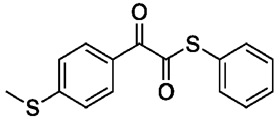 | 50% |
| **68** | 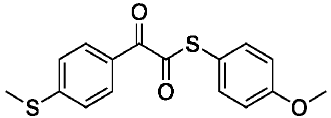 | 48% |
| **69** | 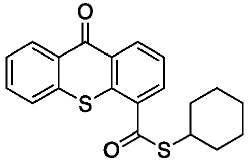 | 50% |
| **70** | 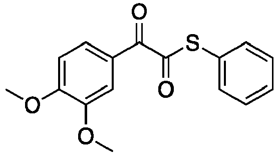 | 42% |
| **71** | 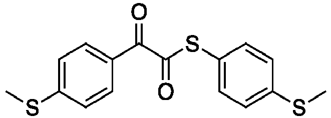 | 42% |
| **72** | 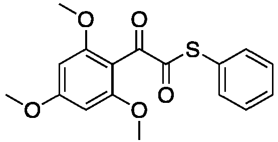 | 50% |
| **73** | 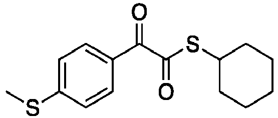 | 32% |
| **74** | 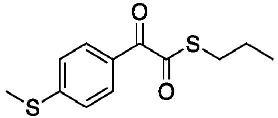 | 43% |
| **75** | 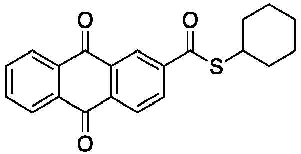 | 32% |
| **76** | 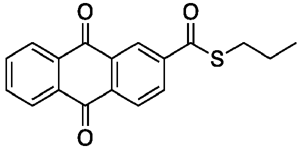 | 37% |
| **77** | 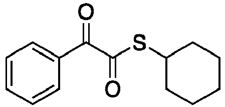 | 28% |
| **78** | 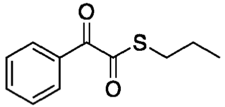 | 40% |
| **79** | 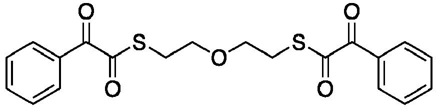 | 21% |
| **80** | 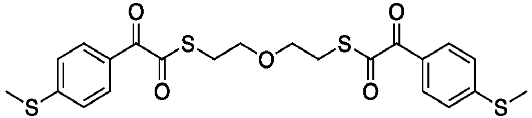 | 22% |
| **81** | 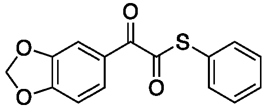 | 36% |
| **82** | 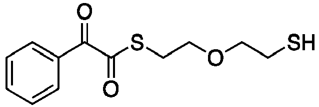 | 43% |
| **83** | 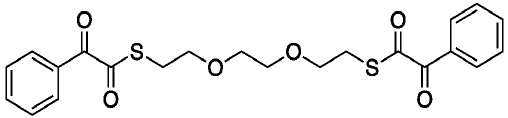 | 30% |
| **84** | 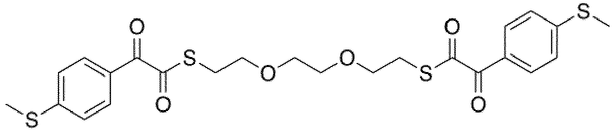 | Obtained as a mixture of **84** and **86** |
| **85** | 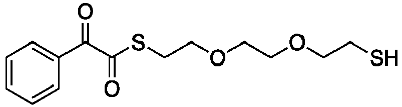 | 81% |
| **86** | 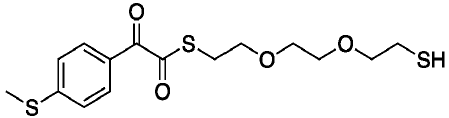 | Obtained as a mixture of **84** and **86** |
| **87** | 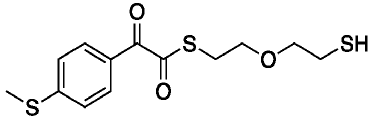 | 68% |

### Example 6: Curing performances

The curing performance of photoinitiators (Pls) was assessed using real time FTIR measurement.

The photoinitiators were dissolved at 1wt% in trimethylolpropane triacrylate (TMPTA) or ethoxylated trimethylolpropane triacrylate (SR454) at 20°C and were stirred overnight.

The photosensitive formulations were deposited on a polypropylene film and the sample thickness was controlled using a calibrated bar (24 µm, 12 µm or 6 µm). The samples were polymerized under air or in laminate (between two polypropylene films) using a LED@365nm (I₀ = 120 mW.cm⁻²) or 385nm (I₀ = 160 mW.cm⁻²) or 405nm (I₀ = 110 mW.cm⁻²) at room temperature.

For radical polymerization, the evolution or the double bond content of the acrylate function was continuously followed by real-time Fourier Transform Infrared (FTIR) spectroscopy (JASCO FTIR 6600) at about 1630 cm⁻¹ for thin samples. The conversion rates were measured over time.

The L*, a*, b* values of the photosensitive formulations were determined before and after photopolymerization using a spectrophotometer from Thorlabs in transmission experiments.

### Acrylate conversion (1% of photoinitiator)

Tables 1a and 1b show the acrylate conversion (Final conversion (%)) and polymerisation rate (Rp) in laminate and under air at 3 wavelengths in 2 different acrylates (SR454-ethoxylated TMPTA and TMPTA)

**Table 1a :**

| | **SR454** | | | | | | | | | | | |
|---|---|---|---|---|---|---|---|---|---|---|---|---|
| | **Final conversion (%) - laminate** | | | **Rp (/s) - laminate** | | | **Final conversion (%) - air** | | | **Rp (/s) - air** | | |
| | **365 nm** | **385 nm** | **405 nm** | **365 nm** | **385 nm** | **405 nm** | **365 nm** | **385 nm** | **405 nm** | **365 nm** | **385 nm** | **405 nm** |
| **3** | | 88% | 73% | | 12.08 | 5.14 | | 65% | 34% | | 3.62 | 0.35 |
| **4** | | 76% | 41% | | 1.93 | 2.51 | | | | | | |
| **6** | | 91% | 76% | | 13.12 | 7.47 | | 54% | 45% | | 1.36 | 0.27 |
| **7** | | 87% | 73% | | 11.83 | 4.81 | | | | | | |
| **8** | | 66% | 49% | | 6.08 | 0.16 | | | | | | |
| **9** | | 85% | 75% | | 10.56 | 6.29 | | | | | | |
| **10** | | 93% | 81% | | 12.57 | 11.39 | | 66% | 66% | | 3.25 | 0.77 |
| **11** | | 88% | 67% | | 12.09 | 2.54 | | | | | | |
| **12** | | | | | | | | 48% | 53% | | 1.24 | 0.43 |
| **28** | | 87% | 61% | | 12.31 | 5.04 | | 55% | 48% | | 4.40 | 0.32 |
| **29** | | 86% | 60% | | 15.44 | 2.96 | | 59% | 23% | | 2.50 | 0.12 |
| **30** | | 87% | 68% | | 13.38 | 3.27 | | | | | | |
| **31** | | 72% | 54% | | 1.43 | 2.10 | | | | | | |
| **32** | | 38% | 36% | | 1.24 | 0.81 | | | | | | |
| **33** | | 83% | 65% | | 8.22 | 0.26 | | | | | | |
| **34** | | 92% | 72% | | 13.78 | 5.62 | | 59% | 62% | | 11.08 | 0.60 |
| **35** | | 73% | 43% | | 6.41 | 0.28 | | | | | | |
| **36** | | | | | | | | | | | | |
| **37** | | | | | | | | | | | | |
| **38** | | | | | | | | | | | | |
| **39** | | | | | | | | | | | | |
| **40** | | | | | | | | | | | | |
| **41** | 88% | 88% | 75% | 10.97 | 10.63 | 3.78 | 74% | 70% | 68% | 4.39 | 3.69 | 1.17 |
| **42** | 78% | 76% | 66% | 6.01 | 5.50 | 0.76 | 59% | 67% | 66% | 1.41 | 1.52 | 0.58 |
| **43** | 87% | 87% | 60% | 10.67 | 9.73 | 7.47 | 69% | 82% | 64% | 3.92 | 6.21 | 0.59 |
| **44** | 85% | 86% | 69% | 9.76 | 9.51 | 5.51 | 75% | 78% | 75% | 6.28 | 4.93 | 0.77 |
| **45** | 84% | 83% | 64% | 5.17 | 6.12 | 0.65 | 67% | 72% | 73% | 1.76 | 2.39 | 0.97 |
| **46** | 82% | 78% | 75% | 8.13 | 6.32 | 0.29 | | | | | | |
| **47** | | 92% | 66% | | 6.56 | 1.38 | | | | | | |
| **48** | | 93% | 67% | | 7.79 | 3.21 | | | | | | |
| **49** | | 96% | 74% | | 7.50 | 0.29 | | | | | | |
| **50** | | 95% | 52% | | 4.81 | 0.19 | | | | | | |
| **51** | | 95% | 61% | | 8.67 | 1.42 | | | | | | |
| **52** | | 93% | 70% | | 7.97 | 1.70 | | | | | | |
| **53** | | 74% | 58% | | 7.56 | 4.63 | | | | | | |
| **54** | | 82% | 67% | | 12.60 | 4.75 | | | | | | |
| **55** | | 52% | 68% | | 5.24 | 4.14 | | | | | | |
| **56** | | 77% | 66% | | 10.40 | 6.02 | | | | | | |
| **58** | | 86% | 78% | | 13.33 | 9.99 | | | | | | |
| **59** | | 82% | 67% | | 5.27 | 1.65 | | | | | | |
| **60** | | 79% | 44% | | 4.98 | 1.68 | | | | | | |
| **61** | 81% | 87% | 76% | 7.61 | 8.61 | 3.03 | 69% | 77% | 62% | 2.65 | 2.48 | 1.03 |
| **62** | | 63% | 44% | | 1.63 | 0.74 | | | | | | |
| **63** | | 81% | 22% | | 8.24 | 0.32 | | | | | | |
| **64** | | 69% | 60% | | 5.48 | 0.97 | | | | | | |
| **65** | | 91% | 71% | | 10.44 | 4.73 | | 71% | 58% | | 7.30 | 0.55 |
| **66** | 81% | 70% | 14% | 6.21 | 2.44 | 0.13 | 65% | 61% | 57% | 1.88 | 1.10 | 0.51 |
| **67** | 87% | 84% | 37% | 6.89 | 8.71 | 0.24 | 82% | 81% | 73% | 5.82 | 4.99 | 1.19 |
| **68** | 78% | 82% | 17% | 5.46 | 4.61 | 0.10 | 83% | 81% | 62% | 3.71 | 3.15 | 0.49 |
| **69** | | 84% | 75% | | 10.38 | 5.73 | | | | | | |
| **70** | 87% | 81% | 53% | 8.08 | 7.99 | 0.72 | 84% | 72% | 43% | 4.51 | 2.09 | 0.72 |
| **71** | 84% | 76% | 38% | 5.41 | 4.75 | 0.18 | 73% | 75% | 59% | 4.99 | 2.21 | 0.76 |
| **72** | 69% | 76% | 42% | 3.84 | 1.96 | 0.28 | 82% | 76% | 74% | 2.43 | 1.39 | 0.60 |
| **73** | 79% | 62% | 52% | 0.56 | 1.29 | 0.26 | 84% | 90% | 61% | 2.05 | 5.12 | 0.30 |
| **74** | 74% | 72% | 75% | 0.62 | 2.65 | 0.31 | 89% | 90% | 75% | 4.18 | 4.02 | 0.48 |
| **75** | 84% | 81% | 73% | 10.04 | 9.28 | 4.56 | 71% | 63% | 49% | 6.09 | 3.37 | 0.44 |
| **76** | 89% | 89% | 80% | 10.92 | 10.25 | 4.61 | 64% | 63% | 67% | 5.84 | 3.40 | 0.64 |
| **77** | 77% | 76% | 22% | 4.80 | 5.66 | 0.17 | 78% | 69% | 69% | 3.25 | 0.66 | 0.71 |
| **78** | 71% | 75% | 20% | 4.57 | 6.42 | 0.21 | 61% | 67% | 69% | 0.9 | 0.55 | 0.57 |
| **79** | 66% | 57% | 21% | 0.69 | 0.68 | 0.33 | 64% | 68% | 71% | 0.32 | 0.74 | 0.33 |
| **81** | 85% | 81% | 43% | 10.49 | 6.33 | 0.38 | 85% | 79% | 72% | 5.19 | 3.71 | 0.64 |
| **82** | 79% | 75% | 68% | 10.73 | 7.85 | 0.79 | 55% | 54% | 66% | 0.36 | 0.39 | 0.27 |
| **83** | 57% | 34% | 39% | 0.74 | 0.36 | 0.20 | 45% | 62% | 66% | 0.22 | 2.59 | 0.35 |
| **85** | 79% | 80% | 74% | 8.30 | 10.92 | 1.34 | 77% | 74% | 63% | 0.8 | 0.5 | 0.32 |

**Table 1b :**

| | **TMPTA** | | | | | | | | | | | |
|---|---|---|---|---|---|---|---|---|---|---|---|---|
| | **Final conversion (%) laminate** | | | **Rp (/s) - laminate** | | | **Final conversion (%) - air** | | | **Rp (/s) - air** | | |
| | **365 nm** | **385 nm** | **405 nm** | **365 nm** | **385 nm** | **405 nm** | **365 nm** | **385 nm** | **405 nm** | **365 nm** | **385 nm** | **405 nm** |
| **3** | | 45% | 49% | | 6.36 | 1.84 | | 65% | 34% | | 3.62 | 0.35 |
| **4** | | 4% | 6% | | / | / | | | | | | |
| **6** | | 64% | 50% | | 9.44 | 5.07 | | 54% | 45% | | 1.36 | 0.27 |
| **7** | | 47% | 41% | | 4.52 | 1.65 | | | | | | |
| **8** | | | 30% | | | 0.22 | | | | | | |
| **9** | | 30% | 31% | | 3.01 | 1.84 | | | | | | |
| **10** | | 44% | 42% | | 6.24 | 2.38 | | 66% | 66% | | 3.25 | 0.77 |
| **11** | | 52% | 37% | | 4.18 | 0.57 | | | | | | |
| **12** | | 71% | 58% | | 10.42 | 6.27 | | 48% | 53% | | 1.24 | 0.43 |
| **28** | | 62% | 51% | | 9.33 | 0.76 | | 55% | 48% | | 4.40 | 0.32 |
| **29** | | 67% | 43% | | 7.27 | 0.74 | | 59% | 23% | | 2.50 | 0.12 |
| **30** | | 71% | 47% | | 8.38 | 1.62 | | | | | | |
| **31** | | 58% | 42% | | 1.39 | 0.49 | | | | | | |
| **32** | | 29% | 57% | | 0.81 | 0.14 | | | | | | |
| **33** | | 34% | 27% | | 2.52 | 0.09 | | | | | | |
| **34** | | 76% | 43% | | 9.58 | 1.13 | | 59% | 62% | | 11.08 | 0.60 |
| **35** | | 41% | 38% | | 3.04 | 0.69 | | | | | | |
| **36** | | 52% | 21% | | 1.17 | 0.30 | | | | | | |
| **37** | | 48% | 42% | | 1.47 | 0.42 | | | | | | |
| **38** | | 54% | 54% | | 1.30 | 0.83 | | | | | | |
| **39** | | 47% | 30% | | 2.45 | 2.51 | | | | | | |
| **40** | | 32% | 15% | | 3.58 | 0.04 | | | | | | |
| **41** | 70% | 73% | 52% | 8.32 | 7.62 | 2.90 | | | | | | |
| **42** | 57% | 48% | 46% | 0.14 | 3.44 | 0.26 | | | | | | |
| **43** | 69% | 67% | 46% | 7.83 | 8.79 | 3.70 | | | | | | |
| **44** | 63% | 70% | 52% | 8.54 | 8.14 | 2.07 | 47% | 36% | 33% | 0.56 | 0.52 | 0.17 |
| **45** | 61% | 55% | 48% | 3.03 | 5.67 | 0.21 | | | | | | |
| **46** | 62% | 52% | 34% | 5.69 | 5.31 | 0.73 | | | | | | |
| **47** | | 59% | 37% | | 2.55 | 0.54 | | | | | | |
| **48** | | 76% | 40% | | 5.68 | 0.49 | | | | | | |
| **49** | | 78% | 53% | | 7.09 | 0.26 | | | | | | |
| **50** | | 78% | 16% | | 4.74 | 0.13 | | | | | | |
| **51** | | 82% | 53% | | 4.75 | 0.60 | | | | | | |
| **52** | | 33% | 47% | | 2.77 | 0.65 | | | | | | |
| **53** | | 43% | 29% | | 5.74 | 2.04 | | | | | | |
| **54** | | 50% | 32% | | 6.76 | 1.91 | | | | | | |
| **55** | | 48% | 24% | | 3.97 | 1.00 | | | | | | |
| **56** | | | 41% | | | 2.23 | | | | | | |
| **58** | | | 54% | | | 4.39 | | | | | | |
| **59** | | 46% | 42% | | 2.06 | 0.66 | | | | | | |
| **60** | | 56% | 32% | | 3.70 | 0.44 | | | | | | |
| **61** | | 58% | 47% | | 4.74 | 1.79 | | | | | | |
| **62** | | 59% | 45% | | 2.78 | 0.66 | | | | | | |
| **63** | | 46% | 10% | | 3.56 | 0.25 | | | | | | |
| **64** | | 43% | 26% | | 2.81 | 0.82 | | | | | | |
| **65** | | 59% | 35% | | 8.68 | 1.22 | | 71% | 58% | | 7.30 | 0.55 |
| **66** | | 49% | 4% | | 0.80 | 0.05 | | | | | | |
| **67** | 49% | 62% | 18% | 9.75 | 5.06 | 0.11 | 70% | 83% | 61% | 4.10 | 5.27 | 0.53 |
| **68** | 61% | 62% | 40% | 4.02 | 3.71 | 0.21 | 53% | 70% | 69% | 2.50 | 3.48 | 0.47 |
| **69** | | 63% | 49% | | 5.17 | 0.86 | | | | | | |
| **70** | 61% | 52% | 41% | 2.36 | 6.04 | 0.23 | 44% | 58% | 57% | 0.71 | 1.14 | 0.54 |
| **71** | 48% | 52% | 14% | 2.65 | 2.13 | 0.08 | 59% | 80% | 34% | 1.78 | 3.03 | 0.28 |
| **72** | 27% | 32% | 38% | 4.03 | 0.81 | 0.51 | 69% | 65% | 60% | 0.84 | 0.86 | 0.63 |
| **73** | 32% | 60% | 9% | 0.22 | 0.85 | 0.05 | 63% | 73% | 63% | 0.35 | 1.11 | 0.33 |
| **74** | 53% | 27% | 12% | 0.27 | 0.17 | 0.07 | 83% | 88% | 50% | 2.67 | 3.45 | 0.24 |
| **75** | 57% | 59% | 52% | 4.66 | 4.44 | 0.71 | 62% | 55% | 53% | 1.83 | 0.74 | 0.28 |
| **76** | 60% | 71% | 53% | 10.07 | 5.61 | 2.00 | 43% | 52% | 35% | 0.66 | 1.48 | 0.15 |
| **77** | 50% | 44% | 1% | 1.59 | 0.81 | / | 54% | 54% | 52% | 0.37 | 0.24 | 0.37 |
| **78** | 36% | 36% | 25% | 1.59 | 0.94 | 0.12 | 44% | 55% | 58% | 0.38 | 0.32 | 0.39 |
| **79** | 22% | 20% | 3% | 0.15 | 0.13 | / | 62% | 57% | 48% | 0.52 | 0.5 | 0.42 |
| **81** | 67% | 60% | 7% | 2.40 | 4.09 | / | 58% | 63% | 61% | 2.55 | 1.50 | 0.50 |
| **82** | 46% | 49% | 17% | 3.80 | 4.31 | 0.13 | 58% | 51% | 40% | 0.56 | 0.48 | 0.24 |

### Concentration of Photoinitiator (0.5%, 1% , 2%)

Table 2a, 2b and 2c below show the concentration effect of Pls in SR454 (ethoxylated TMPTA) under three different wavelengths 365nm, 385 nm and 405 nm (FC stands for Final conversion and Rp stands for polymerisation rate).

**Table 1c :**

| | **LED@365nm - SR454** | | | | | | | | | | | |
|---|---|---|---|---|---|---|---|---|---|---|---|---|
| | **0.5 wt% PI** | | | | **1 wt% PI** | | | | **2 wt% PI** | | | |
| | **Laminate** | | **Air** | | **Laminate** | | **Air** | | **Laminate** | | **Air** | |
| | **FC (%)** | **Rp** | **FC (%)** | **Rp** | **FC (%)** | **Rp** | **FC (%)** | **Rp** | **FC (%)** | **Rp** | **FC (%)** | **Rp** |
| **67** | 79% | 6.26 | 77% | 2.84 | 86% | 8.44 | 85% | 3.67 | 92% | 8.11 | 88% | 3.81 |
| **73** | 69% | 0.43 | 83% | 1.67 | 71% | 0.71 | 83% | 2.43 | 75% | 0.76 | 88% | 2.23 |
| **74** | 53% | 0.54 | 69% | 2.62 | 80% | 1.02 | 86% | 3.05 | 86% | 2.30 | 86% | 3.01 |
| **79** | 51% | 0.53 | 60% | 0.61 | 64% | 0.92 | 76% | 1.83 | 73% | 2.19 | 59% | 0.24 |
| **81** | 79% | 6.53 | 77% | 2.14 | 84% | 13.18 | 78% | 2.40 | 91% | 7.67 | 79% | 3.32 |
| **82** | 72% | 6.17 | 8% | 0.03 | 79% | 9.53 | 53% | 0.81 | 83% | 10.42 | 39% | 0.16 |
| **MBF** | 82% | 9.80 | 68% | 4.32 | 78% | 13.29 | 65% | 4.74 | 79% | 9.30 | 69% | 4.59 |
| **TPO-L** | 85% | 12.18 | 75% | 8.47 | 84% | 15.14 | 73% | 9.49 | 89% | 12.36 | 50% | 4.28 |

**Table 2b :**

| | **LED@385nm - SR454** | | | | | | | | | | | |
|---|---|---|---|---|---|---|---|---|---|---|---|---|
| | **0.5% PI** | | | | **1% PI** | | | | **2% PI** | | | |
| | **Laminate** | | **Air** | | **Laminate** | | **Air** | | **Laminate** | | **Air** | |
| | **FC (%)** | **Rp** | **FC (%)** | **Rp** | **FC (%)** | **Rp** | **FC (%)** | **Rp** | **FC (%)** | **Rp** | **FC (%)** | **Rp** |
| **67** | 81% | 7.25 | 80% | 3.27 | 87% | 8.99 | 87% | 4.60 | 91% | 9.45 | 84% | 4.24 |
| **73** | 20% | 0.11 | 71% | 1.04 | 61% | 0.42 | 85% | 2.39 | 81% | 1.48 | 82% | 2.36 |
| **74** | 36% | 0.19 | 71% | 0.98 | 69% | 0.49 | 76% | 1.35 | 78% | 0.96 | 90% | 3.69 |
| **79** | 43% | 0.47 | 65% | 0.52 | 38% | 0.40 | 79% | 0.83 | 64% | 1.00 | 77% | 0.91 |
| **81** | 78% | 4.14 | 84% | 2.13 | 81% | 4.66 | 85% | 2.94 | 88% | 7.14 | 75% | 2.39 |
| **82** | 79% | 5.76 | 60% | 0.44 | 80% | 10.21 | 64% | 0.59 | 84% | 12.02 | 62% | 1.32 |
| **MBF** | 67% | 7.19 | 56% | 0.55 | 78% | 8.62 | 67% | 3.01 | 83% | 12.04 | 73% | 5.04 |
| **TPO-L** | 83% | 12.84 | 72% | 9.75 | 86% | 13.83 | 75% | 10.60 | 88% | 14.77 | 58% | 7.50 |

**Table 2c :**

| | **LED@405nm - SR454** | | | | | | | | | | | |
|---|---|---|---|---|---|---|---|---|---|---|---|---|
| | **0.5% PI** | | | | **1% PI** | | | | **2% PI** | | | |
| | **Laminate** | | **Air** | | **Laminate** | | **Air** | | **Laminate** | | **Air** | |
| | **FC (%)** | **Rp** | **FC (%)** | **Rp** | **FC (%)** | **Rp** | **FC (%)** | **Rp** | **FC (%)** | **Rp** | **FC (%)** | **Rp** |
| **67** | 37% | 0.34 | 72% | 0.63 | 42% | 0.29 | 73% | 1.09 | 51% | 0.54 | 79% | 0.94 |
| **73** | 7% | 0.06 | 78% | 0.71 | 16% | 0.10 | 61% | 0.48 | 12% | 0.06 | 76% | 0.76 |
| **74** | 11% | 0.08 | 74% | 0.64 | 13% | 0.14 | 75% | 0.66 | 8% | 0.05 | 79% | 0.84 |
| **79** | 24% | 0.12 | 72% | 0.61 | 4% | 0.06 | 71% | 0.52 | 4% | 0.03 | 41% | 0.30 |
| **81** | 26% | 0.12 | 76% | 0.68 | 20% | 0.09 | 72% | 0.59 | 42% | 0.19 | 72% | 0.70 |
| **82** | 52% | 0.55 | 71% | 0.59 | 65% | 0.44 | 66% | 0.49 | 72% | 2.49 | 64% | 0.52 |
| **MBF** | 55% | 0.62 | 65% | 0.46 | 72% | 1.27 | 65% | 0.63 | 77% | 3.79 | 82% | 1.00 |
| **TPO-L** | 80% | 7.18 | 60% | 0.55 | 81% | 6.88 | 55% | 0.47 | 85% | 10.88 | 68% | 2.63 |

### Color Index

Table 3 details the photobleaching behaviour of the investigated molecules compared to state-of-the-art Speedcure TPO-L. Photobleaching behaviour is important for applications such as clear coatings, white inks and overprint varnishes. It is also important in 3D printing, where yellowness inhibits full depth cure, blocking the light from the printer.

| | **LED@385nm - SR454 - thin samples** | | | | **LED@385nm - TMPTA - thin samples** | | | | **LED@385nm - SR454 - thick samples (1.4mm)** | |
|---|---|---|---|---|---|---|---|---|---|---|
| | **Laminate** | | **Air** | | **Laminate** | | **Air** | | **Air** | |
| | **Before** | **After** | **Before** | **After** | **Before** | **After** | **Before** | **After** | **Before** | **After** |
| **44** | 5.80 | 10.37 | 5.69 | 12.45 | 6.28 | 8.68 | 5.42 | 5.47 | | |
| **67** | 6.83 | 6.08 | 9.10 | 5.85 | 6.98 | 5.35 | 7.27 | 5.66 | 52.56 | 40.85 |
| **70** | 7.34 | 5.16 | 6.65 | 5.46 | 7.60 | 5.21 | 5.66 | 5.25 | | |
| **72** | 5.81 | 5.21 | 5.46 | 5.53 | 5.95 | 5.33 | 5.02 | 4.72 | | |
| **73** | 6.08 | 5.21 | 5.93 | 5.49 | 5.30 | 5.09 | 6.08 | 5.69 | 47.67 | 45.34 |
| **74** | 6.20 | 5.28 | 6.99 | 5.53 | 7.16 | 5.57 | 5.50 | 4.58 | 38.94 | 33.46 |
| **75** | 5.73 | 7.07 | 6.99 | 6.03 | 7.45 | 9.78 | 6.27 | 5.47 | | |
| **76** | 5.76 | 11.28 | 5.45 | 9.98 | 5.48 | 15.33 | 5.98 | 5.83 | | |
| **77** | 5.32 | 5.28 | 5.84 | 5.98 | 5.85 | 5.24 | 6.01 | 5.87 | | |
| **78** | 5.69 | 5.86 | 5.70 | 5.72 | 5.38 | 5.28 | 5.35 | 5.17 | | |
| **79** | 4.99 | 4.84 | 5.21 | 4.43 | 4.82 | 4.66 | 5.14 | 4.85 | 11.92 | 13.95 |
| **81** | 7.87 | 4.63 | 5.83 | 4.53 | 9.16 | 5.18 | 5.18 | 5.01 | 55.27 | 32.76 |
| **82** | 4.91 | 4.94 | 4.62 | 4.81 | 4.88 | 5.28 | 4.40 | 4.16 | 5.23 | 7.45 |
| **TPO-L** | 5.29 | 5.15 | 5.48 | 5.13 | 5.34 | 5.55 | 5.53 | 5.43 | 5.12 | 7.42 |

### Thickness

Table 4 below details the ability of each PI to depth cure at 385nm at 4 different thicknesses. This ties in with Table 3 and ability to photobleach.

**Table 4**

| | **LED@385nm - SR454** | | | | | | | |
|---|---|---|---|---|---|---|---|---|
| | **6 µm** | | **12 µm** | | **24 µm** | | **1.4 mm** | |
| | **FC (%)** | **Rp** | **FC (%)** | **Rp** | **FC (%)** | **Rp** | **FC (%)** | **Rp** |
| **67** | 73% | 3.05 | 82% | 4.86 | 81% | 5.03 | 46% | 0.21 |
| **73** | 71% | 2.11 | 86% | 3.08 | 88% | 3.39 | No polym | No polym |
| **74** | 79% | 2.63 | 85% | 3.08 | 88% | 3.39 | No polym | No polym |
| **79** | 66% | 0.41 | 73% | 0.78 | 68% | 0.76 | 92% | 2.43 |
| **81** | 78% | 2.01 | 79% | 3.06 | 79% | 3.85 | 89% | 0.20 |
| **82** | 47% | 0.23 | 56% | 1.93 | 54% | 0.49 | 98% | 13.12 |
| **MBF** | 42% | 0.20 | 46% | 0.40 | 60% | 3.67 | 98% | 13.23 |
| **TPO-L** | 73% | 3.05 | 82% | 4.86 | 81% | 5.03 | 100% | 14.34 |

### Stability (40°C)

The stability was measured for PIs at 40°C over 4 weeks. This is done by comparing acrylate conversion and polymerization rate before and after ageing in both laminate and air. These are compared to state-of-the-art Speedcure TPO-L, where phosphine oxides are known in the art for their long term instability in certain formulations, the thioesters described here largely outperform TPO-L after 4 weeks. Results are summarized in Table 5 (FC stands for Final conversion and Rp stands for polymerisation rate):

**Table 5**

| | **LED@385nm - SR454** | | | | | | | | | | | |
|---|---|---|---|---|---|---|---|---|---|---|---|---|
| | **Fresh** | | | | **After 2 weeks at 40°C** | | | | **After 4 weeks at 40°C** | | | |
| | **Laminate** | | **Air** | | **Laminate** | | **Air** | | **Laminate** | | **Air** | |
| | **FC (%)** | **Rp** | **FC (%)** | **Rp** | **FC (%)** | **Rp** | **FC (%)** | **Rp** | **FC (%)** | **Rp** | **FC (%)** | **Rp** |
| **67** | 88% | 14.13 | 81% | 5.03 | 86% | 8.88 | 84% | 5.00 | 89% | 10.65 | 76% | 3.46 |
| **73** | 68% | 0.83 | 88% | 3.39 | 79% | 1.77 | 87% | 3.19 | 73% | 0.35 | 85% | 3.28 |
| **74** | 79% | 2.28 | 87% | 4.15 | 79% | 0.65 | 84% | 3.55 | 82% | 0.65 | 87% | 3.37 |
| **79** | 57% | 0.87 | 68% | 0.76 | 70% | 1.84 | 55% | 0.67 | 75% | 7.55 | 66% | 1.19 |
| **81** | 83% | 6.70 | 79% | 3.85 | 83% | 6.72 | 85% | 4.13 | 65% | 2.06 | 66% | 0.66 |
| **82** | 75% | 10.13 | 54% | 0.49 | 78% | 9.05 | 68% | 1.89 | 85% | 2.38 | 69% | 2.81 |
| **MBF** | 77% | 12.92 | 60% | 3.67 | 75% | 10.53 | 64% | 4.67 | 88% | 13.64 | 74% | 8.58 |
| **TPO-L** | 87% | 14.50 | 63% | 9.09 | 87% | 16.86 | 73% | 8.55 | 80% | 7.09 | 58% | 1.49 |

## Claims

1. A compound of formula (I), (II) or (III): 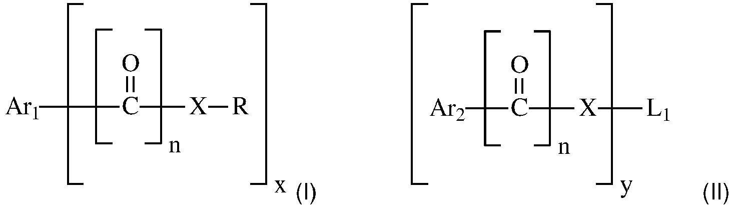 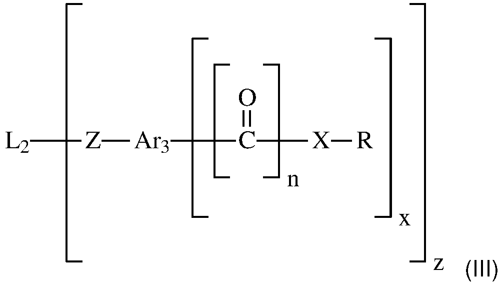 wherein:
each n is independently 1 or 2;
each x is independently an integer from 1 to 6;
each y and z is independently an integer from 2 to 6;
each X is independently S, Se or Te;
each Ar₁, Ar₂ and Ar₃ is independently an optionally substituted aryl provided that:
- each -[C(=O)]ₙ-X-R moiety is directly connected to an aromatic ring having 6 carbon ring atoms;
- when Ar₁ is an optionally substituted phenyl then n is equal to 2;
each R is independently chosen from the group consisting of:
- an optionally substituted linear or branched -(C₁-C₂₀)alkyl, said alkyl being optionally interrupted by one or more groups chosen from -O-, -C(=O)- and -S-;
- an optionally substituted linear or branched -(C₂-C₂₀)alkenyl, said alkenyl being optionally interrupted by one or more groups chosen from -O- and -C(=O)-;
- an optionally substituted -(C₅-C₁₂)aryl; and
- an optionally substituted -(C₃-C₁₂)cycloalkyl said cycloalkyl being optionally interrupted by one or more heteroatoms independently chosen from O, N and S;
each Z is independently a bond, -CH₂-, -O-, -S-, -NR¹-, -C(=O)-O-, -O-C(=O)-, -C(=O)-S-, -S-C(=O)-, -C(=O)-NR¹-, -NR¹-C(=O)-, -O-C(=O)-NR¹-, -NR¹-C(=O)-O-, -O-C(=O)-O-, -NR¹-C(=O)-NR¹-, wherein each R¹ is independently H, a linear or branched -(C₁-C₆)alkyl or a -(C₅-C₁₂)aryl;
L₁ is a y-valent linker; and
L₂ is a z-valent linker.

2. A compound according to claim 1 wherein each Ar₁, Ar₂ and Ar₃ is independently an optionally substituted aryl selected from phenyl, naphthyl, anthracenyl, pyrenyl, benzodioxolyl, thioxanthonyl, anthraquinonyl and naphthoquinonyl.

3. A compound according to claim 1 or 2, wherein the compound is according to formula (I) and Ar₁ is an aryl according to one of the following formulae (Ar₁-a) to (Ar₁-g): 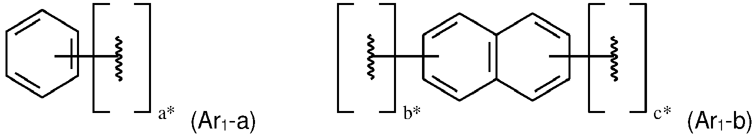 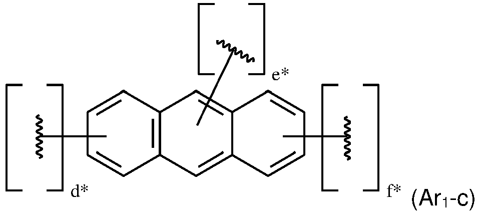 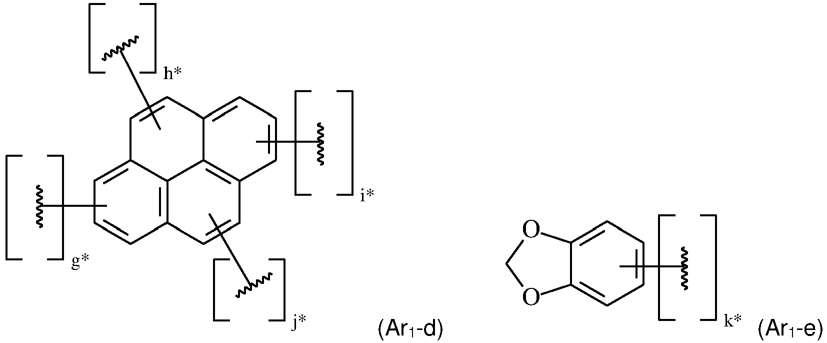 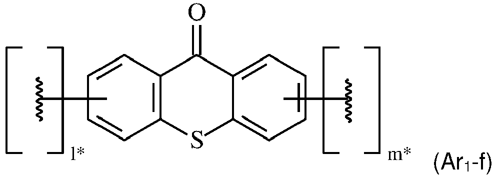 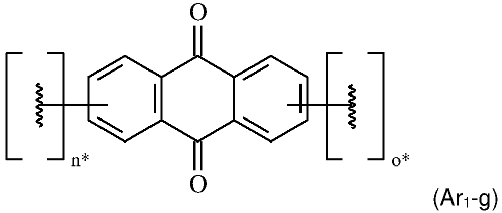 wherein
a* is an integer from 1 to 4 and a* = x;
b* and c* are independently an integer from 0 to 2 and b* + c* = x;
d*, e* and f* are independently an integer from 0 to 2 and d* + e* + f* = x;
g*, h*, i* and j* are independently an integer from 0 to 2 and g* + h* + i* + j* = x;
k* is an integer from 1 to 2 and k* = x;
l* and m* are independently an integer from 0 to 2 and l* + m* = x;
n* and o* are independently an integer from 0 to 2 and n* + o* = x;
each symbol 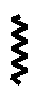 represents a point of attachment to a -[C(=O)]ₙ-X-R moiety; and
each aryl is independently optionally substituted by one or more groups selected from -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C,₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{d} and Rₑ identical or different are independently chosen from H and linear or branched -(C₁-C₂₀)alkyl, and R_{c} is chosen from linear or branched -(C₁-C₂₀)alkyl and -(C₅-C₁₂)aryl optionally substituted by one or more of -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -NR_{d}Rₑ, -NO₂ and -CN.

4. A compound according to claim 1 or 2, wherein the compound is according to formula (II) and Ar₂ is an aryl according to one of the following formulae (Ar₂-a) to (Ar₂-h): 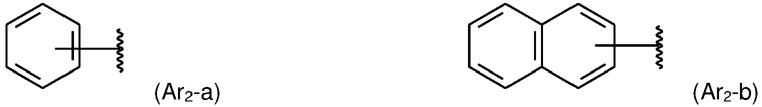 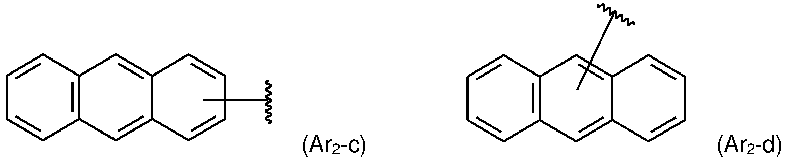 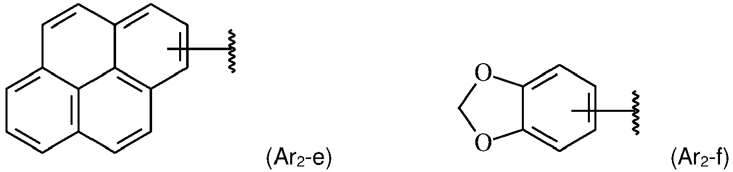 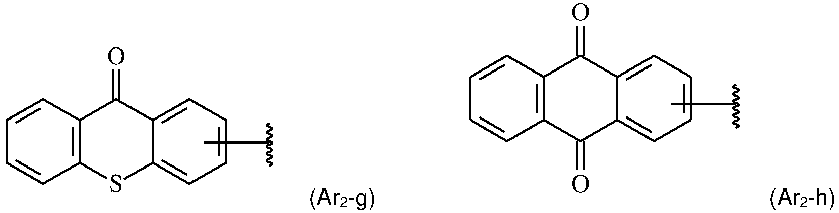 wherein
each symbol 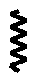 represents a point of attachment to a -[C(=O)]ₙ-X-R moiety; and
each aryl is independently optionally substituted by one or more groups selected from -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C,₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{d} and Rₑ identical or different are independently chosen from H and linear or branched -(C₁-C₂₀)alkyl, and R_{c} is chosen from linear or branched -(C₁-C₂₀)alkyl and -(C₅-C₁₂)aryl optionally substituted by one or more of -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -NR_{d}Rₑ, -NO₂ and -CN.

5. A compound according to claim 1 or 2, wherein the compound is according to formula (III) and Ar₃ is an aryl according to one of the following formulae (Ar₃-a) to (Ar₃-h): 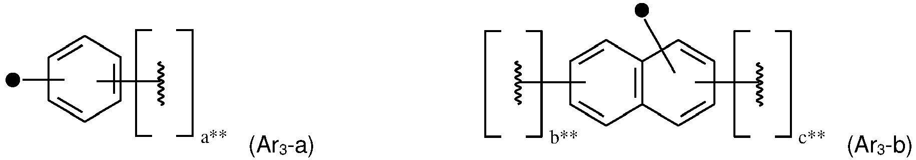 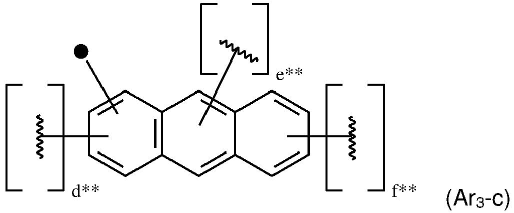 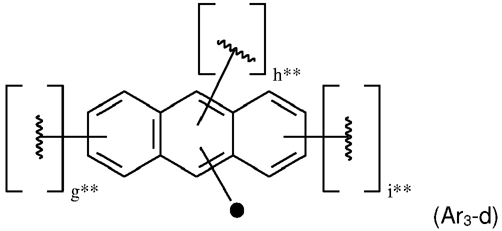 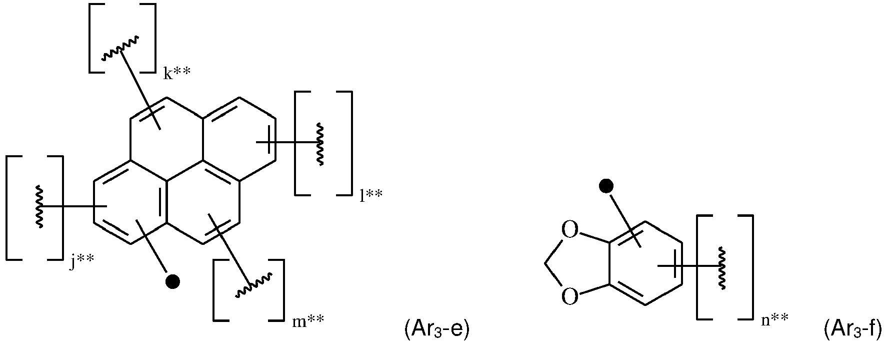 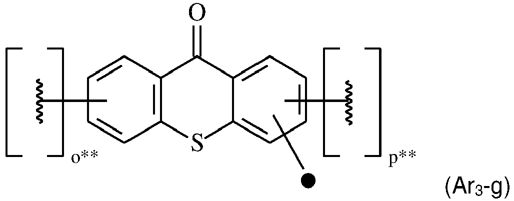 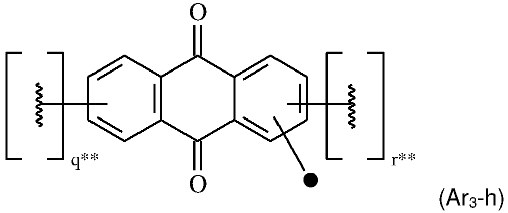 wherein
a** is an integer from 1 to 4 and a** = x;
b** and c** are independently an integer from 0 to 2 and b** + c** = x;
d**, e** and f** are independently an integer from 0 to 2 and d** + e** + f** = x;
g**, h** and i** are independently an integer from 0 to 2 and g** + h** + i** = x;
j**, k**, l** and m** are independently an integer from 0 to 2 and j** + k** + l** + m** = x;
n** is an integer from 1 to 2 and n** = x;
o** and p** are independently an integer from 0 to 2 and o** + p** = x;
q** and r** are independently an integer from 0 to 2 and q** + r** = x;
each symbol 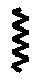 represents a point of attachment to a -[C(=O)]ₙ-X-R moiety;
each symbol • represents a point of attachment to a L-Z- moiety;
each aryl is independently optionally substituted by one or more groups selected from -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C,₂)aryl, -O(C₅-C,₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{d} and Rₑ identical or different are independently chosen from H and linear or branched -(C₁-C₂₀)alkyl, and R_{c} is chosen from linear or branched -(C₁-C₂₀)alkyl and -(C₅-C₁₂)aryl optionally substituted by one or more of -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -NR_{d}Rₑ, -NO₂ and -CN.

6. A compound according to any one of claims 1, 2, 4 and 5, wherein L₁ and L₂ are independently selected from:
- a divalent moiety according to any one of formulae (L0) to (L7):
-CH₂-CH₂-O-CH₂-O-CH₂-CH₂-[S-S-CH₂-CH₂-O-CH₂-O-CH₂-CH₂]_{a*}- (L0)
-(CR⁶R⁷)ₐ- (L1)
-[(CR⁸R⁹)_{b}-W]_{c}-(CR⁸R⁹)_{b}- (L2)
-[(CR¹⁰R¹¹)_{d}-W]ₑ-(CR¹²R¹³)_{f}-[W-(CR¹⁰R¹¹)_{d}]ₑ- (L3)
-[(CR¹⁴R¹⁵)_{g}-O-C(=O)-(CR¹⁶R¹⁷)ₕ-C(=O)-O]ᵢ-(CR¹⁴R¹⁵)_{g}- (L4)
-(CR¹⁸R¹⁹)ⱼ-C(=O)-O-(CR²⁰R²¹)ₖ-O-C(=O)-(CR¹⁸R¹⁹)ⱼ- (L5)
-(CR²²R²³)ₗ-Cy-[A-Cy]ₒ-(CR²²R²³)ₗ- (L6) 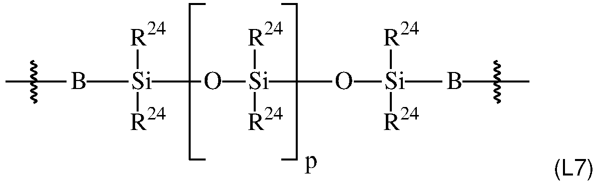
wherein:
- R⁶, R⁷, R¹², R¹³, R¹⁴, R¹⁵, R¹⁶, R¹⁷, R¹⁸, R¹⁹, R²⁰, R²¹, R²² and R²³ are independently H or alkyl;
- R⁸, R⁹, R¹⁰ and R¹¹ are independently H or methyl;
- each R²⁴ is independently alkyl, haloalkyl, alkenyl, cycloalkyl, aryl, aralkyl, alkaryl, alkoxy or aryloxy;
- each W is independently O or S;
- each Cy is an optionally substituted ring, in particular an optionally substituted arylene or an optionally substituted cycloalkylene;
- A is a bond or a linker such as -O-, -S-, Alk, -C(=O)-, -C(=O)-O-Alk-O-C(=O)-, -SO-, -SO₂-, -C(=CCl₂)- and -Alk-Ph-Alk-;
- each Alk is independently an optionally substituted alkylene;
- Ph is an optionally substituted phenylene;
- each B is independently a bond or a hydrocarbon linker which is optionally interrupted by one or more functional groups selected from -O-, -(C=O)-, -(C=O)-O- and -O-(C=O)- ;
- a, f, g, h and k are independently an integer from 2 to 20;
- each I is independently an integer from 0 to 20;
- b and d are independently an integer from 2 to 4;
- o is an integer equal to 0 or 1;
- c, i and j are independently an integer from 1 to 20;
- each e is independently an integer from 0 to 20 with the proviso that at least one e is not 0;
- p is 0 to 100;
- a trivalent moiety according to any one of formulae (L8) to (L11): 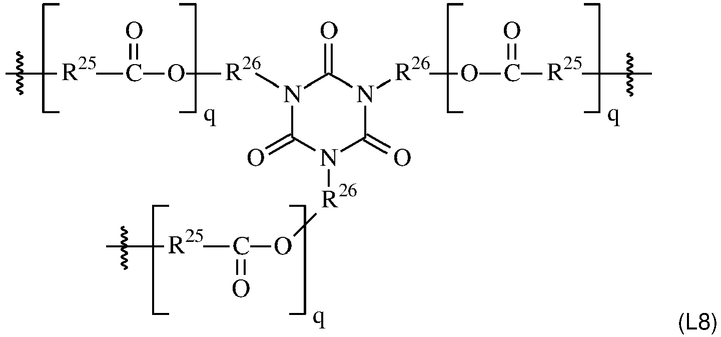 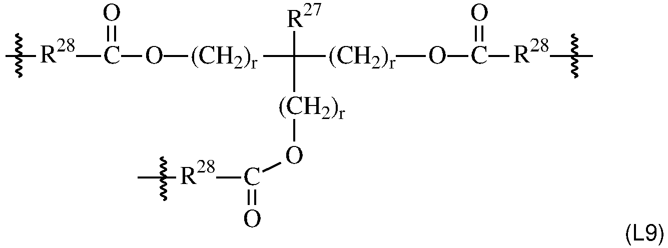 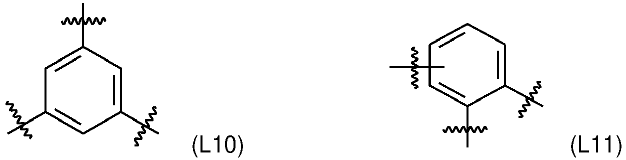 wherein:
- each R²⁵ and R²⁶ is independently an alkylene;
- R²⁷ is H, alkyl or alkoxy, preferably R²⁷ is H or alkyl;
- each R²⁸ is independently an alkylene;
- each q is independently an integer equal to 0 or 1;
- each r is independently an integer from 0 to 2 with the proviso that not more than one r is equal to 0, preferably each r is equal to 1 or one r is equal to 0 and the two other r are equal to 1;
- a tetravalent moiety according to formula (L12) or (L13): 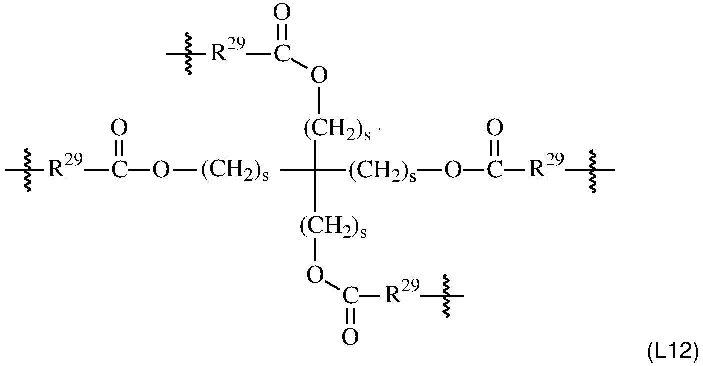 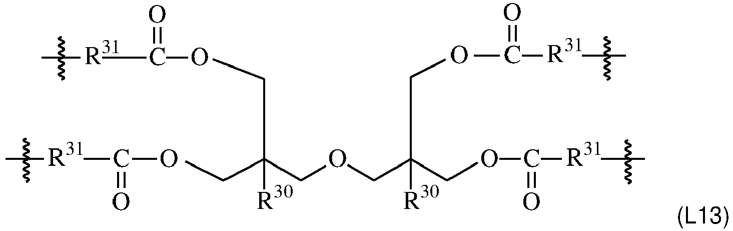 wherein:
- each R²⁹ and R³¹ is independently alkylene;
- each R³⁰ is independently H, alkyl or alkoxy, preferably R³⁰ is alkyl;
- each s is independently an integer from 0 to 2 with the proviso that not more than one s is equal to 0, preferably each s is equal to 1;
- a hexavalent moiety according to formula (L14): 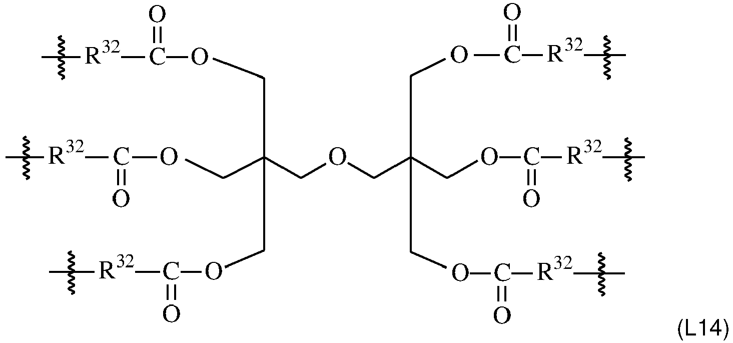 wherein each R³² is independently a linear or branched alkylene;
where
alkyl refers to a linear or branched -(C₁-C₂₀)alkyl,
alkylene refers to a linear or branched -(C₁-C₂₀)alkylene-,
alkoxy refers to linear or branched -O(C₁-C₂₀)alkyl;
aryl refers to a -(C₅-C₁₂)aryl;
alkenyl refers to a linear or branched -(C₂-C₂₀)alkenyl;
cycloalkyl refers to a -(C₃-C₁₂)cycloalkyl;
alkaryl refers to a -(C₅-C₁₂)-arylene-(C₁-C₂₀)alkyl;
aralkyl refers to a -(C₁-C₂₀)-alkylene-(C₅-C₁₂)aryl;
"optionally substituted" refers to none, or one or more substituents chosen from -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C,₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are defined above.

7. A compound according to any one of claims 1 to 3 and 5 to 6, wherein R is a phenyl or a naphthyl, said phenyl or naphthyl being optionally substituted by one or more groups selected from halogen, -CF₃, -NO₂, -CN, -(C₅-C,₂)aryl, linear or branched -(C₁-C₂₀)alkyl group, linear or branched -O(C₁-C₂₀)alkyl group, linear or branched -S(C₁-C₂₀)alkyl group, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined in claim 1, preferably Rₐ, R_{b}, R_{c}, R_{d} and Rₑ are independently a linear or branched (C₁-C₂₀)alkyl.

8. A compound according to any one of claims 1 to 3 and 5 to 6, wherein R is chosen from a linear or branched -(C₁-C₂₀)alkyl, cyclopentyl, cyclohexyl, tetrahydrofuranyl, methyltetrahydrofuranyl and tetrahydropyranyl, said alkyl being optionally interrupted by one or more groups chosen from -O- and -S-, said alkyl being optionally substituted by one or more groups chosen from -(C₅-C₁₂)aryl, -C(=O)ORₐ and -NR_{d}Rₑ wherein Rₐ, R_{d} and Rₑ are as defined above, said cyclohexyl being optionally substituted by one or more groups chosen from the group consisting of -OH, -SH, halogen, linear or branched -(C₁-C₂₀)alkyl group, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₅-C₁₂)aryl, -O(C₅-C₁₂)aryl, -S(C₅-C₁₂)aryl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined in claim 1.

9. A compound according to any one of claims 1 to 8, wherein X is S.

10. A process for the preparation of a compound of formula (I), (II) or (III) according to any one of claims 1 to 9, comprising
- reacting a compound of formula (A) with a compound of formula (B) to obtain a compound of formula (I); or
reacting a compound of formula (C) with a compound of formula (D) to obtain a compound of formula (II); or
reacting a compound of formula (E) with a compound of formula (B) to obtain a compound of formula (III): 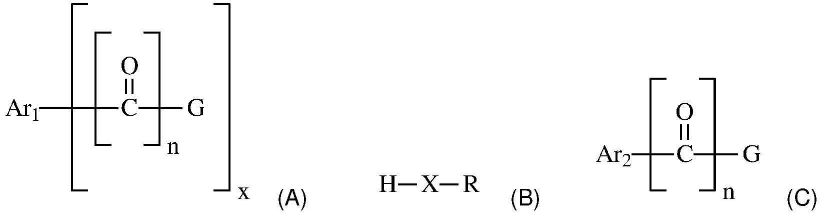 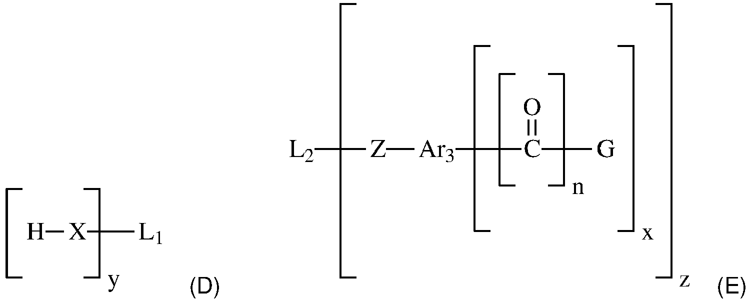
wherein
Ar₁, Ar₂, Ar₃, L₁, L₂, R, X, Z, n, x, y and z are as defined in any one of claims 1 to 9;
each G is independently OH, a halogen atom or -O-C(=O)-J;
J is alkyl or aryl, in particular tert-butyl.

11. A curable composition comprising:
- one or more compounds of formula (I), (II) or (III) according to any one of claims 1 to 9; and
- one or more ethylenically unsaturated compounds.

12. The curable composition according to claim 11, wherein the one or more ethylenically unsaturated compounds comprise at least one of a cyanoacrylate-functionalized compound, a (meth)acrylate-functionalized compound and mixtures thereof.

13. The curable composition according to claim 11 or 12, wherein the curable composition further comprises an anionic synergist, in particular a metallocene, more particularly a ferrocene.

14. Use of a compound of formula (I), (II) or (III) according to any one of claims 1 to 9 as a photoinitiator, in particular as a photoinitiator having photobleaching properties.

15. Use according to claim 14 wherein the photoinitiator is activable by irradiation with a LED light source, preferably a LED light source having a maximum output wavelength in the range of 250 to 550 nm, in particular 250 to 460 nm, more particularly 340 to 430 nm.

16. A process for curing one or more ethylenically unsaturated compounds, said process comprising:
- mixing one or more ethylenically unsaturated compounds with a compound of formula (I), (II) or (III) as defined in any one of claims 1 to 9; and
- irradiating said mixture with at least one light source, preferably with at least one LED light source, more preferably with at least one LED light source having a maximum output wavelength in the range of 250 to 550 nm, in particular 250 to 460 nm, more particularly 340 to 430 nm.

17. The process according to claim 15, wherein the irradiation is carried out under air or under inert atmosphere, preferably under air.

18. A cured product obtainable by curing the curable composition according to any one of claims 11 to 13.
